# EUROPEAN PATENT APPLICATION

(11) **EP 2 565 185 A1**
(43) Date of publication of application: **06.03.2013**
(21) Application number: 11775045.5
(22) Date of filing: 27.04.2011
(51) Int. Cl.: C07D 235/06, A61K 31/4184, A61K 31/4188, A61K 31/427, A61K 31/437, A61K 31/4439, A61K 31/444, A61K 31/454, A61K 31/4545, A61K 31/496, A61K 31/506, A61K 31/5377, A61P 5/18, A61P 19/08, A61P 19/10, C07D 401/04, C07D 403/04, C07D 405/12, C07D 405/14, C07D 417/04, C07D 471/04

(54) **[5,6]HETEROCYCLIC COMPOUND**

(30) Priority: 28.04.2010 JP 2010103349
(71) Applicant: Daiichi Sankyo Company, Limited, Tokyo 103-8426 (JP)
(72) Inventor: AOKI, Kazumasa, Tokyo 134-0081 (JP); MATSUI, Satoshi, Tokyo 134-0081 (JP); YOSHIKAWA, Kenji, Tokyo 134-0081 (JP); SHIMIZU,Hiroki, Tokyo 134-0081 (JP); SASAKI, Junko, Tokyo 134-0081 (JP); NAKAJIMA, Katsuyoshi, Tokyo 134-0081 (JP); KANNO, Osamu, Tokyo 134-0081 (JP); OIZUMI, Kiyoshi, Tokyo 134-0081 (JP)
(74) Representative: Wallace, Sheila Jane
(86) International application number: PCT/JP2011/060241
(87) International publication number: WO 2011/136264

(57) **Abstract**

Abstract: An object of the present invention is to provide a novel low molecular weight compound exhibiting an osteogenesis-promoting action. This object is achieved by a compound having the general formula (I) or a pharmacologically acceptable salt thereof. In the general formula (I), R¹ and R² represent hydrogen atoms, and the like; R³ represents a hydrogen atom, and the like; X, Y, and Z represent nitrogen atoms, and the like; A represents a phenylene group, and the like; n represents 1 or 2, and the like; and V and W represent oxygen atoms, and the like.

## Description

### Technical Field

The present invention relates to a compound or a pharmacologically acceptable salt thereof useful for the prevention or treatment of diseases associated with bone metabolism, for example, osteoporosis, osteitis fibrosa (hyperparathyroidism), osteomalacia, and Paget's disease.

### Background Art

Generally in normal bone metabolism, bone resorption by osteoclasts and osteogenesis by osteoblasts are balanced, whereby homeostasis is maintained. It is presumed that diseases associated with bone metabolism develop when the balance between bone resorption and osteogenesis is disrupted. Such diseases include osteoporosis, osteitis fibrosa (hyperparathyroidism), osteomalacia, Paget's disease, and the like. Particularly, osteoporosis often develops in postmenopausal women and elderly people with accompanying symptoms of pain such as low back pain, bone fracture, etc. Particularly, bone fracture in elderly people is serious because it leads to generalized weakness and dementia. For such diseases associated with bone metabolism, hormone replacement therapies with estrogen and therapeutic agents such as bisphosphonates and calcitonins, both of which inhibit the activity of osteoclasts, have been employed.
However, although many of these therapeutic agents are reported to have a bone resorption-inhibiting action, etc., none of them has yet been clearly shown to have an osteogenesis-promoting action. Particularly, impaired osteogenic ability due to reduced bone turnover is reported to be the main cause of senile osteoporosis (Non Patent Reference 1), and thus a medicinal agent promoting osteogenesis is considered to be effective.

In view of the above, development of a highly clinically effective, orally administrable osteogenesis promoter is demanded.
Recently, benzothiepine derivatives having an alkaline phosphatase-inducing activity (Patent References 1 and 2), N-quinolylanthranilic acid derivatives (Patent Reference 3), triazolopyridazine derivatives (Patent Reference 4), and thienopyridine derivatives (Patent Reference 5) are reported to be useful for promotion of osteogenesis and for the treatment of diseases associated with bone metabolism. However, their clinical utility remains unknown.

### Citation List

### Patent References

Patent Reference 1: U.S. Patent No. 6346521
Patent Reference 2: U.S. Patent No. 6632807
Patent Reference 3: Japanese Patent Laid-Open No. 9-188665
Patent Reference 4: U.S. Patent No. 7173033
Patent Reference 5: Japanese Patent Laid-Open No. 2007-131617

### Non Patent Literature

Non Patent Reference 1: New Eng. J. Med. 314, 1976 (1986)

### Summary of Invention

### Technical Problem

In order to reduce pain and risk of bone fracture in diseases associated with bone metabolism such as osteoporosis, bone mass and bone strength need to be increased. As a means of increasing bone mass and bone strength, it is considered to be important to promote osteogenesis by osteoblasts. Accordingly, an object of the present invention is to provide a highly safe, orally administrable novel low molecular weight compound exhibiting an osteogenesis-promoting action (and/or a bone-resorption inhibiting action).

### Solution to Problem

The present inventors conducted an intensive study in order to develop a therapeutic medication with an osteogenesis-promoting action. As a result, they have found an excellent compound of the present invention that exhibits a potent osteogenesis-promoting action (and/or a bone-resorption inhibiting action) and is potentially capable of serving as a therapeutic medication for the prevention or treatment of diseases associated with bone metabolism, thereby completing the present invention.

Accordingly, the present invention is as follows.
(1) A compound having the general formula (I) or a pharmacologically acceptable salt thereof: 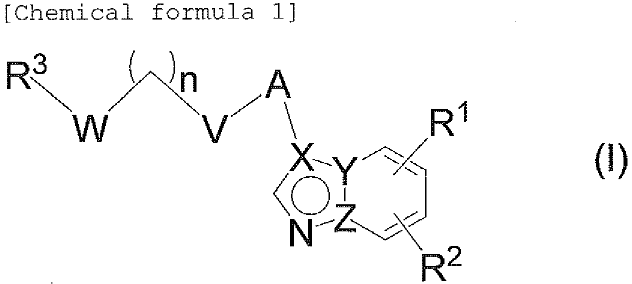 wherein each substituent is defined as follows:
   R¹ and R² each independently represent
   a hydrogen atom or a group selected from a substituent group α, or together form a substituent having bonds at two positions,
   R³ represents:
      a hydrogen atom,
      a C1-C6 alkyl group optionally substituted by a substituent group α,
      a tetrahydropyranyl group optionally substituted by a substituent group α,
      a tetrahydrofuranyl group optionally substituted by a substituent group α,
      a dioxanyl group optionally substituted by a substituent group α,
      a C1-C6 alkoxycarbonyl group,
      a heterocyclic group optionally substituted by a group selected from a substituent group α, or
      a C6-C10 aryl group optionally substituted by a substituent group α,
   X, Y, and Z represent:
      when X is a nitrogen atom, Y and Z are carbon atoms,
      when Y is a nitrogen atom, X and Z are carbon atoms, or
      when Z is a nitrogen atom, X and Y are carbon atoms,
   A represents: a phenylene group optionally substituted by a group selected from a substituent group α, or
   a hetero ring having bonds at two positions, wherein the hetero ring is optionally substituted by a group selected from a substituent group α,
   V represents: -O-,-NH-, or -S-,
   n represents: an integer from 1 to 6, and
   W represents: -O-, -NH-, or -S-, wherein
   the substituent group α includes:
      a hydroxyl group, a halogen group, a cyano group, a C1-C6 alkyl group, a C3-C6 cycloalkyl group, a C3-C6 cycloalkoxy group, a halo C1-C6 alkyl group, a C2-C6 alkynyl group, a C1-C6 alkoxy group, a halo C1-C6 alkoxy group, a C1-C6 alkylsulfonyl group, a formyl group, a C1-C6 alkylcarbonyl group, a carboxy group, a C1-C6 alkoxycarbonyl group, a C1-C6 alkylamino group, a C3-C6 cycloalkylcarbonyl group, a phenyl group optionally substituted by a group selected from a substituent group β, a heterocyclic group optionally substituted by a group selected from a substituent group β, a carbonyl group to which a heterocyclic group is bound, and a C1-C6 alkylenedioxylene group, and
   the substituent group β includes:
      a nitro group, a cyano group, an aminosulfonyl group, a di C1-C6 alkylamino group, a di C1-C6 alkylaminocarbonyl group, a di C1-C6 alkylaminocarbonyloxy group, a di C1-C6 alkylaminosulfonyl group, a carboxy group, a C1-C6 alkyl group, a C1-C6 alkoxy group, a formyl group, a C1-C6 alkylcarbonyl group, a C1-C6 alkylcarbonylamino group, a C1-C6 alkylsulfonylamino group, a morpholinylcarbonyl group, and a carbamoyl group.

Also, preferred aspects of the present invention are the following.
(2) A compound or a pharmacologically acceptable salt thereof according to (1), wherein the heterocyclic group is an azetidinyl group, a pyrrolidinyl group, a piperidinyl group, a morpholinyl group, a pyrazinyl group, a pyridinyl group, a tetrahydropyridinyl group, a 2-oxo-1,2-dihydropyridinyl group, a pyrrolyl group, a tetrahydropyranyl group, a tetrahydrofuranyl group, a dioxanyl group, a pyrimidyl group, a pyrazoyl group, an imidazoyl group, or an oxazoyl group, and the hetero ring is azetidine, pyrrolidine, piperidine, morpholine, pyrazine, pyridine, tetrahydropyridine, 2-oxo-1,2-dihydropyridine, pyrrole, tetrahydropyran, tetrahydrofuran, dioxane, pyrimidine, pyrazole, imidazole, or oxazole.
(3) A compound or a pharmacologically acceptable salt thereof according to (1) or (2), wherein A is a group selected from the following groups: 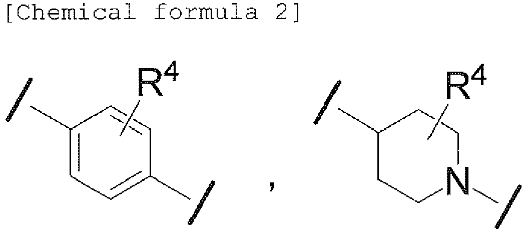 wherein, R⁴ represents: a hydrogen atom or a group selected from the substituent group α.
(4) A compound or a pharmacologically acceptable salt thereof according to any one of (1) to (3), wherein R¹ and R² are each independently a hydrogen atom, a hydroxyl group, a cyano group, a fluorine atom, a chlorine atom, a methyl group, an ethyl group, a methoxy group, an ethoxy group, a difluoromethyl group, a trifluoromethyl group, a difluoromethoxy group, a trifluoromethoxy group, a phenyl group optionally substituted by a group selected from the substituent group β, a heterocyclic group optionally substituted by a group selected from the substituent group β, or a carbamoyl group.
(5) A compound or a pharmacologically acceptable salt thereof according to (1), wherein the general formula (I) is the general formula (Ia): 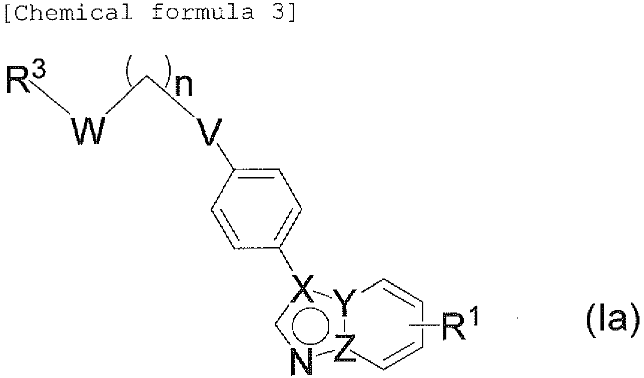
(6) A compound or a pharmacologically acceptable salt thereof according to any one of (1) to (5), wherein R³ is a hydrogen atom, a C1-C6 alkyl group substituted by a hydroxyl group, a tetrahydropyranyl group, a tetrahydrofuranyl group, a dioxanyl group, a C1-C6 alkoxycarbonyl group, a piperidinyl group optionally substituted by a group selected from the substituent group α, or a phenyl group optionally substituted by the substituent group α.
(7) A compound or a pharmacologically acceptable salt thereof according to any one of (1) to (6), wherein V is -O- or -NH-.
(8) A compound or a pharmacologically acceptable salt thereof according to any one of (1) to (7), wherein W is -O- or -NH-.
(9) A compound or a pharmacologically acceptable salt thereof according to any one of (1) to (8), wherein n is an integer from 1 to 3.
(10) A compound selected from the following group of compounds or a pharmacologically acceptable salt thereof:
   2-[4-(1H-benzimidazol-1-yl)phenoxy]ethanol,
   2-[4-(7-chloroimidazo[1,2-a]pyridin-3-yl)phenoxy]ethanol,
   2-(2-{[4-(1H-benzimidazol-1-yl)phenyl]amino}ethoxy)ethanol,
   2-{[4-(6-chloro-1H-benzimidazol-1-yl)phenyl]amino}ethanol,
   2-{[4-{5-methoxy-1H-benzimidazol-1-yl)phenyl]amino}ethanol,
   1-[4-(2-methoxyethoxy)phenyl]-1H-benzimidazole,
   2-[4-(6-fluoroimidazo[1,2-a]pyridin-3-yl)phenoxy]ethanol,
   2-{4-[6-(1H-pyrrol-3-yl)imidazo[1,2-a]pyridin-3-yl]phenoxy}ethanol,
   2-[4-(5-pyridin-4-yl-1H-benzimidazol-1-yl)phenoxy]ethanol,
   2-({1-[7-(1-acetyl-1,2,3,6-tetrahydropyridin-4-yl)imidazo[1,2-a]pyridin-3-yl]piperidin-4-yl}oxy)ethanol,
   2-[4-(6-pyridin-4-ylpyrazolo[1,5-a]pyridin-3-yl)phenoxy]ethanol,
   4-{2-[4-(1H-benzimidazol-1-yl)phenoxy]ethoxy}benzoic acid,
   4-{1-[4-(2-hydroxyethoxy)phenyl]-1H-benzimidazol-5-yl)benzoic acid,
   N-(4-{1-[4-(2-hydroxyethoxy)phenyl]-1H-benzimidazol-5-yl}phenyl)acetamide,
   4-{1-[4-(2-hydroxyethoxy)phenyl]-1H-benzimidazol-5-yl}benzamide,
   2-(4-{5-[6-(morpholin-4-ylcarbonyl)pyridin-3-yl]-1H-benzimidazol-1-yl}phenoxy)ethanol,
   7-[4-(morpholin-4-ylcarbonyl)phenyl]-3-{4-[2-(tetrahydro-2H-pyran-4-yloxy)ethoxy]piperidin-1-yl}imidazo[1,2-a]pyridine,
   3-{4-[2-(tetrahydro-2H-pyran-2-yloxy)ethoxy]piperidin-1-yl}pyrazolo[1,5-a]pyridine,
   3-{4-[2-(tetrahydro-2H-pyran-4-yloxy)ethoxy]phenyl}imidazo[1,2-a]pyridine-6-carboxamide,
   6-methoxy-3-{4-[2-(tetrahydro-2H-pyran-4-yloxy)ethoxy]phenyl}imidazo[1,2-a]pyridine,
   6-ethynyl-3-{4-[2-(tetrahydro-2H-pyran-4-yloxy)ethoxy]phenyl}imidazo[1,2-a]pyridine,
   6-morpholin-4-yl-3-{4-[2-(tetrahydro-2H-pyran-4-yloxy)ethoxy]phenyl}imidazo[1,2-a]pyridine,
   6-(1H-pyrazol-1-yl)-3-{4-[2-(tetrahydro-2H-pyran-4-yloxy)ethoxy]phenyl}imidazo[1,2-a]pyridine,
   1-{4-[2-(tetrahydro-2H-pyran-4-yloxy)ethoxy]phenyl}-1H-benzimidazole-6-carbonitrile, and
   6-(difluoromethoxy)-1-{4-[2-(tetrahydro-2H-pyran-4-yloxy)ethoxy]phenyl}-1H-benzimidazole.

(11) A pharmaceutical composition comprising a compound or a pharmacologically acceptable salt thereof according to any one of (1) to (10) as an active ingredient.
(12) A pharmaceutical composition according to (11), wherein the pharmaceutical composition is used for promoting osteogenesis.
(13) A pharmaceutical composition according to (11), wherein the pharmaceutical composition is used for improving bone metabolism.
(14) A pharmaceutical composition according to (11), wherein the pharmaceutical composition is used for the prevention or treatment of a disease associated with bone metabolism.
(15) A pharmaceutical composition according to (14), wherein the disease associated with bone metabolism is osteoporosis.

The prevent invention further encompasses the inventions described below.
(16) A method for improving bone metabolism, comprising administering an effective amount of a pharmaceutical composition according to (11) to a mammal.
(17) A method for preventing or treating a disease associated with bone metabolism, comprising administering an effective amount of a pharmaceutical composition according to (11) to a mammal.
(18) A method for preventing or treating osteoporosis, comprising administering an effective amount of a pharmaceutical composition according to (11) to a mammal.

### Advantageous Effects of Invention

The compound of the present invention has low toxicity and exhibits favorable disposition. Also, it has an osteogenesis-promoting action, and thus is useful for the prevention or treatment of metabolic bone disease associated with reduced osteogenic ability relative to bone resorption ability. Examples of such metabolic bone disease include osteoporosis, osteitis fibrosa (hyperparathyroidism), osteomalacia, and further, Paget's disease, which affects systemic parameters of bone metabolism. In particular, the compound of the present invention is useful for senile osteoporosis associated with impaired osteogenic ability. Further, application of the osteogenesis promoter of the present invention in the field of orthopedics for the promotion of healing of bone fracture, a bone defect, and bone diseases such as osteoarthritis as well as in the field of dentistry for the treatment of periodontal disease, stabilization of artificial tooth root, etc. is anticipated.

### Description of Embodiments

The present invention will be described in detail hereinbelow.
In the present specification, terms such as substituents used to denote a compound have the following meanings:

### A halogen group:

A fluorine group, a chlorine group, or a bromine group

### A C1-C6 alkyl group:

A linear or branched alkyl group having a carbon number of 1 to 6. It is preferably a methyl group, an ethyl group, a propyl group, an isopropyl group, or a t-butyl group.

### A C2-C6 alkynyl group:

A linear or branched alkynyl group having a carbon number of 2 to 6. It is preferably an ethynyl group, or a propynyl group.

### A C1-C6 alkylcarbonyl group:

A group in which a carbonyl group is bound to the aforementioned C1-C6 alkyl group. It is preferably an acetyl group, an ethylcarbonyl group, a propylcarbonyl group, an isopropylcarbonyl group, or a butylcarbonyl group.

### A C1-C6 alkylsulfonyl group:

A group in which a sulfonyl group is bound to the aforementioned C1-C6 alkyl group. It is preferably a methylsulfonyl group, an ethylsulfonyl group, a propylsulfonyl group, an isopropylsulfonyl group, or a butylsulfonyl group, of which a methylsulfonyl group or an ethylsulfonyl group is more preferred.

### A C1-C6 alkoxy group:

A group in which an oxygen atom is bound to the aforementioned C1-C6 alkyl group. It is preferably a methoxy group, an ethoxy group, a propoxy group, an isopropoxy group, or a t-butoxy group.

### A C1-C6 alkoxycarbonyl group:

A group in which a carbonyl group is bound to the aforementioned C1-C6 alkoxy group. It is preferably a methoxycarbonyl group, an ethoxycarbonyl group, a propoxycarbonyl group, an isopropoxycarbonyl group, or a t-butoxycarbonyl group.

### A C1-C6 alkylamino group:

A group in which one of the aforementioned C1-C6 alkyl groups is bound to an amino group. It is preferably a methylamino group, an ethylamino group, a propylamino group, an isopropylamino group, or a butylamino group.

### A C1-C6 alkoxycarbonylamino group:

A group in which a carbonylamino group is bound to the aforementioned C1-C6 alkoxy group. It is preferably a methoxycarbonylamino group or an ethoxycarbonylamino group.

### A C1-C6 alkylsulfonylamino group:

A group in which a sulfonylamino group is bound to the aforementioned C1-C6 alkyl group. It is preferably a methylsulfonylamino group or an ethylsulfonylamino group.

### A halo C1-C6 alkyl group:

The aforementioned C1-C6 alkyl group that is substituted with a halogen group. Examples thereof include a fluoromethyl group, a difluoromethyl group, a trifluoromethyl group, a fluoroethyl group, a difluoroethyl group, a trifluoroethyl group, a fluoropropyl group, a difluoropropyl group, a trifluoropropyl group, a fluorobutyl group, a difluorobutyl group, a trifluorobutyl group, a fluoropentyl group, a difluoropentyl group, a trifluoropentyl group, a fluorohexyl group, a difluorohexyl group, a trifluorohexyl group, a pentafluoroethyl group, a hexafluoropropyl group, a nonafluorobutyl group, a chloromethyl group, a dichloromethyl group, a trichloromethyl group, a chloroethyl group, a dichloroethyl group, a trichloroethyl group, a chloropropyl group, a dichloropropyl group, or a trichloropropyl group.

### A halo C1-C6 alkoxy group:

The aforementioned C1-C6 alkoxy group that is substituted with a halogen atom. Examples thereof include a fluoromethoxy group, a difluoromethoxy group, a trifluoromethoxy group, a fluoroethoxy group, a difluoroethoxy group, a trifluoroethoxy group, a fluoropropoxy group, a difluoropropoxy group, a trifluoropropoxy group, a fluorobutoxy group, a difluorobutoxy group, a trifluorobutoxy group, a fluoropentyloxy group, a difluoropentyloxy group, a trifluoropentyloxy group, a fluorohexyloxy group, a difluorohexyloxy group, a trifluorohexyloxy group, a pentafluoroethoxy group, a hexafluoropropoxy group, a nonafluorobutoxy group, a chloromethoxy group, a dichloromethoxy group, a trichloromethoxy group, a chloroethoxy group, a dichloroethoxy group, a trichloroethoxy group, a chloropropoxy group, a dichloropropoxy group, or a trichloropropoxy group.

### A C3-C6 cycloalkyl group:

A cyclic alkyl group having a carbon number of 3 to 6. It is preferably a cyclopropyl group, a cyclobutyl group, a cyclopentyl group, or a cyclohexyl group.

### A C3-C6 cycloalkylcarbonyl group:

A group in which a carbonyl group is bound to the aforementioned C3-C6 cycloalkyl group. It is preferably a cyclopropylcarbonyl group, a cyclobutylcarbonyl group, a cyclopentylcarbonyl group, or a cyclohexylcarbonyl group.

### A C3-C6 cycloalkoxy group:

A group in which an oxygen atom is bound to the aforementioned C3-C6 cycloalkyl group. It is preferably a cyclopropyloxy group, a cyclobutyloxy group, a cyclopentyloxy group, or a cyclohexyloxy group.

### A di C1-C6 alkylamino group:

A group in which two of the aforementioned C1-C6 alkyl groups are bound to an amino group. It is preferably a dimethylamino group.

### A di C1-C6 alkylaminocarbonyl group:

A group in which two of the aforementioned C1-C6 alkyl groups are bound to an aminocarbonyl group. It is preferably a dimethylaminocarbonyl group.

### A di C1-C6 alkylaminocarbonyloxy group:

A group in which an oxygen atom is bound to the aforementioned C1-C6 alkylaminocarbonyl group. It is preferably a dimethylaminocarbonyloxy group.

### A di C1-C6 alkylaminosulfonyl group:

A group in which two of the aforementioned C1-C6 alkyl groups are bound to an aminosulfonyl group. It is preferably a dimethylaminosulfonyl group.

### A C6-C10 aryl group:

An aromatic group having a carbon number of 6 to 10. It is preferably a phenyl group, an indenyl group, or a naphthalenyl group.

### A C1-C6 alkylenedioxy group:

An alkylene group having a carbon number of 1 to 6, to both ends of which oxygen atoms are bound. It is preferably a methylenedioxy group or an ethylenedioxy group.

### A hetero ring or heterocyclic group:

A 5- to 7-membered heterocyclic group having 1 to 3 sulfur atom(s), oxygen atom(s), and/or nitrogen atom(s). Examples thereof include an aromatic heterocyclic group such as furyl, thienyl, pyrrolyl, azepinyl, pyrazolyl, imidazolyl, oxazolyl, isoxazolyl, thiazolyl, isothiazolyl, 1,2,3-oxadiazolyl, 1,3,4-oxadiazolyl, triazolyl, tetrazolyl, thiadiazolyl, pyranyl, pyridyl, pyridazinyl, pyrimidinyl, and pyrazinyl, and a saturated heterocyclic group in a partially or fully reduced form such as tetrahydropyranyl, morpholinyl, thiomorpholinyl, pyrrolidinyl, pyrrolinyl, imidazolidinyl, pyrazolidinyl, piperidinyl, piperazinyl, oxazolidinyl, isoxazolidinyl, thiazolidinyl, and pyrazolidinyl. It is preferably a 5-or 6-membered aromatic heterocyclic group.

The "aromatic heterocyclic group" may be condensed with another cyclic group. Examples include a group such as benzothienyl, isobenzofuranyl, chromenyl, xanthenyl, phenoxathiinyl, indolizinyl, isoindolyl, indolyl, indazolyl, purinyl, quinolidinyl, isoquinolyl, quinolyl, phthalazinyl, naphthyridinyl, quinoxalinyl, quinazolinyl, carbazolyl, carbolinyl, acridinyl, and isoindolinyl.

It is preferably an azetidinyl group, a pyrrolidinyl group, a piperidinyl group, a morpholinyl group, a piperazinyl group, a pyrazinyl group, an azepanyl group, a 1,4-diazepanyl group, a pyrrolyl group, a thiazoyl group, a pyridinyl group, a tetrahydropyridinyl group, a 2-oxo-1,2-dihydropyridinyl group, a tetrahydropyranyl group, a tetrahydrofuranyl group, a dioxanyl group, a pyrimidyl group, a pyrazoyl group, an imidazoyl group, an oxazoyl group, a tetrahydroisoquinolyl group, or a decahydroisoquinolyl group.

Preferred substituents in the compound having the general formula (I) are as follows:
R¹ and R² each independently represent:
   a hydrogen atom, a hydroxyl group, a cyano group, a fluorine atom, a chlorine atom, a methyl group, an ethyl group, a methoxy group, an ethoxy group, a difluoromethyl group, a trifluoromethyl group, a difluoromethoxy group, a trifluoromethoxy group, a phenyl group optionally substituted by a group selected from a substituent group β, a heterocyclic group optionally substituted by a group selected from a substituent group β, or a carbamoyl group, R³ represents:
   a hydrogen atom, a C1-C6 alkyl group substituted by a hydroxyl group, a tetrahydropyranyl group, a tetrahydrofuranyl group, a dioxanyl group, a C1-C6 alkoxycarbonyl group, a piperidinyl group optionally substituted by a group selected from a substituent group α, or a phenyl group optionally substituted by a substituent group α,
X, Y, and Z represent:
   when X is a nitrogen atom, Y and Z are carbon atoms, when Y is a nitrogen atom, X and Z are carbon atoms, or when Z is a nitrogen atom, X and Y are carbon atoms,
A represents:
   a phenylene group optionally substituted by a group selected from a substituent group α, or a hetero ring having bonds at two positions in which the hetero ring is optionally substituted by a group selected from a substituent group α ,
V represents: -O- or -NH-,
n represents: an integer from 1 to 3, and
W represents: -O- or -NH-.

As a compound having the general formula (I), a compound having the general formula (Ia) is preferred, and the ones described in the Examples are more preferred.

The phrase "optionally substituted by" refers to either being unsubstituted or being substituted by one to three substituents.
The term "treatment" refers to curing diseases or symptoms.
The term "pharmacologically acceptable salt thereof" refers to a salt that can be used as a medicine. A compound of the present invention having an acidic group or a basic group can be obtained as a basic salt or an acidic salt through reaction with a base or an acid, respectively; therefore, such a salt is referred to as a "pharmacologically acceptable salt thereof."
Preferred examples of a pharmacologically acceptable "basic salt" of a compound of the present invention include an alkali metal salt such as a sodium salt, a potassium salt, and a lithium salt; an alkaline earth metal salt such as a magnesium salt and a calcium salt; an organic basic salt such as an N-methylmorpholine salt, a triethylamine salt, a tributylamine salt, a diisopropylethylamine salt, a dicyclohexylamine salt, an N-methylpiperidine salt, a pyridine salt, a 4-pyrrolidinopyridine salt, and a picoline salt; or an amino acid salt such as a glycine salt, a lysine salt, an arginine salt, an ornithine salt, a glutamic acid salt, and an aspartic acid salt, of which an alkali metal salt is preferred.

Preferred examples of a pharmacologically acceptable "acidic salt" of a compound of the present invention include a hydrohalide such as hydrofluoride, hydrochloride, hydrobromide, and hydroiodide; an inorganic acid salt such as nitrate, perchlorate, sulfate, and phosphate; an organic acid salt such as lower alkanesulfonate such as methanesulfonate, trifluoromethanesulfonate, and ethanesulfonate, arylsulfonate such as benzenesulfonate and p-toluenesulfonate, an organic acid salt such as acetate, malate, fumarate, succinate, citrate, ascorbate, tartrate, oxalate, and maleate; and an amino acid salt such as a glycine salt, a lysine salt, an arginine salt, an ornithine salt, a glutamic acid salt, and an aspartic acid salt, of which a hydrohalide, particularly a hydrochloride, is most preferred.

A compound of the present invention or a pharmacologically acceptable salt thereof may absorb water, contain hygroscopic water, or form a hydrate, when left in the atmosphere or subjected to recrystallization. The present invention also encompasses compounds in such various forms of hydrates, solvates, and crystal polymorphs.

A compound of the present invention, a pharmacologically acceptable salt thereof, or a solvate thereof may be present as various isomers such as geometric isomers including a cis-form, a trans-form, etc., tautomers, or enantiomers such as a D-form and an L-form, depending on the kind or combination of substituents. Unless otherwise specifically restricted, a compound of the present invention encompasses all of these isomers and stereoisomers, and a mixture containing these isomers and stereoisomers in any ratio. A mixture of these isomers can be separated by publicly known means of separation.
A compound of the present invention also encompasses a labeled compound, namely a compound of the present invention in which one or more atoms is substituted with isotopes (for example, ²H, ³H, ¹³C, ¹⁴C, and ³⁵S).

Further, the present invention also encompasses so-called prodrugs of a compound of the present invention which are pharmacologically acceptable. A pharmacologically acceptable prodrug is a compound having a group that can be converted into an amino group, a hydroxyl group, a carboxyl group, and the like of the compound of the invention by hydrolysis or under physiological conditions. Examples of a group forming such a prodrug include ones described in Prog. Med., Vol. 5, pages 2157 to 2161, 1985; and "Iyakuhin no kaihatu" (literal translation: development of pharmaceutical product) (Hirokawa Shoten Ltd.) Vol. 7, Bunshi Sekkei (literal translation: molecular design) pages 163 to 198. More specifically, examples of a prodrug of a compound of the present invention having an amino group include a compound in which the amino group is acylated, alkylated, or phosphorylated (for example, the compound in which the amino group is converted into eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, and tert-butyl). Also, more specifically, examples of a prodrug of a compound of the present invention having a hydroxyl group include a compound in which the hydroxyl group is acylated, alkylated, phosphorylated, or borated (for example, the compound in which the hydroxyl group is converted into acetyl, palmitoyl, propanoyl, pivaloyl, succinyl, fumaryl, alanyl, and dimethylaminomethylcarbonyl). Also, more specifically, examples of a prodrug of a compound of the present invention having a carboxyl group include a compound in which the carboxyl group is esterified or amidated (for example, the compound in which the carboxyl group is ethyl-esterified, phenyl-esterified, carboxymethyl-esterified, dimethylaminomethyl-esterified, pivaloyloxymethyl-esterified, ethoxycarbonyloxyethyl-esterified, amidated, or methylamidated).

### (Production method)

A compound of the present invention can be produced by applying various publicly known production methods, while taking advantage of characteristics based on the basic structure of the compound or the kind of substituent. Examples of publicly known methods include methods described in "ORGANIC FUNCTIONAL GROUP PREPARATIONS", second edition, ACADEMIC PRESS, INC., 1989, and "Comprehensive Organic Transformations", VCH Publishers Inc., 1989, and the like.
Upon production of a compound of the present invention, depending on the kind of functional group, it may be effective, from the production technique point of view, to protect the functional group of a raw material or intermediate compound with an appropriate protective group or replace the functional group by a readily-convertible group in advance.
Examples of the functional group include an amino group, a hydroxyl group, and a carboxyl group, and examples of the protective group thereof include ones described in "Protective Groups in Organic Synthesis (third edition, 1999)" written by T.W. Greene and P.G. Wuts. These protective groups can be appropriately selected in accordance with their reaction conditions. According to these methods, a desired compound can be obtained by introducing the substituent and carrying out the reaction, and then removing the protective group or converting the substituent into a desired group, as needed.
Further, a prodrug of a compound of the present invention can be produced by, similarly to the aforementioned protective groups, introducing a specific group into a raw material or intermediate compound, or carrying out the reactions using a compound produced according to the present invention. The reaction can be carried out by using a method publicly known to those skilled in the art such as methods normally performed, for example, esterification, amidation, dehydration, and hydrogenation.
Hereinbelow, the production method of the compound of the present invention will be described. However, the production method is not limited to the below-described methods in any way.

### (Method A)

Method (A) is a production method composed of (Step A1), the step of producing a compound (a-3) by coupling a compound (a-1) with a compound (a-2), and (Step A2), the step of producing a compound (a-5), which is a compound of the present invention, by coupling the compound (a-3) with a compound (a-4). 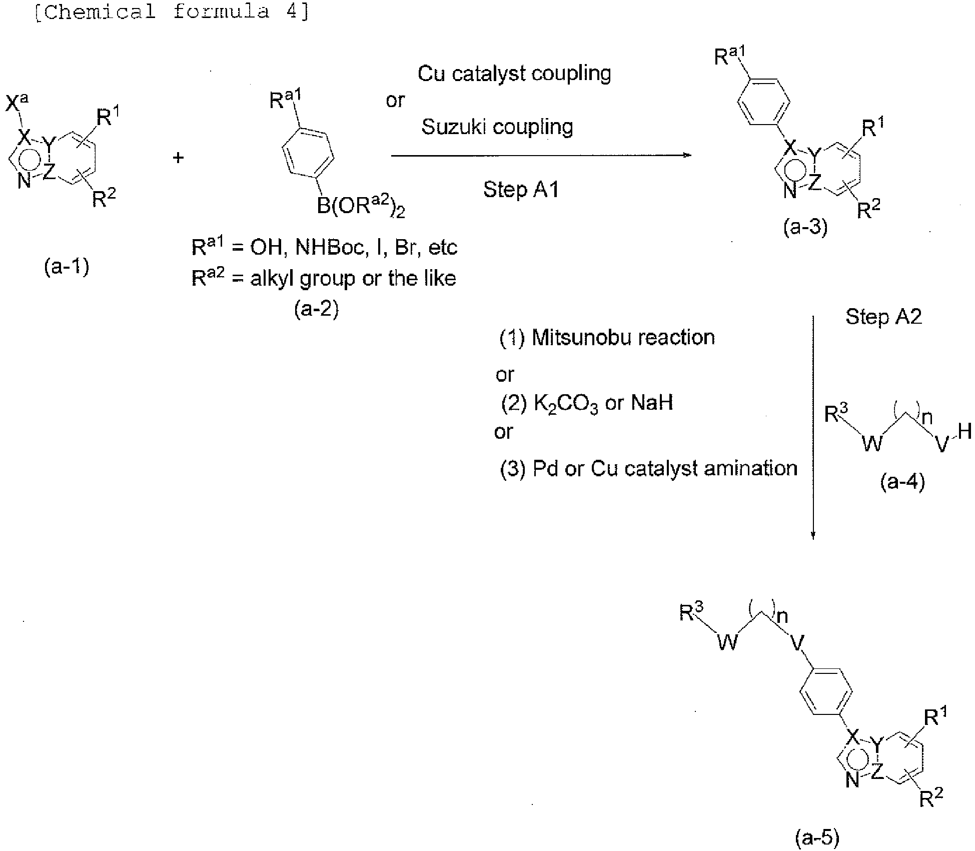 In the above scheme, R¹, R², R³, Y, Z, n, V, and W have the same meanings as above, and when X is a nitrogen atom, X^{a} is a hydrogen atom, and when X is a carbon atom, X^{a} is an iodine atom. Also, R^{a1} is a hydroxyl group, - NHBoc, an iodine atom, or a bromine atom, wherein Boc represents a t-butoxycarbonyl group, and R^{a2} represents an alkyl group or the like.

Step A1: This step is a coupling reaction using a palladium catalyst or a copper catalyst, and is a step of producing a compound (a-3) from a compound (a-1). A coupling reaction using a palladium catalyst is a so-called Suzuki coupling, and is performed in the presence of a palladium catalyst, a ligand, a base, and a solvent by heating. This coupling reaction can be performed in accordance with the methods described in, for example, Tetrahedron Letters, 32, 20, 1991, 2273-2276, Tetrahedron, 49, 43, 1993, 9713-9720, Synthesis, 18, 2007, 2853-2861, Angewandte Chemie, International Edition, 46, 17, 2007, 3135-3138, Journal of the American Chemical Society, 116, 15, 1994, 6985-6986, Heterocycles, 26, 10, 1987, 2711-2716, Synthetic Communications, 30, 19, 2000, 3501-3510, Tetrahedron Letters, 42, 37, 2001, 6523-6526, Tetrahedron Letters, 42, 33, 2001, 5659-5662, Journal of Organic Chemistry, 68, 24, 2003, 9412-9415, Journal of Organic Chemistry, 68, 20, 2003, 7733-7741, Journal of Organic Chemistry, 70, 6, 2005, 2191-2194, Synlett, 13, 2005, 2057-2061, European Journal of Organic Chemistry, 8, 2006, 1917-1925, Organic Letters, 8, 16, 2006, 3605-3608, Journal of Organic Chemistry, 71, 10, 2006, 3816-3821, Chemistry A European Journal, 12, 19, 2006, 5142-5148, Organic Letters, 5, 6, 2003, 815-818, Journal of Organic Chemistry, 73, 4, 2008, 1429-1434, and the like. A coupling reaction using a copper catalyst can be performed in accordance with the methods described in Organic Letters, 2, 9, 2000, 1233-1236, Tetrahedron Letters, 44, 19, 2003, 3863-3865, Tetrahedron Letters, 39, 19, 1998, 2941-2944, Journal of Organic Chemistry, 66, 4, 2001, 1528-1531, and the like.

Step A2: This is a step of converting the compound (a-3) into a compound (a-5). The compound (a-5) can be produced by a method including a step that is mainly composed of a condensation reaction, i.e., a so-called Mitsunobu reaction, formation of a phenyl ether by a Williamson etherification reaction, or formation of aniline by an amination reaction using a palladium catalyst or a copper catalyst. The amination reaction using a palladium catalyst can be performed in accordance with the methods described in Journal of Organic Chemistry, 65, 4, 2000, 1144-1157, Journal of Organic Chemistry, 65, 4, 2000, 1158-1174, Journal of the American Chemical Society, 125, 22, 2003, 6653-6655, and the like. The amination reaction using a copper catalyst can be performed in accordance with the methods described in Journal of the American Chemical Society, 123, 31, 2001, 7727-7729, Journal of Organic Chemistry, 70, 13, 2005, 5164-5173, Journal of Organic Chemistry, 68, 11, 2003, 4367-4370, and the like.

### (Method B)

Method B is a method for producing a compound of the present invention (b-2) by introducing an appropriate substituent into the ring of a compound (b-1), which is producible in accordance with method A. 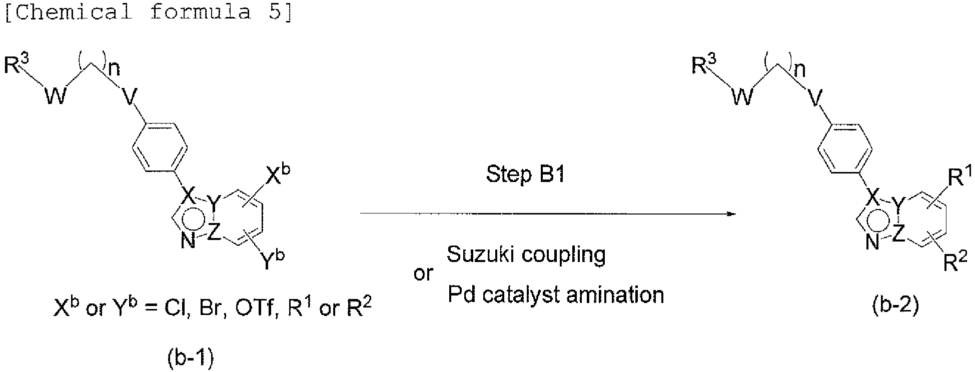 In the above scheme, R¹, R², R³, X, Y, Z, n, V, and W have the same meanings as above, and X^{b} or Y^{b} represents R¹, R², a halogen atom, or a trifluoromethanesulfonyloxy group.

Step B1: This step is a reaction using a palladium catalyst, and is a step of converting a compound (b-1) into a compound (b-2). This step can be performed in a similar manner to the reactions involved in Step A1 or Step A2 of method A.

### (Method C)

Method C is a method for producing a compound of the present invention (c-2) by removing a protective group in a compound (c-1), which is producible in accordance with method A. 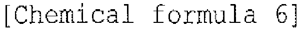 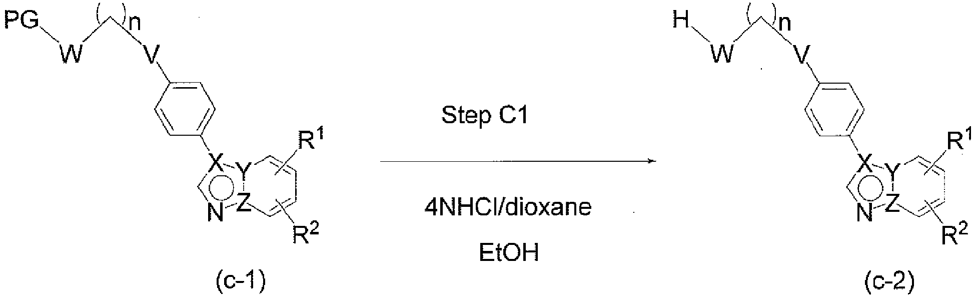 In the above scheme, R¹, R², X, Y, Z, n, V, and W have the same meanings as above, and PG represents a protective group of W.

Step C1: Although W in the compound (c-1) is protected with a protective group, this step removes the protective group. The deprotection of the hydroxyl group can be performed in accordance with, for example, the methods described in "Protective Groups in Organic Synthesis (third edition, 1999)" written by T. W. Greene and P. G. Wuts.

### (Method D)

Method D is a method for producing a compound of the present invention (a-5), and is an alternative to method A. Method D is a method for producing a compound of the present invention (a-5) by coupling the compound (a-1) with a compound (d-1), which is producible in accordance with Step A2 of method A. 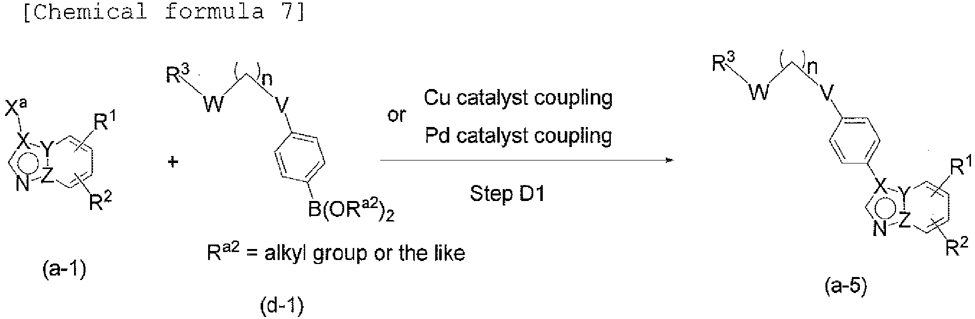 In the above scheme, R¹, R², R³, R^{a2}, X, Y, Z, n, V, W, and X^{a} have the same meanings as above.
Step D1: Reactions in this step can be performed under similar conditions to Step A1 of method A.

### (Method E)

Method E is a method for producing a compound of the present invention (e-6), in the case that the main skeletal structure is imidazopyridine. 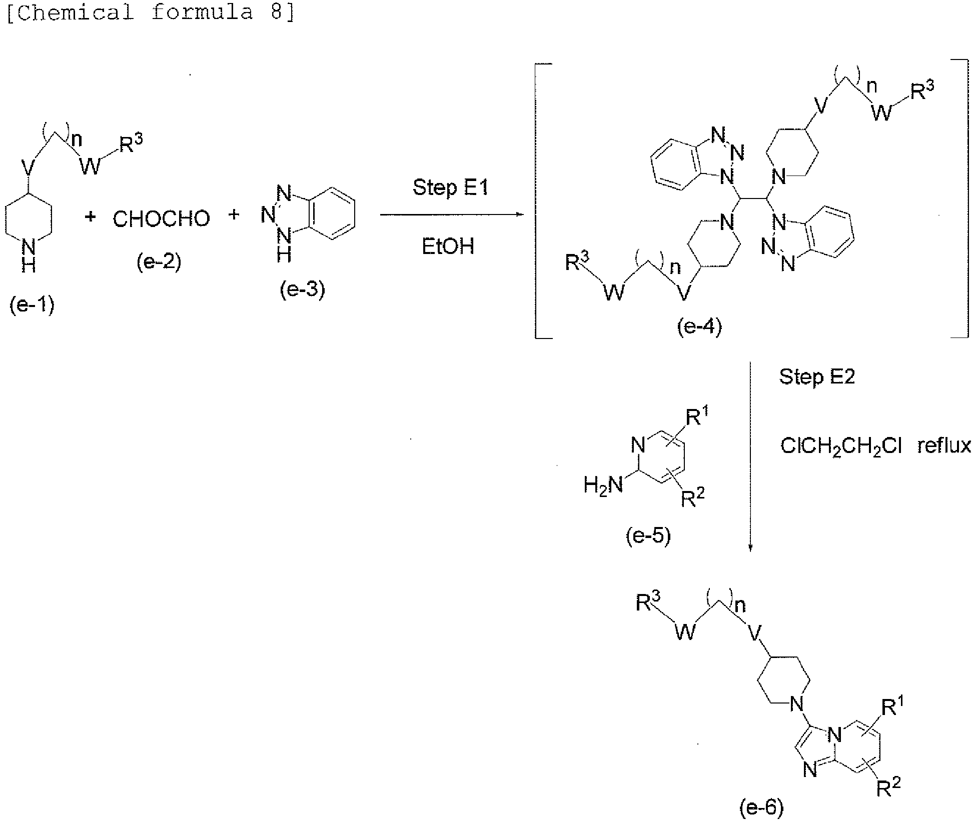 In the above scheme, R¹, R², R³, n, V, and W have the same meanings as above.

Step E1 and E2: This is a step of producing a compound (e-6) from a compound (e-1), which can be performed in accordance with the methods described in Journal of Organic Chemistry, 68, 12, 2003, 4935 to 4937. Step E1 is a step of producing an intermediate (e-4) by allowing a compound (e-1) to react in ethanol at room temperature in the presence of an aqueous solution of benzotriazole (e-3) and glyoxal (e-2). Step E2 is a step of producing a compound (e-6) by heating the intermediate (e-4) and a compound (e-5) in 1,2-dichloroethane.

### (Method F)

Method F is a method for producing a compound of the present invention (f-8), in the case that the main skeletal structure is pyrazolopyridine. 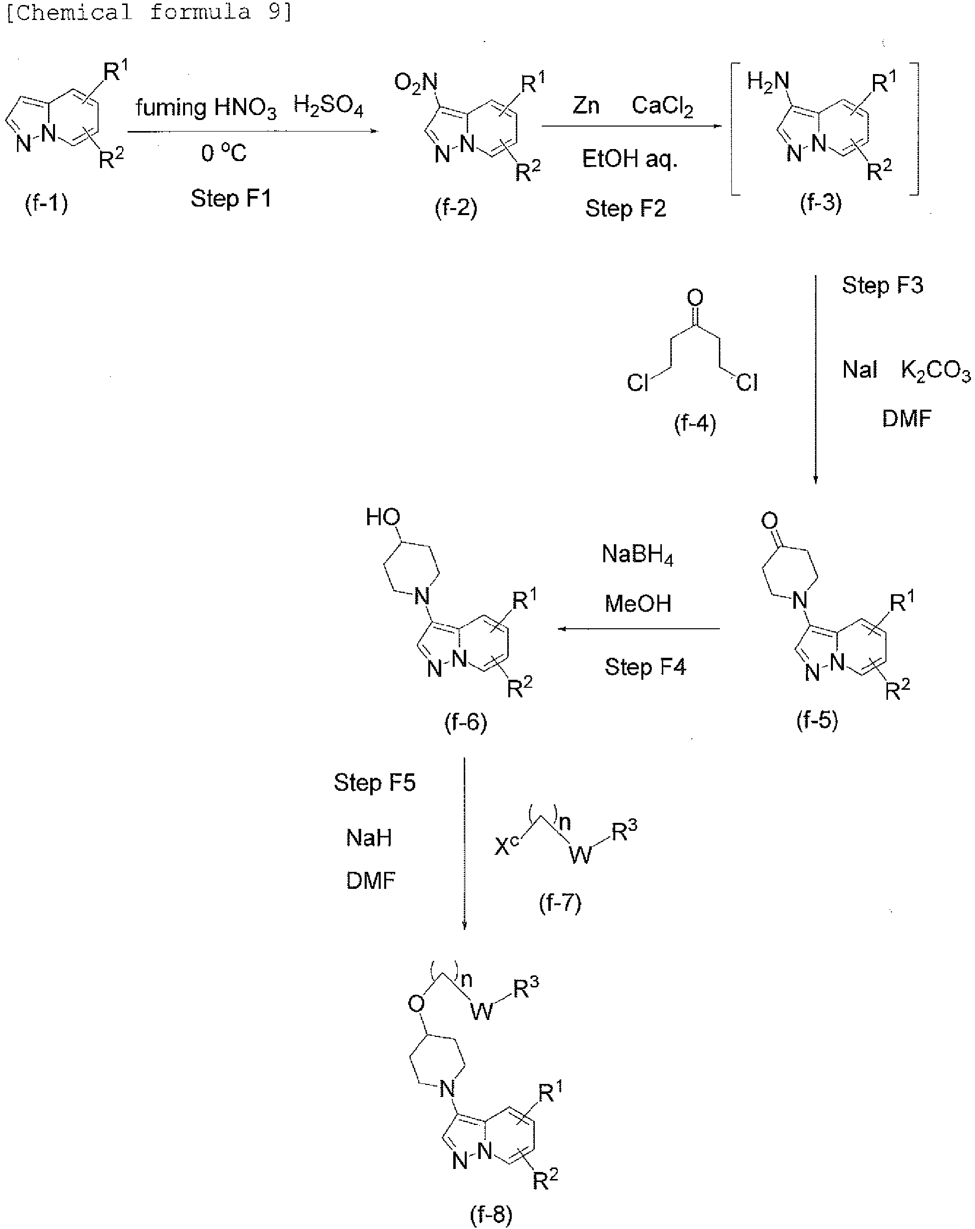 In the above scheme, R¹, R², R³, n, and W have the same meanings as above, and X^{c} represents a leaving group such as a halogen atom or a toluenesulfonyloxy group.

Step F1: This is a step of producing a compound (f-2) from a compound (f-1), which is a so-called nitration reaction of an aromatic ring. This can be done by allowing the compound (f-1) to undergo a reaction in fuming nitric acid and concentrated sulfuric acid while cooling.
Step F2: This is a step of producing a compound (f-3) from the compound (f-2), which is a so-called reduction reaction of an aromatic nitro group. This can be done by allowing the compound (f-2) to undergo a reaction in an aqueous solution of ethanol while heating under reflux in the presence of calcium chloride and zinc.
Step F3: This is a step of producing a compound (f-5) from the compound (f-3), which is a cyclization reaction to convert an aromatic amino group into a piperidine ring. This can be done by allowing the compound (f-3) to undergo a reaction in the presence of 1,5-dichloropentane-3-one (f-4), sodium iodide, and potassium carbonate in N,N-dimethylformamide at room temperature, and also while heating.
Step F4: This is a step of producing a compound (f-6) from the compound (f-5), and is a reduction reaction of a ketone. This can be done by allowing the compound (f-5) to undergo a reaction in the presence of sodium borohydride in methanol while cooling, and also at room temperature.
Step F5: This is a step of producing a compound (f-8) from the compound (f-6), and is a so-called Williamson etherification reaction. This can be done by allowing the compound (f-6) to undergo a reaction in the presence of a compound (f-7) and sodium hydride in N,N-dimethylformamide at room temperature, and also while heating.

### (Method G)

Method G is a method for producing a compound of the present invention (g-4) by removing the protective group of the amino group present in the ring and then performing an acylation or sulfonamidation reaction. 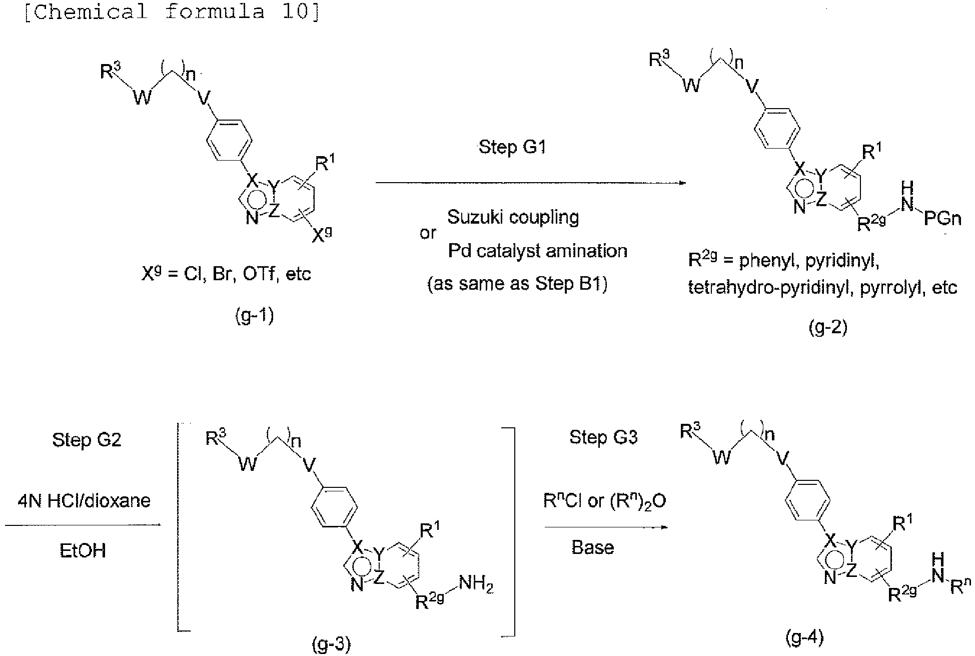 In the above scheme, R¹, R³, X, Y, Z, W, n, and V have the same meanings as above, PGn represents a protective group of an amino group, Rⁿ represents an alkylacyl group or an alkylsulfonyl group, X^{g} represents a leaving group such as a halogen group, and R^{2g} represents a phenyl group or a heterocyclic group such as a pyridyl group, pyrrolyl group, or a tetrahydropyridyl group.

Step G1: This is a step of producing a compound (g-2) by a similar coupling reaction to Step B1 from a compound (g-1), which is producible in accordance with method A. Step G2: This is a step of removing a protective group. This step can be performed under similar conditions to method C.
Step G3: This is a step of producing a compound (g-4) from a compound (g-3). This can be done by allowing an acid anhydride (Rⁿ)₂O, acyl chloride RⁿCl, or sulfonyl chloride RⁿCl to react with the compound (g-3) in the presence of an organic base.

### (Method H)

Method H is a method for producing a compound of the present invention (h-2) by reducing a side chain ester group of a compound (h-1), which is producible in accordance with method A. 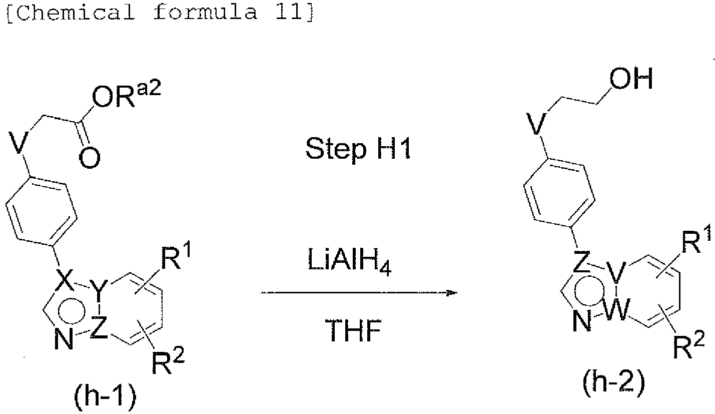 In the above scheme, R¹, R², R^{a2}, X, Y, Z, and V have the same meanings as above.

Step H1: This is a step of producing a compound (h-2) from a compound (h-1), and is a reduction reaction. This step can be done by allowing the compound (h-1) to undergo a reaction in the presence of lithium aluminum hydride in tetrahydrofuran whilst ice-cooling to room temperature.

Method I is a method for producing a compound of the present invention (i-2) by converting an ester group present in the ring of a compound (i-1) into an amide group. 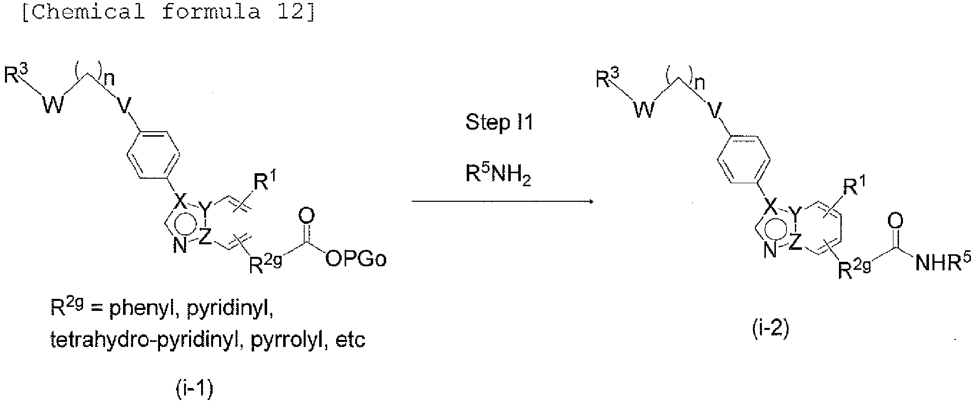 In the above scheme, R¹, R³, R^{2g}, X, Y, Z, W, n, and V have the same meanings as above, PGo represents a protective group of a carboxy group, and R⁵ represents an alkyl group.

Step I1: This is a step of converting a compound (i-1) into a compound (i-2) by an amidation reaction. This amidation reaction may be performed by a method of directly converting an ester group into an amide group or a method of hydrolyzing an ester group and then amidating it by a condensation reaction with an amine. The amidation reaction for directly converting an ester group into an amide group can be performed in accordance with the methods described in, for example, Chem. Rev., 1948, 45, 203, J. Am. Chem. Soc., 1950, 72, 1888, Org. Biol. Chem., 1962, 84, 4457, J. Am. Chem. Soc., 1973, 95, 875, J. Am. Chem. Soc., 1981, 103, 7090, and the like.

A compound of the present invention produced by the aforementioned method can be isolated and purified by a publicly known method, for example, extraction, precipitation, distillation, chromatography, fractional crystallization, and recrystallization.
Also, in the case that the compound having the general formula (I) of the present invention or a production intermediate thereof contains an asymmetric carbon, enantiomers exist. Each of these enantiomers can be isolated and purified by standard methods such as fractional crystallization (salt fractionation) in which an enantiomer is recrystallized with an appropriate salt, and column chromatography. Examples of reference literature for a method of separating an enantiomer from racemates include J. Jacques et al., "Enantiomers, Racemates and Resolution, John Wiley And Sons, Inc."

The compound of the present invention is highly safe and exhibits favorable disposition, and also, has an excellent osteogenesis-promoting action. Hence, the compound of the present invention can be used for the prevention or treatment (particularly, treatment) of diseases associated with bone metabolism such as osteoporosis, Paget's disease of bone, and osteoarthritis, and thus is useful.

When administering a compound of the present invention or a pharmacologically acceptable salt thereof to a mammal (particularly, a human), it can be administered systemically or locally by an oral or parenteral route.

The dosage form of a pharmaceutical composition of the present invention is selected depending on the administration method, and is producible by preparation methods normally employed for various kinds of formulations.
Examples of dosage forms for an oral pharmaceutical composition include a tablet, a pill, a powder, a granule, a capsule, a liquid medicine, a suspension, an emulsion, a syrup, and an elixir. Medicines in these dosage forms can be prepared by standard methods, using any agent appropriately selected as needed from among those normally employed as additives such as an excipient, a binder, a disintegrant, a lubricant, a swelling agent, a swelling aid, a coating agent, a plasticizer, a stabilizer, an antiseptic, an antioxidant, a colorant, a solubilizing aid, a suspending agent, an emulsifier, a sweetener, a preservative, a buffer, a diluent, and a humectant.

Examples of dosage forms for a parenteral pharmaceutical composition include an injection, an ointment, a gel, a cream, a poultice, an aerosol, an inhalant, a spray, an eye drop, a nasal drop, and a suppository. Medicines in these dosage forms can be prepared by standard methods, using any agent appropriately selected as needed from among those normally employed as additives such as a stabilizer, an antiseptic, a solubilizing aid, a humectant, a preservative, an antioxidant, a fragrance, a gelling agent, a neutralizer, a solubilizing aid, a buffer, an isotonic agent, a surfactant, a colorant, a buffer, a viscosity enhancer, a humectant, a filler, an absorption promoter, a suspending agent, and a binder.

The dose of a compound having the general formula (I) or a pharmacologically acceptable salt thereof varies depending on the symptoms, age, body weight, and the kind, dose, etc. of the drug to be administered in combination. However, normally, a compound having the general formula (I) or a pharmacologically acceptable salt thereof is preferably administered in a range of 0.001 to 1000 mg, in terms of the amount of the compound having the general formula (I), per adult (presumed to weigh approximately 60 kg) per dose, systemically or locally, once to several times a month, once to several times a week, or once to several times a day, orally or parenterally, or via the intravenous route continuously for one to 24 hours a day.

Furthermore, other active ingredients can be used in combination with a pharmaceutical composition of the present invention as needed as long as such active ingredient does not impair the efficacy of the present invention.
The present invention also encompasses a method for preventing/treating the aforementioned diseases, comprising administering a compound of the present invention or a pharmacologically acceptable salt thereof.
The present invention further encompasses use of a compound of the present invention or a pharmacologically acceptable salt thereof for the production of the aforementioned pharmaceutical composition.

### (Formulation example 1) Powder

Five grams of a compound of the present invention, 895 g of lactose, and 100 g of corn starch are mixed by a blender to give a powder.

### (Formulation example 2) Granule

Five grams of a compound of the present invention, 865 g of lactose, and 100 g of low-substituted hydroxypropylcellulose are mixed, followed by addition of 300 g of a 10% aqueous solution of hydroxypropylcellulose. The resulting mixture is kneaded and granulated using extrusion granulation equipment, and then dried to give a granule.

### (Formulation example 3) Tablet

Five grams of a compound of the present invention, 90 g of lactose, 34 g of corn starch, 20 g of crystalline cellulose, and 1 g of magnesium stearate are mixed by a blender, followed by tabletting using a tablet machine to give a tablet.

### (Test example)

### (Test example 1) Osteoblast differentiation test

ST2 cells, murine bone marrow-derived stromal cells, (obtained from RIKEN) were used.
In this test, α-MEM media (obtained from GIBCO BRL Cat. No. 10370-021) containing 10% (v/v) of inactivated calf serum (obtained from Hyclone Laboratories, Inc.) and 1% (v/v) of Penicillin-Streptomycin Liquid (obtained from GIBCO BRL Cat. No. 15140-122) (hereinbelow, abbreviated as 10%-FBS-αMEM) were used. In this test, all culturing was performed in a CO₂ incubator (37°C, 95% humidity, 5% CO₂).

The aforementioned cells were detached with 2 mL of a 0.25% trypsin solution (obtained from GIBCO BRL Cat. No. 15050-065) and dispersed in 10 mL of 10%-FBS-αMEM.
Subsequently, the cells were collected by centrifugation (25°C, 800 rpm, five minutes). Then, a cell suspension containing 40000 of the cells/mL of 10%-FBS-αMEM was prepared. The cell suspension was then dispensed into 96-well plates (the product of Falcon), 100 µL per well, at a density of 4000 cells/well, followed by culturing for 24 hours. To the wells, except for the below-described well containing a control group, the compound was dispensed at final concentrations of 0.01, 0.03, 0.1, and 0.3 µg/ml. To the well of a control group, DMSO was dispensed at a final concentration of 0.1% (v/v). After four days of culturing, the activity of alkaline phosphatase (ALP) was measured in each group.

The measurement of ALP activity was performed as follows. That is, the medium in each well of the culture plates was completely removed. Each well was then washed by dispensing 100 µL of Dulbecco's phosphate buffer (obtained from GIBCO BRL Cat. No. 14190-144) and then removing it. A cell lysate solution containing 10 mM MgCl₂ and 2% (v/v) TritonX-100 (Sigma) was prepared and dispensed at 50 µL/well, followed by stirring at room temperature for five minutes. An ALP substrate solution containing 50 mM diethanolamine (Wako Pure Chemical Industries, Ltd., Cat. No. 099-03112) and 20 mM p-nitrophenyl phosphate (Wako Pure Chemical Industries, Ltd., Cat. No. 147-02343) was prepared and dispensed at 50 µL/well, and the plates were left to stand at room temperature for 10 minutes. Subsequently, absorbance was measured by a microplate reader (Bio-Rad Laboratories, Inc.). Setting the measurement value of the control group of each plate at 100%, alkaline phosphatase activity (increase (%)) in the test compound-addition group was calculated, which was assessed as the degree of osteoblast differentiation. (For example, if the absorbance of the plate to which a solvent (0.1% DMSO) is added is 0.2 and the absorbance of the plate of the compound-addition group is 0.4, then the alkaline phosphatase activity is calculated as 0.4 / 0.2 x 100 = 200%, indicating that the activity is increased twofold.)

In this test, the compounds of Examples 3 to 17, 19 to 21, 24 to 28, 30, 31, 34 to 36, 38, 40 to 72, 74 to 84, 86 to 107, 109 to 148, and 150 exhibited alkaline phosphatase activity of 200% or more at 0.1 µg/mL.

### (Test example 2) Osteoclast formation-inhibition test

Eighteen day-old ICR mice are purchased from Japan SLC, Inc. and used in the following experiment. Mice are sacrificed by cervical dislocation, and the left and right femur and the tibia are excised. After removal of surrounding tissues, the femur and the tibia thus excised are minced with scissors. To the femur and the tibia thus minced, 10 mL of 15%-FBS-αMEM is added, followed by stirring for one minute. Subsequently, the supernatant is collected, which is filtered through a cell strainer (Becton, Dickinson and Company). Then, a suspension of 500 thousand cells/mL of 15%-FBS-αMEM was prepared. The cell suspension is then dispensed into 96-well microplates, 100 µL per well, at a density of 50000 cells/well, followed by culturing for 24 hours. Activated vitamin D3 (Sigma, Cat. No. D1530) is dispensed into each well at a final concentration of 20 nM. To the wells, except for the below-described well containing a control group, the compound is dispensed at final concentrations of 0.01, 0.03, 0.1, and 0.3 µg/ml. To the well of a control group, DMSO is dispensed at a final concentration of 0.1% (v/v). After five days of culturing, the activity of tartrate-resistant acid phosphatase (TRAP) is measured in each group.

The measurement of TRAP activity is performed as follows. That is, the medium in each well of the culture plates is completely removed. Each well is then washed by dispensing 100 µL of Dulbecco's phosphate buffer (obtained from GIBCO BRL Cat. No. 14190-144) and then removing it. An acetone:ethanol mixture (1:1) is added to the wells and left for one minute for fixation. The fixation mixture is then removed and staining is performed using a Leukocyte acid phosphatase kit (Sigma, Cat. No. 387-A) at 37°C for 30 minutes. After removing the staining liquid, 100 µL of 10% sodium dodecyl sulfate (Wako Pure Chemical Industries, Ltd. Cat. No. 191-07145) is dispensed, followed by stirring for five minutes.
Subsequently, absorbance is measured by a microplate reader (Bio-Rad Laboratories, Inc.). Setting the measurement value of the control group of each plate at 100%, the decrease (%) in TRAP activity in the test compound-addition group is calculated, which is assessed as the osteoclast formation-inhibiting activity.

### (Test example 3) Effect on bone density

Eight to 12 week old female F344 rats were purchased from Charles River Laboratories and used in the following experiment. Rats were anesthetized with an intraperitoneal administration of 40 mg/kg of Somnopentyl (Kyoritsu Seiyaku Corporation), and then oophorectomy or sham surgery was performed. From the day after surgery, a suspension of the test compound in a 0.5% methyl cellulose solution (Wako Pure Chemical Industries, Ltd. Cat. No. 133-14255) was orally administered once a day, six days a week. Six weeks after administration, the rats were euthanized by removal of whole blood from the lower abdominal aorta under Somnopentyl anesthesia, and the left and right femur was excised.

After removal of soft tissues, the bone density of the femur thus excised was measured by a DXA apparatus, DCS-600R (Aloka Co., Ltd.). The bone density was assessed in the whole femur as well as in three equal sections of the whole femur, namely the proximal end, the shaft, and the distal end.

In this test, the compounds of Examples 24, 89, 91 to 94, 96, and 148 significantly increased the bone density at 10 mg/kg or less.

### Examples

### <Reference Example 1>

### 4-{[Tert-butyl(dimethyl)silyl]oxy}-2-nitroaniline

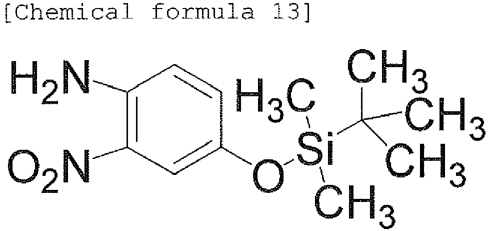

Into dichloromethane (520 mL), 4-amino-3-nitrophenol (19.52 g, 126.7 mmol) and imidazole (13.8 g, 203 mmol) were dissolved. After ice cooling, tertbutyldimethylsilyl chloride (24.82 g , 164.7 mmol) was added, followed by stirring for one hour, and then overnight at room temperature. Chloroform (300 mL) was added, and the resulting organic layer was sequentially washed with a saturated solution of sodium bicarbonate, water, and saturated brine, and the organic layer was dried over anhydrous sodium sulfate. After filtration, the solvent was distilled off under reduced pressure to give the desired title compound (34.25 g, yield 100%).
¹H-NMR (CDCl₃) δ: 0.20 (6H, s), 0.99 (9H, s), 5.81 (2H, br s), 6.71 (1H, d, J = 8.9 Hz), 6.98 (1H, dd, J = 8.9, 2.8 Hz), 7.57 (1H, d, J = 2.8 Hz).

### <Reference Example 2>

### 2-[2-(4-Bromophenoxy)ethoxy]tetrahydro-2H-pyran

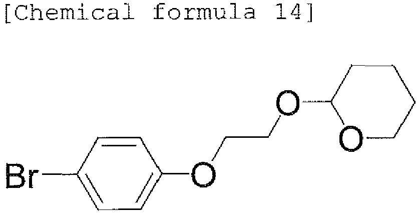

N,N-Dimethylformamide (150 mL) was added to 4-bromophenol (18.2 g, 104 mmol), 2-(2-bromoethoxy)tetrahydro-2H-pyran (26.9 mL, 178 mmol), and potassium carbonate (36.0 g, 260 mmol), followed by stirring at 60°C overnight. The resulting mixture was left to cool, and the solvent was distilled off under reduced pressure. Ethyl acetate and water were added for extraction. The resulting organic layer was washed with water and saturated brine, dried over anhydrous sodium sulfate, and then filtered. The solvent was then distilled off under reduced pressure and the residue thus obtained was purified by silica gel chromatography (hexane : ethyl acetate, 90 : 10 - 30 : 70, V/V) to give the desired title compound (28.86 g, yield 86%).
¹H-NMR (CDCl₃) δ: 1.47-1.67 (4H, m), 1.69-1.89 (2H, m), 3.49-3.56 (1H, m), 3.77-3.84 (1H, m), 3.85-3.92 (1H, m), 4.00-4.07 (1H, m), 4.07-4.17 (2H, m), 4.67-4.72 (1H, m), 6.79-6.84 (2H, m), 7.33-7.39 (2H, m).

### <Reference Example 3>

### 2-(Tetrahydro-2H-pyran-4-yloxy)ethanol

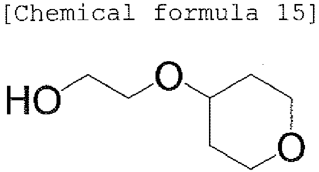

### (3a) 1,4,8-Trioxaspiro[4.5]decane

Tetrahydro-4H-pyran (6.00 g, 60.0 mmol) was dissolved in benzene (120 mL), to which ethylene glycol (11.2 g, 180 mmol) and p-toluenesulfonic acid monohydrate (1.14 g, 6.00 mmol) were added, and the resulting mixture was refluxed for two hours while removing water generated using a Dean-Stark tube. To the resulting reaction liquid, a saturated aqueous solution of sodium bicarbonate was added, followed by extraction with ethyl acetate. The resulting organic layer was washed with saturated brine and dried over anhydrous sodium sulfate. After filtration, the solvent was distilled off under reduced pressure and the residue thus obtained was purified by basic silica gel column chromatography (hexane : ethyl acetate, 100 : 0 - 0 : 100, V/V) to give the desired title compound (7.79 g, yield 90%).
¹H-NMR (CDCl₃) δ: 1.74 (4H, t, J = 5.5 Hz), 3.74-3.80 (4H, m), 3.98 (4H, s).

### (3b) 2-(Tetrahydro-2H-pyran-4-yloxy)ethanol

Into tetrahydrofuran (1.0 mL), 1,4,8-trioxaspiro[4.5]decane (0.43 g, 3.0 mmol) produced in Reference Example 3 (3a) was dissolved, and the resulting mixture was cooled to -50°C. Subsequently, a 1.0 M solution of borane-tetrahydrofuran complex in tetrahydrofuran (3.6 mL) and trimethylsilyl trifluoromethanesulfonate (33 mg, 0.15 mmol) were added. The mixture was warmed to room temperature, followed by stirring for 18 hours. After adding a small amount of water, the mixture was purified by basic silica gel column chromatography (hexane : ethyl acetate, 100 : 0 - 0 : 100, V/V) to give the desired title compound (0.39 g, yield 88%).
¹H-NMR (CDCl₃) δ: 1.53-1.65 (2H, m), 1.87-1.95 (2H, m), 2.01 (1H, t, J = 6.3 Hz), 3.40-3.48 (2H, m), 3.50-3.57 (1H, m), 3.57-3.61 (2H, m), 3.71-3.77 (2H, m), 3.92-3.99 (2H, m).

### <Reference Example 4>

### 4-{2-[4-(4,4,5,5-Tetramethyl-1,3,2-dioxaborolan-2-yl)phenoxy]ethoxy}tetrahydro-2H-pyran

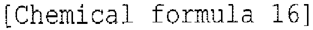

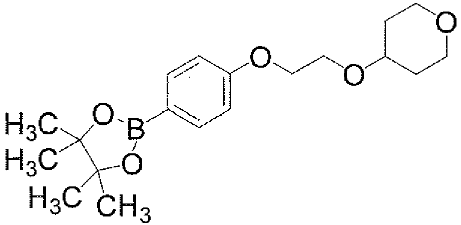

Using 4-(4,4,5,5-tetramethyl-1,3,2-dioxaborolan-2-yl)phenol (16.5 g, 74.9 mmol) and 2-(tetrahydro-2H-pyran-4-yloxy)ethanol (13.19 g, 90.23 mmol) synthesized in Reference Example 3, the desired title compound (16.2 g, yield 56%) was obtained by the same method as in Example 12 (12a).
¹H-NMR (CDCl₃) δ: 1.33 (12H, s), 1.57-1.68 (2H, m), 1.88-1.97 (2H, m), 3.40-3.48 (2H, m), 3.55-3.63 (1H, m), 3.83 (2H, t, J = 5.0 Hz), 3.92-3.99 (2H, m), 4.15 (2H, t, J = 5.0 Hz), 6.91 (2H, d, J = 8.7 Hz), 7.74 (2H, d, J = 8.7 Hz).

### <Reference Example 5>

### 4-[2-(Tetrahydro-2H-pyran-4-yloxy)ethoxy]piperidine

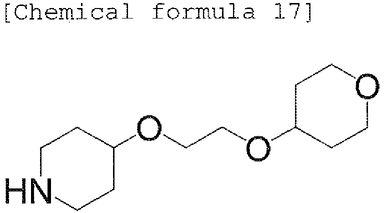

### (5a) Benzyl 4-[2-(tetrahydro-2H-pyran-2-yloxy)ethoxy]piperidine-1-carboxylate

Into N,N-dimethylformamide (500 mL), 2-{tetrahydro-2H-pyran-2-yloxy)ethyl 4-methylbenzenesulfonate (49.8 g, 166 mmol) was dissolved, to which benzyl 4-hydroxy-1-piperidinecarboxylate (31.1 g, 132 mmol) was added. Further, sodium hydride (content 55%) (7.23 g, 166 mmol) was added all at once, and the resulting mixture was stirred at room temperature for two hours under a nitrogen atmosphere. To the reaction liquid, dichloromethane and water were added for extraction, and the resulting aqueous layer was further extracted with dichloromethane. The organic layer thus obtained was dried over anhydrous sodium sulfate. After filtration, the solvent was distilled off under reduced pressure and the residue thus obtained was purified by silica gel column chromatography (hexane : ethyl acetate, 100 : 0 - 50 : 50, V/V) to give the desired title compound (45.6 g, yield 95%).
¹H-NMR (CDCl₃) δ: 1.49-1.62 (6H, m), 1.69-1.75 (1H, m), 1.78-1.88 (3H, m), 3.20-3.25 (2H, m), 3.45-3.66 (5H, m), 3.74-3.90 (4H, m), 4.64 (1H, t, J = 3.7 Hz), 5.12 (2H, s), 7.29-7.38 (5H, m).

### (5b) Benzyl 4-(2-hydroxyethoxy)piperidine-1-carboxylate

Benzyl 4- [2- (tetrahydro-2H-pyran-2-yloxy)ethoxy]piperidine-1-carboxylate (45.6 g, 125 mmol) produced in Reference Example 5 (5a) was dissolved in methanol, to which p-toluenesulfonic acid monohydrate (11.9 g, 62.7 mmol) was added, followed by stirring at room temperature for 2.5 hours. Sodium bicarbonate (powder) was added to the reaction liquid and the solvent was distilled off under reduced pressure. A solid precipitated, which was filtered off while washing with ethyl acetate. The filtrate was then concentrated under reduced pressure. The residue thus obtained was purified by silica gel column chromatography (hexane : ethyl acetate, 60 : 40 - 0 : 100, V/V) to give the desired title compound (29.1 g, yield 83%).
¹H-NMR (CDCl₃) δ: 1.52-1.60 (2H, m), 1.82-1.90 (2H, m), 1.98 (1H, t, J = 6.0 Hz), 3.18-3.24 (2H, m), 3.50-3.55 (1H, m), 3.58 (3H, t, J = 4.6 Hz), 3.73 (2H, dt, J = 6.0, 4.6 Hz), 3.79-3.86 (2H, m), 5.13 (2H, s), 7.29-7.38 (5H, m) .

### (5c) Benzyl 4-(2-{[(4-methylphenyl)sulfonyl]oxy}ethoxy)piperidine-1-carboxylate

Into dichloromethane (360 mL), benzyl 4-(2-hydroxyethoxy)piperidine-1-carboxylate (20.0 g, 71.6 mmol) produced in Reference Example 5 (5b) was dissolved, to which N,N-dimethylaminopyridine (875 mg, 7.16 mmol) and triethylamine (20.0 mL, 143 mmol) were added. Further, p-toluenesulfonyl chloride (17.7 g, 93.1 mmol) was gradually added, followed by stirring at room temperature for 2.5 hours. To the reaction liquid, water was added, followed by extraction with dichloromethane. The resulting organic layer was dried over anhydrous magnesium sulfate. After filtration, the solvent was distilled off under reduced pressure, and the residue thus obtained was purified by silica gel column chromatography (hexane : ethyl acetate, 100 : 0 - 50 : 50, V/V) to give the desired title compound (30.7 g, yield 99%) .
¹H-NMR (CDCl₃) δ: 1.44-1.51 (2H, m), 1.70-1.77 (2H, m), 2.44 (3H, s), 3.19-3.24 (2H, m), 3.44-3.48 (1H, m), 3.65 (2H, t, J = 4.7 Hz), 3.66-3.70 (2H, m), 4.15 (2H, t, J = 4.7 Hz), 5.12 (2H, s), 7.30-7.38 (7H, m), 7.79 (2H, d, J = 8.6 Hz).

### (5d) 4-[2-(Tetrahydro-2H-pyran-4-yloxy)ethoxy]piperidine-1-carboxylate

Into N,N-dimethylformamide (10mL), benzyl 4-(2-{[(4-methylphenyl) sulfonyl] oxy} ethoxy) piperidine-1-carboxylate (1.64 g, 3.79 mmol) produced in Reference Example 5 (5c) was dissolved, to which tetrahydro-2H-pyran-4-ol (540 µ.L, 5.69 mmol) and sodium hydride (content 55%) (248 mg, 5.69 mmol) were added, followed by stirring at room temperature for 20 hours. To the reaction liquid, water and ethyl acetate were added for extraction, and the resulting organic layer was sequentially washed with water and saturated brine, and then dried over anhydrous sodium sulfate. After filtration, the solvent was distilled off under reduced pressure and the residue thus obtained was purified by silica gel column chromatography (ethyl acetate : hexane, 0 : 100 - 40 : 60, V/V) to give the desired title compound (510 mg, yield 37%).
¹H-NMR (CDCl₃) δ: 1.58-1.60 (4H, m), 1.86-1.89 (4H, m), 3.20-3.23 (2H, m), 3.40-3.46 (2H, m), 3.51-3.53 (2H, m), 3.60-3.63 (4H, m), 3.80-3.82 (2H, m), 3.92-3.94 (2H, m), 5.12 (2H, s), 7.34-7.35 (5H, m).

### (5e) 4-[2-(Tetrahydro-2H-pyran-4-yloxy)ethoxy]piperidine

Into methanol (15 mL), benzyl 4-[2-(tetrahydro-2H-pyran-4-yloxy)ethoxy]piperidine-1-carboxylate (505 mg, 1.39 mmol) produced in Reference Example 5 (5d) was dissolved, and under a nitrogen atmosphere, 10% palladium hydroxide (50 mg) was added, and hydrogen substitution was performed three times. Under a hydrogen atmosphere, the mixture was stirred at room temperature for 18 hours. The reaction liquid was filtered through Celite and the solvent was distilled off under reduced pressure to give the desired title compound (318 mg, yield 100%).
¹H-NMR (CDCl₃) δ: 1.43-1.52 (2H, m), 1.58-1.61 (2H, m), 1.85-1.96 (4H, m), 2.61-2.67 (2H, m), 3.09-3.11 (2H, m), 3.40-3.46 (3H, m), 3.50-3.57 (1H, m), 3.61-3.62 (4H, m), 3.93-3.96 (2H, m).

### <Reference Example 6>

### 2-[(3R)-Tetrahydrofuran-3-yloxy]ethanol

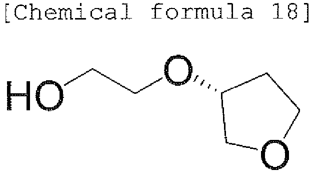

### (6a) (3R)-3-[2-(Benzyloxy)ethoxy]tetrahydrofuran

Using 2-(benzyloxy)ethyl 4-methylbenzenesulfonate (7.00 g, 22.8 mmol) and (3R)-tetrahydrofuran-3-ol (2.2 mL, 27.4 mmol), the desired title compound (4.23 g, yield 83%) was obtained by the same method as in Reference Example 5 (5a).
¹H-NMR (CDC1₃) δ: 1.94-2.05 (2H, m), 3.57-3.65 (4H, m), 3.77-3.94 (4H, m), 4.14-4.19 (1H, m), 4.57 (2H, s), 7.26-7.36 (5H, m).

### (6b) 2-[(3R)-Tetrahydrofuran-3-yloxy]ethanol

Using (3R)-3-[2-(benzyloxy)ethoxy]tetrahydrofuran (4.21 g, 19.0 mmol) produced in Reference Example 6 (6a), the desired title compound (2.41 g, yield 96%) was obtained by the same method as in Reference Example 5 (5e).
¹H-NMR (CDC1₃) δ: 1.98-2.04 (2H, m), 2.24 (1H, br s), 3.49-3.59 (2H, m), 3.69-3.95 (6H, m), 4.14-4.19 (1H, m).

### <Reference Example 7>

### Tert-butyl 4-(2-hydroxyethoxy)benzoate

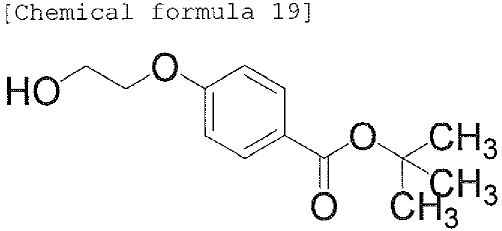

### (7a) Tert-butyl 4-hydroxybenzoate

Into tert-butyl alcohol (200 mL), 4-hydroxybenzoate (10.0 g, 72.4 mmol) and 4-dimethylaminopyridine (354 mg, 2.90 mmol) were dissolved, to which 1-methyl-3-(3-dimethylaminopropyl)carbodiimide hydrochloride (15.3 g, 79.6 mmol) was added, followed by stirring overnight under a nitrogen atmosphere. The resulting reaction liquid was concentrated under reduced pressure, to which ethyl acetate and hexane were added, followed by decantation. The resulting liquid was concentrated under reduced pressure again, and the residue thus obtained was purified by silica gel column chromatography (hexane : ethyl acetate, 100 : 0 - 70 : 30, V/V) to give the desired title compound (5.48 g, yield 39%).
¹H-NMR (CDCl₃) δ: 1.59 (9H, s), 6.18 (1H, s), 6.85 (2H, d, J = 8.3 Hz), 7.90 (2H, d, J = 8.3 Hz) .

### (7b) Tert-butyl 4-[2-(benzyloxy)ethoxy]benzoate

Using tert-butyl 4-hydroxybenzoate (2.00 g, 10.3 mmol) synthesized in Reference Example 7 (7a) and [(3-bromoethoxy)methyl]benzene (2.44 mL, 15.5 mmol), the desired title compound (2.49 g, yield 74%) was obtained by the same method as in Reference Example 12 (12a). ¹H-NMR (CDCl₃) δ: 1.58 (9H, s), 3.84 (2H, t, J = 4.8 Hz), 4.19 (2H, t, J = 4.8 Hz), 4.64 (2H, s), 6.91 (2H, d, J = 8.7 Hz), 7.27-7.38 (5H, m), 7.93 (2H, d, J = 8.7 Hz).

### (7c) Tert-butyl 4-(2-hydroxyethoxy)benzoate

Using tert-butyl 4-[2-(benzyloxy)ethoxy]benzoate (2.49 g, 7.58 mmol) synthesized in Reference Example 7 (7b), the desired title compound (1.81 g, yield 100%) was obtained by the same method as in Reference Example 5 (5e).
¹H-NMR (CDCl₃) δ: 1.58 (9H, s), 2.00 (1H, t, J = 6.2 Hz), 3.97-4.02 (2H, m), 4.13 (2H, t, J = 4.4 Hz), 6.92 (2H, d, J = 9.2 Hz), 7.94 (2H, d, J = 9.2 Hz).

### <Reference Example 8>

### (2S)-2-({2-[4-(4,4,5,5-Tetramethyl-1,3,2-dioxaborolan-2-yl)phenoxy]ethoxy}methyl}-1,4-dioxane

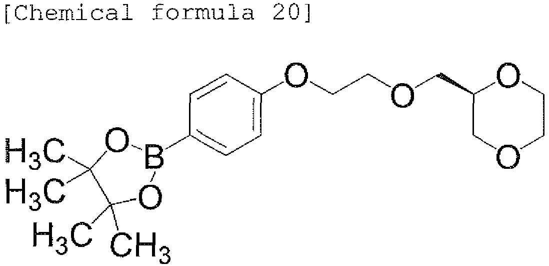

### (8a) 2-{2-[4-(4,4,5,5-Tetramethyl-1,3,2-dioxaborolan-2-yl)phenoxy]ethoxy}tetrahydro-2H-pyran

Into N,N-dimethylformamide (250 mL), 4-(4,4,5,5-tetramethyl-1,3,2-dioxaborolan-2-yl)phenol (10.0 g, 45.5 mmol) and 2-(2-bromoethoxy)tetrahydro-2H-pyran (10.0 g, 45.5 mmol) were dissolved. While ice cooling, sodium hydride (content 55%) (2.98 g, 68.3 mmol) was added. The resulting mixture was stirred for three hours while ice cooling, and for another 17 hours at room temperature. Ethyl acetate was then added, and excess sodium hydride was neutralized with 1M hydrochloric acid. Further, water was added for extraction. The resulting organic layer was washed with saturated brine and dried over anhydrous sodium sulfate. The solvent was then distilled off under reduced pressure. The residue thus obtained was purified by silica gel column chromatography (hexane : ethyl acetate, 100 : 0 - 70 : 30, V/V) to give the desired title compound (13.3 g, yield 84%).
¹H-NMR (CDCl₃) δ: 1.33 (12H, s), 1.47-1.68 (2H, m), 1.69-1.79 (2H, m), 1.79-1.91 (2H, m), 3.48-3.57 (1H, m), 3.79-3.85 (1H, m), 3.87-3.93 (1H, m), 4.01-4.10 (1H, m), 4.15-4.22 (2H, m), 4.71 (1H, t, J = 3.5 Hz), 6.92 (2H, dt, J = 9.0, 2.1 Hz), 7.74 (2H, dt, J = 8.8, 2.1 Hz).

### (8b) 2-[4-(4,4,5,5-Tetramethyl-1,3,2-dioxaborolan-2-yl)phenoxy]ethanol

Using 2-{2-[4-(4,4,5,5-tetramethyl-1,3,2-dioxaborolan-2-yl)phenoxy]ethoxy}tetrahydro-2H-pyran (13.0 g, 37.3 mmol) synthesized in Reference Example 8 (8a), the desired title compound (9.45 g, yield 96%) was obtained by the same method as in Reference Example 5 (5b).
¹H-NMR (CDCl₃) δ: 1.33 (12H, s), 3.97 (2H, t, J = 4.5 Hz), 4.11 (2H, t, J = 4.5 Hz), 6.91 (2H, dt, J = 8.8, 2.1 Hz), 7.75 (2H, dt, J = 8.8, 2.1 Hz).

### (8c) 2-[4-(4,4,5,5-Tetramethyl-1,3,2-dioxaborolan-2-yl)phenoxy]ethyl benzenesulfonate

Into dichloromethane (100 mL), 2-[4-(4,4,5,5-tetramethyl-1,3,2-dioxaborolan-2-yl)phenoxy]ethanol (4.41 g, 16.7 mmol) synthesized in Example 8 (8b) was dissolved, to which triethylamine (4.62 mL, 33.2 mmol), 4-dimethylaminopyridine (400 mg, 3.27 mmol), and p-toluenesulfonyl chloride (4.80 g, 25.2 mmol) were sequentially added while ice cooling. The resulting mixture was warmed back to room temperature and stirred for a further two hours. The resulting reaction liquid was sequentially washed with water and saturated brine, and then dried over anhydrous sodium sulfate. The solvent was then distilled off under reduced pressure. The residue thus obtained was purified by silica gel column chromatography (hexane : ethyl acetate, 100 : 0 - 70 : 30, V/V) to give the desired title compound (6.1 g, yield 88%).
¹H-NMR (CDCl₃) δ: 1.33 (12H, s), 2.44 (3H, s), 4.16 (2H, t, J = 4.7 Hz), 4.37 (2H, t, J = 4.7 Hz), 6.75 (2H, d, J = 8.4 Hz), 7.33 (2H, d, J = 8.4 Hz), 7.70 (2H, d, J = 8.4 Hz), 7.81 (2H, d, J = 8.4 Hz).

### (8d) (2S)-2-({2-[4-(4,4,5,5-Tetramethyl-1,3,2-dioxaborolan-2-yl)phenoxy]ethoxy}methyl)-1,4-dioxane

Using (2R)-1,4-dioxan-2-yl methanol (4.99 g, 42.4 mmol) and 2-[4-(4,4,5,5-tetramethyl-1,3,2-dioxaborolan-2-yl)phenoxy]ethyl benzenesulfonate (16.7 g, 39.9 mmol) synthesized in Reference Example 8 (8c), the desired title compound (3.68 g, yield 25%) was obtained by the same method as in Reference Example 5 (5d).
¹H-NMR (CDCl₃) 5: 1.33 (12H, s), 3.39-3.46 (1H, m), 3.50-3.86 (10H, m), 4.15 (2H, t, J = 4.8 Hz), 6.88-6.91 (2H, m), 7.72-7.75 (2H, m).

In the Examples to be described below,
Examples 24 and 25 were produced by method A,
Examples 3 to 5, 7 to 13, 15, and 16 were produced by method C,
Examples 19 to 23 were produced by method D,
Example 18 was produced by method F,
Example 14 was produced by method G,
Examples 1 and 2 were produced by method H, and
Example 17 was produced by method I.

### <Example 1>

### 2-[4-(1H-Benzimidazol-1-yl)phenoxy]ethanol

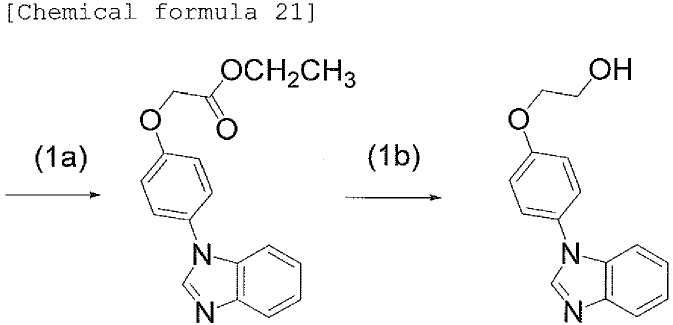

### (1a) Ethyl [4-(1H-benzimidazol-1-yl)phenoxy]acetate

To benzimidazole (495 mg, 4.19 mmol), [4-(2-ethoxy-2-oxoethoxy)phenyl]boronic acid (1.41g, 6.33 mmol), copper(II) acetate (1.15 g, 6.33 mmol), pyridine (683 mL, 8.44 mmol), and molecular sieve 4A (4 g), dichloromethane (80 mL) was added, followed by stirring overnight at room temperature. The resulting mixture was filtered while washing with dichloromethane, and the solvent was then distilled off under reduced pressure. The residue thus obtained was purified by basic silica gel column chromatography (hexane : ethyl acetate, 30 : 70 - 0 : 100, V/V) to give the desired title compound (672 mg, yield 54%).
¹H-NMR (CDCl₃) δ: 1.33 (3H, t, J = 7.2 Hz), 4.31 (2H, q, J = 7.2 Hz), 4.71 (2H, s), 7.06-7.12 (2H, m), 7.29-7.36 (2H, m), 7.41-7.48 (3H, m), 7.84-7.90 (1H, m), 8.05 (1H, s).

### (1b) 2-[4-(1H-Benzimidazol-1-yl)phenoxy]ethanol

Into tetrahydrofuran (9 mL), ethyl [4-(1H-benzimidazol-1-yl)phenoxy]acetate (227.2 mg, 0.767 mmol) produced in Example 1 (1a) was dissolved, and the resulting mixture was cooled to 0°C under a nitrogen atmosphere. Lithium aluminum hydride (59.1 mg, 1.56 mmol) was added, and the mixture was stirred for one hour, and then overnight at room temperature. To the resulting reaction liquid, water (60 µL), a 1N aqueous solution of sodium hydroxide (60 µL), and water (180 µL) were sequentially added dropwise. Anhydrous sodium sulfate was then added and the mixture was stirred for a while, followed by filtration through Celite. The solvent was then distilled off under reduced pressure, and the residue thus obtained was purified by basic silica gel column chromatography (hexane : ethyl acetate, 30 : 70 - 0 : 100, V/V, ethyl acetate : methanol, 100 : 0 - 95 : 5, V/V) to give the desired title compound (161 mg, yield 83%).
¹H-NMR (CDCl₃) δ: 2.45 (1H, br s), 4.04 (2H, t, J = 4.3 Hz), 4.18 (2H, t, J = 4.3 Hz), 7.10 (2H, d, J = 8.8 Hz), 7.29-7.37 (2H, m), 7.37-7.49 (3H, m), 7.83-7.90 (1H, m), 8.05 (1H, s).

### <Example 2>

### 2-[4-(7-Chloroimidazo[1,2-a]pyridin-3-yl)phenoxy]ethanol

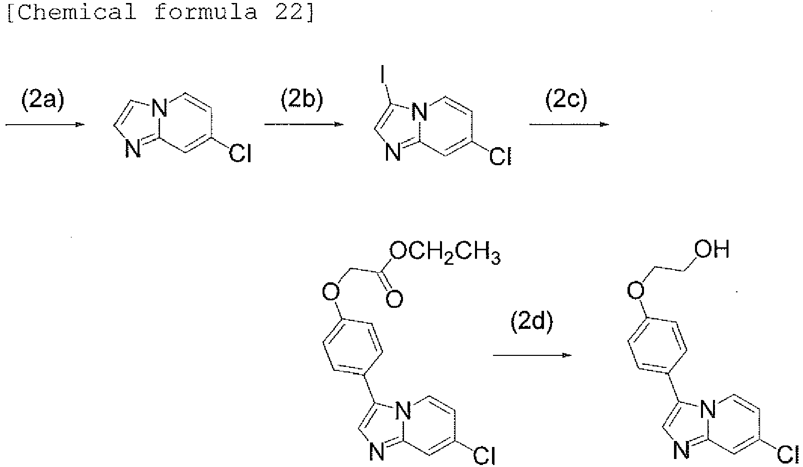

### (2a) 7-Chloroimidazo[1,2-a]pyridine

Into ethanol (50 mL), 2-amino-4-chloropyridine (643 mg, 5.00 mmol) was dissolved, to which a 40% aqueous solution of chloroacetaldehyde (8.25 mL, 50 mmol) was added, followed by heating under reflux for two hours. The resulting mixture was left to cool, and the solvent was distilled off under reduced pressure. The residue thus obtained was purified by basic silica gel column chromatography (dichloromethane) to give the desired title compound (645 mg, yield 85%).
¹H-NMR (CDCl₃) δ: 6.79 (1H, dd, J = 7.4, 2.3 Hz), 7.57 (1H, s), 7.63 (2H, br s), 8.05 (1H, d, J = 7.4 Hz).

### (2b) 7-Chloro-3-iodoimidazo[1,2-a]pyridine

Into acetonitrile (40 mL), 7-chloroimidazo[1,2-a]pyridine (635 mg, 4.16 mmol) produced in Example 2 (2a) was dissolved, to which N-iodosuccinimide (936 mg, 4.16 mmol) was added, followed by stirring at room temperature for three hours. A solid precipitated, which was collected by filtration and purified by basic silica gel column chromatography (dichloromethane : ethyl acetate = 4 : 1, V/V) to give the desired title compound (436 mg, yield 38%).
¹H-NMR (CDCl₃) 5: 6.92 (1H, dd, J = 7.3, 1.8 Hz), 7.63 (1H, d, J = 1.8 Hz), 7.69 (1H, s), 8.06 (1H, d, J = 7.3 Hz).

### (2c) Ethyl [4-(7-chloroimidazo[1,2-a]pyridin-3-yl)phenoxy]acetate

N,N-Dimethylformamide (5 mL) was added to 7-chloro-3-iodoimidazo[1,2-a]pyridine (436 mg, 1.57 mmol) synthesized in Example 2 (2b), [4-(2-ethoxy-2-oxoethoxy)phenyl]boronic acid (386 mg, 1.72 mmol), [1,1"-bis(diphenylphosphino)ferrocene]dichloropalladium(II) complex with dichloromethane (128 mg, 0.157 mmol), and potassium carbonate (649 mg, 4.70 mmol), followed by stirring at 100°C for 2.5 hours under an argon atmosphere. The resulting mixture was left to cool, to which ethyl acetate and water were added. Insoluble matters were filtered off and the resulting solution was subjected to extraction. The resulting organic layer was dried over anhydrous sodium sulfate. After filtration, the solvent was distilled off under reduced pressure, and the residue thus obtained was purified by basic silica gel column chromatography (hexane : ethyl acetate = 2 : 1, V/V) to give the desired title compound (260 mg, yield 50%).
¹H-NMR (CDCl₃) δ: 1.33 (3H, t, J = 7.3 Hz), 4.30 (2H, q, J = 7.3 Hz), 4.70 (2H, s), 6.78 (1H, dd, J = 7.3, 1.8 Hz), 7.06 (2H, d, J = 8.0 Hz), 7.45 (2H, d, J = 8.0 Hz), 7.61 (1H, s), 7.64-7.65 (1H, m), 8.16 (1H, d, J = 7.3 Hz).

### (2d) 2-[4-(7-Chloroimidazo[1,2-a]pyridin-3-yl)phenoxy]ethanol

Using ethyl [4-(7-chloroimidazo[1,2-a]pyridin-3-yl)phenoxy]acetate produced in Example 2 (2c), the desired title compound was obtained by the same method as in Example 1 (1b).
¹H-NMR (CDCl₃) δ: 2.34 (1H, br s), 4.03 (2H, t, J = 4.4 Hz), 4.17 (2H, t, J = 4.4 Hz), 6.79 (1H, dd, J = 7.3, 1.8 Hz), 7.05-7.09 (2H, m), 7.43-7.46 (2H, m), 7.61 (1H, s), 7.66 (1H, d, J = 1.8 Hz), 8.16 (1H, d, J = 7.3 Hz).

### <Example 3>

### 2-(2-{[4-(1H-Benzimidazol-1-yl)phenyl]amino}ethoxy)ethanol

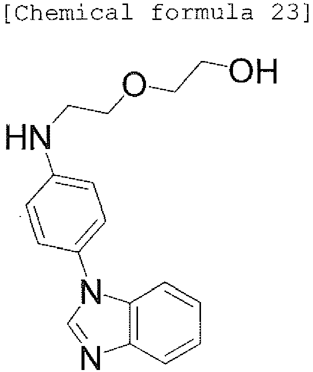

Toluene (6 mL) was added to 1-(4-bromophenyl)-1H-benzimidazole (152 mg, 555 µmol) produced by the same method as in Example 1 (1a), 2-(2-aminoethoxy)ethanol (82.6 µL , 0.82 mmol), tris(dibenzylideneacetone)dipalladium (25 mg, 27 µmol), 2-dicyclohexyl-2'-(N,N-dimethylamino)biphenyl (31.8 mg, 80.5 µmol), and sodium tert-butoxide (132 mg, 1.37 mmol), followed by stirring at 110°C for 21 hours under a nitrogen atmosphere. After the reaction, the resulting product was immediately ice cooled, to which chloroform was added and insoluble matters were filtered off through Celite. The solvent was then distilled off under reduced pressure, and the residue thus obtained was purified by basic silica gel chromatography (hexane : ethyl acetate, 30 : 70 - 0 : 100, V/V, ethyl acetate : methanol, 100 : 0 - 95 : 5, V/V) to give the desired title compound (70 mg, yield 42%).
¹H-NMR (CDCl₃) δ: 3.37-3.43 (2H, m), 3.64-3.68 (2H, m), 3.76-3.85 (4H, m), 4.32 (1H, br s), 6.74-6.80 (2H, m), 7.27-7.36 (4H, m), 7.42-7.47 (1H, m), 7.83-7.89 (1H, m), 8.03 (1H, s).

### <Example 4>

### 2-{[4-(6-Chloro-1H-benzimidazol-1-yl)phenyl]amino}ethanol hydrochloride

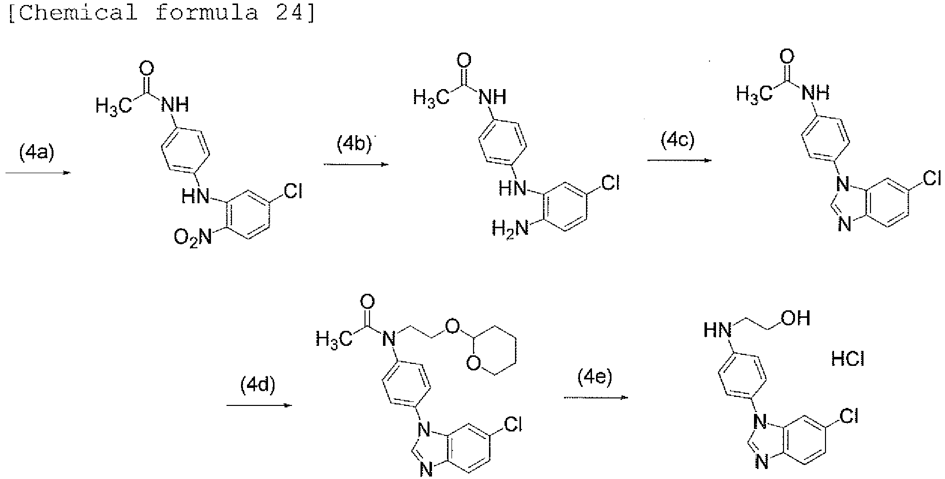

### (4a) N-{4-[(5-Chloro-2-nitrophenyl)amino]phenyl}acetamide

Into ethanol (5 mL), 3,4-dinitrochlorobenzene (500 mg, 2.47 mmol) was dissolved, to which 4-aminoacetanilide (1.11 g, 7.41 mmol) was added, followed by stirring at 50°C for 15 hours. A yellow solid precipitated, which was collected by filtration, sequentially washed with ethanol and 3N hydrochloric acid, and then dried under reduced pressure to give the desired title compound (456.6 mg, yield 60%).
¹H-NMR (CDCl₃) 5: 2.22 (3H, s), 6.71 (1H, dd, J = 9.2, 2.1 Hz), 7.06 (1H, d, J = 2.1 Hz), 7.22-7.25 (3H, m), 7.60 (2H, d, J = 8.7 Hz), 8.15 (1H, d, J = 9.2 Hz), 9.49 (1H, br s).

### (4b) N-{4-[(2-Amino-5-chlorophenyl)amino]phenyl}acetamide

Into a mixed solvent of ethanol (8 mL), tetrahydrofuran (2 mL), and water (2 mL), N-{4-[(5-chloro-2-nitrophenyl)amino]phenyl}acetamide (457 mg, 1.49 mmol) produced in Example 4 (4a) was dissolved, to which ammonium chloride (120 mg, 2.24 mmol) and iron powder (832 mg, 14.9 mmol) were added, followed by stirring at room temperature for 15 hours. The reaction liquid was filtered through Celite while washing with ethanol. The solvent was then distilled off under reduced pressure and the residue thus obtained was purified by basic silica gel chromatography (hexane : ethyl acetate, 95 : 5 - 0 : 100, V/V) to give the desired title compound (303.3 mg, yield 74%).
¹H-NMR (CDCl₃) δ: 2.16 (3H, s), 3. 68 (2H, br s), 5.16 (1H, br s), 6.71 (1H, d, J = 8.5 Hz), 6.75-6.79 (2H, m), 6.91 (1H, dd, J = 8.5, 2.3 Hz), 7.06-7.08 (2H, m), 7.33-7.37 (2H, m).

### (4c) N-[4-(6-Chloro-1H-benzimidazol-1-yl)phenyl]acetamide

Into formic acid (6 mL), N-{4-[(2-amino-5-chlorophenyl)amino]phenyl}acetamide (303 mg, 1.10 mmol) produced in Example 4 (4b) was dissolved, followed by stirring at 100°C for two hours. The resulting reaction liquid was left to cool and then neutralized with a saturated aqueous solution of sodium bicarbonate, followed by extraction with ethyl acetate. The resulting organic layer was washed with saturated brine and dried over anhydrous sodium sulfate. The solvent was then distilled off under reduced pressure. A solid precipitated, which was washed with a mixed solution of ethyl acetate / hexane (1 : 20) and then dried under reduced pressure to give the desired title compound (215.8 mg, yield 69%).
¹H-NMR (CDCl₃) δ: 2.26 (3H, s), 7.32 (1H, dd, J = 8.5, 2.1 Hz), 7.44-7.49 (3H, m), 7.75 (2H, d, J = 8.7 Hz), 7.79 (1H, d, J = 8.7 Hz), 8.07 (1H, s), 8.12 (1H, br s).

### (4d) N-[4-(6-Chloro-1H-benzimidazol-1-yl)phenyl]-N-[2-(tetrahydro-2H-pyran-2-yloxy)ethyl]acetamide

Sodium hydride (content 55%) (90.6 mg, 2.27 mmol) was added to N, N-dimethylformamide (5 mL) and the resulting mixture was ice cooled. A solution of N-[4-(6-chloro-1H-benzimidazol-1-yl)phenyl]acetamide (216 mg, 755 µmol) produced in Example 4(4c) in N,N-dimethylformamide (2 mL) was added dropwise, followed by stirring at 0°C for 30 minutes under a nitrogen atmosphere. To this, 2-(2-bromoethoxy)tetrahydro-2H-pyran (343 µL, 2.27 mmol) was added, followed by stirring at room temperature for one hour. To the resulting reaction liquid, water was added, followed by extraction with ethyl acetate. The resulting organic layer was washed with saturated brine and then dried over anhydrous sodium sulfate. The solvent was then distilled off under reduced pressure and the residue thus obtained was purified by basic silica gel chromatography (hexane : ethyl acetate, 95 : 5 - 20 : 80, V/V) to give the desired title compound (219.6 mg, yield 70%).
¹H-NMR (CDCl₃) δ: 1.48-1.57 (2H, m), 1.65-1.82 (2H, m), 1.97 (3H, s), 3.47-4.09 (6H, m), 4.58-4.61 (1H, m), 7.34 (1H, dd, J = 8.7, 1.8 Hz), 7.49-7.57 (5H, m), 7.80 (1H, d, J = 8.7 Hz), 8.11 (1H, s).

### (4e) 2-{[4-(6-Chloro-1H-benzimidazol-1-yl)phenyl]amino}ethanol hydrochloride

Into methanol (10 mL), N-[4-(6-chloro-1H-benzimidazol-1-yl)phenyl]-N-[2-(tetrahydro-2H-pyran-2-yloxy)ethyl]acetamide (219.6 mg, 530.6 µmol) produced in Example 4 (4d) was dissolved, to which 6N hydrochloric acid (3.5 mL) was added, followed by stirring at room temperature for two hours. The solvent was distilled off under reduced pressure, and further, toluene was added and azeotropic distillation was performed. A solid precipitated, which was sequentially washed with ethyl acetate and methanol and dried under reduced pressure to give the desired title compound (168.4 mg, yield 98%). ¹H-NMR (DMSO-D₆) δ: 3.21 (2H, t, J = 5.7 Hz), 3.62 (2H, t, J = 5.7 Hz), 6.93 (2H, d, J = 8.7 Hz), 7.49 (2H, d, J = 8.7 Hz), 7.56-7.59 (1H, m), 7.68 (1H, s), 7.91 (1H, d, J = 8.8 Hz), 9.42 (1H, s).

### <Example 5>

### 2-{ [4-{5-Methoxy-1H-benzimidazol-1-yl)phenyl]amino}ethanol hydrochloride

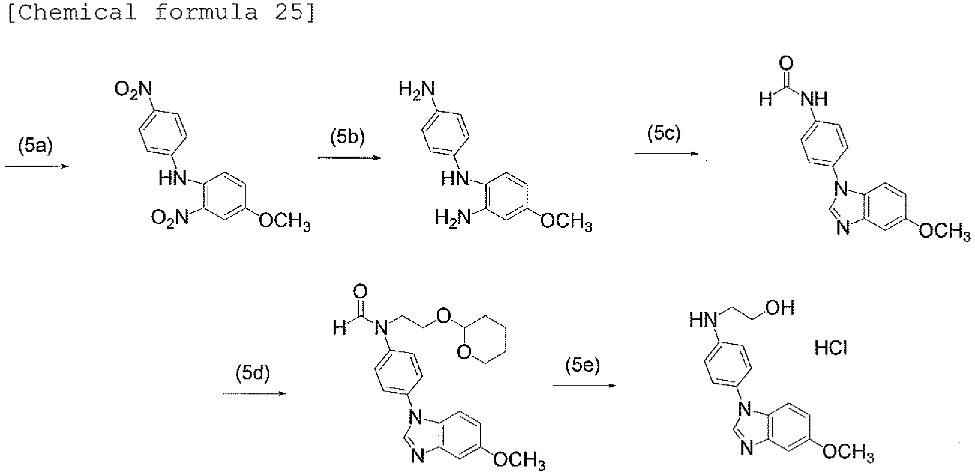

### (5a) 4-Methoxy-2-nitro-N-(4-nitrophenyl)aniline

Toluene (15 mL) was added to 4-methoxy-2-nitroaniline (1.50 g, 8.92 mmol), p-bromonitrobenzene (3.6 g, 17 mmol), 2-dicyclohexylphosphino-2'-(N,N-dimethylamino)biphenyl (516 mg, 892 µmol), sodium tert-butoxide (1.71 g, 17.8 mmol), and tris(dibenzylideneacetone)dipalladium(0) (408 mg, 446 µmol), followed by stirring at 80°C for one hour under a nitrogen atmosphere. The resulting reaction liquid was left to cool, to which dichloromethane (80 mL) was added, followed by filtration through Celite. The solvent was then distilled off under reduced pressure and the residue thus obtained was purified by basic silica gel chromatography (hexane : ethyl acetate, 95 : 5 - 50 : 50, V/V) to give the desired title compound (1.77 g, yield 69%).
¹H-NMR (CDCl₃) 5: 3.88 (3H, s), 7.18-7.24 (3H, m), 7.52 (1H, d, J = 9.3 Hz), 7.67 (1H, d, J = 2.9 Hz), 8.19-8.23 (2H, m).

### (5b) N¹-(4-Aminophenyl)-4-methoxybenzene-1,2-diamine

Into ethyl acetate (20 mL), 4-methoxy-2-nitro-N-(4-nitrophenyl)aniline (500 mg, 1.73 mmol) produced in Example 5 (5a) was dissolved, to which 10% palladium-carbon (100 mg) was added under a nitrogen atmosphere. After hydrogen substitution, the resulting mixture was stirred at room temperature for two hours under a hydrogen atmosphere. Insoluble matters were filtered off while washing with ethyl acetate. The solvent was then distilled off under reduced pressure and the residue thus obtained was purified by basic silica gel chromatography (hexane : ethyl acetate, 95 : 5 - 25 : 75, V/V) to give the desired title compound (355 mg, yield 90%).
¹H-NMR (CDCl₃) δ: 3.37 (2H, br s), 3.77 (3H, s), 3.85 (2H, br s), 4.71 (1H, br s), 6.29 (1H, dd, J = 8.5, 2.7 Hz), 6.35 (1H, d, J = 2.7 Hz), 6.53-6.56 (2H, m), 6.58-6.61 (2H, m), 6.94 (1H, d, J = 8.5 Hz).

### (5c) N-[4-(5-Methoxy-1H-benzimidazol-1-yl)phenyl]formamide

Into formic acid (8 mL), N¹-(4-aminophenyl)-4-methoxybenzene-1,2-diamine (355.9 mg, 1.55 mmol) produced in Example 5 (5b) was dissolved, followed by stirring at 100°C for three hours. The resulting mixture was left to cool and then neutralized with a saturated aqueous solution of sodium bicarbonate, followed by extraction with ethyl acetate. The organic layer thus separated was washed with saturated brine and then dried over anhydrous sodium sulfate. After filtration, the solvent was distilled off under reduced pressure. A solid precipitated, which was washed with a mixed solution of ethyl acetate / hexane (1 : 20) and then dried under reduced pressure to give the desired title compound (200 mg, yield 48%).
¹H-NMR (CDCl₃) δ: 3.90 (3H, s), 6.97-7.00 (1H, m), 7.34-7.39 (3H, m), 7.48-7.53 (2H, m), 7.77 (1H, d, J = 9.2 Hz), 8.04 (1H, s), 8.47 (1H, s).

### (5d) N-[4-(5-Methoxy-1H-benzimidazol-1-yl)phenyl]-N-[2-(tetrahydro-2H-pyran-2-yloxy)ethyl]formamide

Sodium hydride (content 55%) (113 mg, 2.84 mmol) was added to N,N-dimethylformamide (5 mL), and the resulting mixture was cooled to 0°C under a nitrogen atmosphere. The solution thus obtained was added dropwise to N-[4-(5-methoxy-1H-benzimidazol-1-yl)phenyl]formamide (252 mg, 945 µmol) produced in Example 5 (5c) in N,N-dimethylformamide (2 mL), followed by stirring at 0°C for 30 minutes. To this, 2-(2-bromoethoxy)tetrahydro-2H-pyran (429 µL, 2.84 mmol) was added, followed by stirring at room temperature for one hour. To the reaction liquid, water was added, followed by extraction with ethyl acetate. The resulting organic layer was washed with saturated brine and dried over anhydrous sodium sulfate. The solvent was then distilled off under reduced pressure and the residue thus obtained was purified by basic silica gel chromatography (hexane : ethyl acetate, 95 : 5 - 25 : 75, V/V) to give the desired title compound (208 mg, yield 56%).
¹H-NMR (CDCl₃) 5: 1.48-1.64 (4H, m), 1.67-1.73 (2H, m), 3.47-3.54 (1H, m), 3.68-3.81 (2H, m), 3.90 (3H, s), 3.94-4.05 (2H, m), 4.09-4.15 (1H, m), 4.59-4.61 (1H, m), 6.98-7.01 (1H, m), 7.34-7.36 (1H, m), 7.39-7.42 (1H, m), 7.50-7.57 (4H, m), 8.05 (1H, s), 8.53 (1H, s).

### (5e) 2-{[4-(5-Methoxy-1H-benzimidazol-1-yl)phenyl]amino}ethanol hydrochloride

Into methanol (5 mL), N-[4-(5-methoxy-1H-benzimidazol-1-yl)phenyl]-N-[2-(tetrahydro-2H-pyran-2-yloxy)ethyl]formamide (208 mg, 527 µmol) produced in Example 5 (5d) was dissolved, to which 6N hydrochloric acid (3.5 mL) was added, followed by stirring at room temperature for three hours. The solvent was then distilled off under reduced pressure, to which water (10 mL) and ethyl acetate (10 mL) were added for extraction. The aqueous layer thus separated was neutralized with saturated sodium bicarbonate and then extracted with a mixed solvent of chloroform / isopropanol (4 / 1). The combined organic layer was dried over anhydrous magnesium sulfate and the solvent was distilled off under reduced pressure to give the desired title compound (142.1 mg, yield 95%).
¹H-NMR (DMSO-D₆) δ: 3.17-3.20 (2H, m), 3.58-3.61 (2H, m), 3.90 (3H, s), 6.83-6.87 (2H, m), 7.21-7.24 (1H, m), 7.36 (1H, s), 7.46-7.49 (2H, m), 7.59-7.62 (1H, m), 9.73 (1H, s).

### <Example 6>

### 1-[4-(2-Methoxyethoxy)phenyl]-1H-benzoimidazole

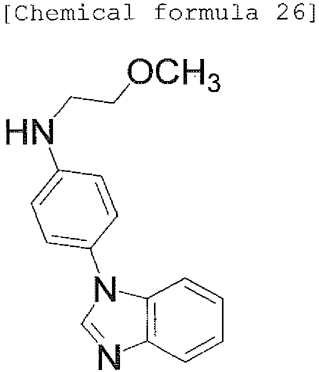

Into dimethyl sulfoxide (20 mL), 1-(4-iodophenyl)-1H-benzoimidazole (1.0 g, 3.1 mmol) produced by the same method as in Example 1 (1a) was dissolved, to which 2-methoxyethanolamine (0.326 mL, 3.75 mmol), copper(I) iodide (59.5 mg, 0.312 mmol), N,N-dimethylglycine (64.4 mg, 0.625 mmol), and potassium carbonate (863 mg, 6.25 mmol) were added, followed by stirring at 90°C for 25 hours under a nitrogen atmosphere. The resulting reaction liquid was left to cool and then added to a saturated aqueous solution of sodium bicarbonate, followed by extraction with ethyl acetate. The resulting organic layer was washed with saturated brine and then dried over anhydrous sodium sulfate. After filtration through Celite, the solvent was distilled off under reduced pressure and the residue thus obtained was purified by basic silica gel column chromatography (hexane : ethyl acetate = 90 : 10 to 0 : 100, V/V) to give the desired title compound (205 mg, 0.769 mmol).
¹H-NMR (DMSO-D₆)δ: 3.21-3.37 (5H, m), 3.52 (2H, t, J = 5.6 Hz), 6.00 (1H, t, J = 5.0 Hz), 6.77 (2H, d, J = 8.7 Hz), 7.21-7.34 (4H, m), 7.41-7.48 (1H, m), 7.75-7.69 (1H, m), 8.34 (1H, s).

### <Example 7>

### 2-[4-(6-Fluoroimidazo[1,2-a]pyridin-3-yl)phenoxy]ethanol

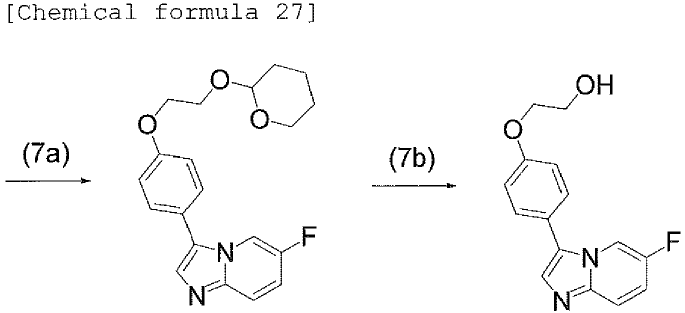

### (7a) 6-Fluoro-3-{4-[2-(tetrahydro-2H-pyran-2-yloxy)ethoxy]phenyl}imidazo[1,2-a]pyridine

Into N,N-dimethylformamide (7 mL), 4-(6-fluoroimidazo[1,2-a]pyridin-3-yl)phenol (133 mg, 0.583 mmol) produced by the same method as in Example 2 (2a), Example 2 (2b), and Example 2 (2c) was dissolved, to which 2-(2-bromoethoxy)tetrahydro-2H-pyran (0.176 mL, 1.17 mmol) and potassium carbonate (322 mg, 2.33 mmol) were added, followed by stirring at 75°C for 24 hours under a nitrogen atmosphere. The resulting reaction liquid was left to cool, and water and ethyl acetate were added for extraction. The resulting organic layer was washed with saturated brine and then dried over anhydrous sodium sulfate. After filtration through Celite, the solvent was distilled off under reduced pressure and the residue thus obtained was purified by basic silica gel column chromatography (hexane : ethyl acetate = 90 : 10 to 0 : 100, V/V) to give the desired title compound (207 mg, yield 100%).
¹H-NMR (DMSO-D₆) δ: 1.37-1.78 (6H, m), 3.42-3.48 (1H, m), 3.72-3.82 (2H, m), 3.91-3.98 (1H, m), 4.18-4.22 (2H, m), 4.65-4.68 (1H, m), 7.10-7.14 (2H, m), 7.32-7.38 (1H, m), 7.56-7.60 (2H, m), 7.68-7.72 (1H, m), 7.74 (1H, s), 8.52-8.55 (1H, m).

### (7b) 2-[4-(6-Fluoroimidazo[1,2-a]pyridin-3-yl)phenoxy] ethanol

Using 6-fluoro-3-{4-[2-(tetrahydro-2H-pyran-2-yloxy)ethoxy]phenyl}imidazo[1,2-a]pyridine produced in Example 7 (7a), the desired title compound was obtained by the same method as in Example 4 (4e).
¹H-NMR (DMSO-D₆) δ: 3.74 (2H, dt, J = 5.5, 4.8 Hz), 4.05 (2H, t, J = 4.8 Hz), 4.91 (1H, t, J = 5.5 Hz), 7.08-7.13 (2H, m), 7.32-7.38 (1H, m), 7.56-7.60 (2H, m), 7.68-7.73 (1H, m), 7.74 (1H, s), 8.55-8.52 (1H, m).

### <Example 8>

### 2-{4-[6-(1H-Pyrrol-3-yl)imidazo[1,2-a]pyridin-3-yl]phenoxy)ethanol

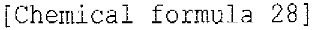

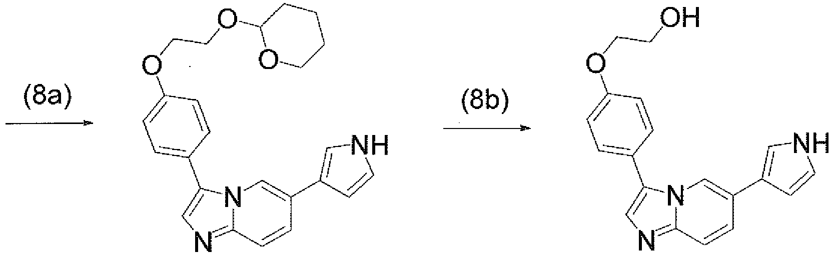

### (8a) 6-(1H-Pyrrol-3-yl)-3-{4-[2-(tetrahydro-2H-pyran-2-yloxy)ethoxy]phenyl}imidazo[1,2-a]pyridine

Into N,N-dimethylformamide (8 mL), 6-chloro-3-{4-[2-(tetrahydro-2H-pyran-2-yloxy)ethoxy]phenyl}imidazo[1,2-a]pyridine (150 mg, 0.412 mmol) produced by the same method as in Example 2 (2a), Example 2 (2b), Example 2 (2c), and Example 7 (7a) was dissolved, to which [1,1'-bis(di-tert-butylphosphino)ferrocene]palladium(II) dichloride (5.24 mg, 8.05 µmol), [1-(triisopropylsilyl)-1H-pyrrol-3-yl]boronic acid (129 mg, 0.483 mmol), and potassium carbonate (111 mg, 0.805 mmol) were added, followed by stirring at 120°C for 24 hours under a nitrogen atmosphere. To the resulting reaction liquid, water was added, followed by extraction with ethyl acetate. The resulting organic layer was washed with saturated brine and dried over anhydrous sodium sulfate. After filtration through Celite, the solvent was distilled off under reduced pressure, and the residue thus obtained was purified by basic silica gel column chromatography (hexane : ethyl acetate = 90 : 10 to 0 : 100, V/V) to give the desired title compound (34.1 mg, yield 21%).
¹H-NMR (CD₃OD) δ: 1.46-1.65 (4H, m), 1.67-1.77 (1H, m), 1.78-1.90 (1H, m), 3.48-3.57 (1H, m), 3.77-3.85 (1H, m), 3.87-3.96 (1H, m), 4.01-4.11 (1H, m), 4.21 (2H, t, J = 4.1 Hz), 4.71 (1H, t, J = 2.8 Hz), 6.32-6.36 (1H, m), 6.76-6.80 (1H, m), 7.08-7.14 (3H, m), 7.46-7.58 (5H, m), 8.33 (1H, br s).

### (8b) 2-{4-[6-(1H-Pyrrol-3-yl)imidazo[1,2-a]pyridin-3-y1]phenoxy}ethanol

Using 6-(1H-pyrrol-3-yl)-3-{4-[2-(tetrahydro-2H-pyran-2-yloxy)ethoxy]phenyl}imidazo[1,2-a]pyridine produced in Example 8 (8a), the desired title compound was obtained by the same method as in Example 4 (4e). ¹H-NMR (DMSO-D₆) δ: 3.75 (2H, dt, J = 5.5, 5.0 Hz), 4.06 (2H, t, J = 5.0 Hz), 4.91 (1H, t, J = 5.5 Hz), 6.27-6.31 (1H, m), 6.80-6.85 (1H, m), 7.10-7.15 (2H, m), 7.25-7.27 (1H, m), 7.51-7.54 (1H, m), 7.56-7.62 (4H, m), 8.38 (1H, s), 11.01 (1H, br s).

### <Example 9>

### 2-[4-(5-Pyridin-4-yl-1H-benzimidazol-1-yl)phenoxy]ethanol

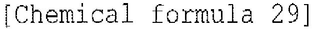

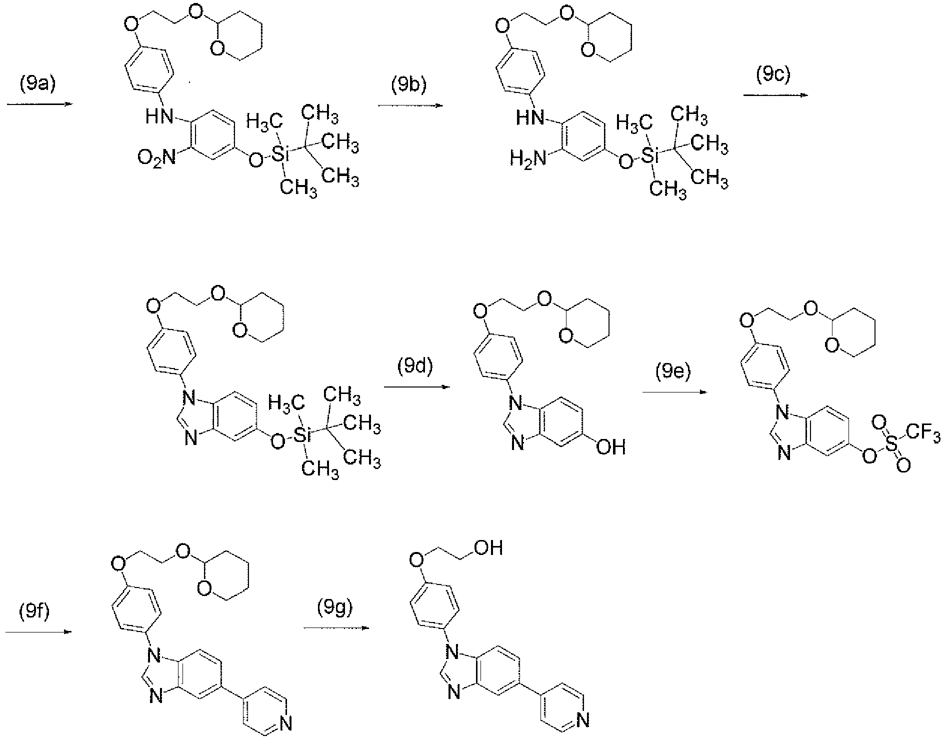

### (9a) 4-{[Tert-butyl(dimethyl)silyl]oxy}-2-nitro-N-{4-[2-(tetrahydro-2H-pyran-2-yloxy)ethoxy]phenyl}aniline

Toluene (80 mL) was added to 4-{[tert-butyl(dimethyl)silyl]oxy}-2-nitroaniline (9.46 g, 35.25 mmol) produced in Reference Example (1), 2-[2-(4-bromophenoxy)ethoxy]tetrahydro-2H-pyran (10.41 g, 34.56 mmol) produced in Reference Example (2), tris(dibenzylideneacetone)dipalladium (1.58 g, 1.72 mmol), 2-di-tert-butylphosphino-2'-(N,N-dimethylamino)biphenyl (1.18 g, 3.56 mmol), and sodium tert-butoxide (4.98 g, 51.8 mmol). The resulting mixture was stirred at 110°C for 10 minutes under a nitrogen atmosphere and then ice cooled. To this, ice-cooled ethyl acetate (200 mL) was poured and insoluble matters were filtered off through Celite. The filtrate was subjected to extraction, and the resulting organic layer was washed with saturated brine and then dried over anhydrous sodium sulfate. After filtration, the solvent was distilled off under reduced pressure, and the residue thus obtained was purified by basic silica gel chromatography (hexane : ethyl acetate, 30 : 70 - 0 : 100, V/V) to give the desired title compound (14.42 g, yield 85%).
¹H-NMR (CDCl₃) δ: 0.21 (6H, s), 0.99 (9H, s), 1.50-1.70 (4H, m), 1.71-1.91 (2H, m), 3.51-3.59 (1H, m), 3.81-3.96 (2H, m), 4.04-4.21 (4H, m), 4.71-4.75 (1H, m), 6.92-6.99 (3H, m), 7.14-7.19 (2H, m), 7.61-7.65 (1H, m), 9.19 (1H, s).

### (9b) 4-{ [Tert-butyl(dimethyl)silyl]oxy}-N¹-{4-[2-(tetrahydro-2H-pyran-2-yloxy)ethoxy]phenyl}benzene-1,2-diamine

To 4-{[tert-butyl(dimethyl)silyl]oxy}-2-nitro-N-(4-[2-(tetrahydro-2H-pyran-2-yloxy)ethoxy]phenyl}aniline (298 mg, 0.611 mmol) produced in Example 9 (9a), 10% palladium hydroxide (50%wet) (60 mg) and ethanol (10 mL) were added under a nitrogen atmosphere, and hydrogen substitution was performed three times. After stirring at room temperature for two hours under a hydrogen atmosphere, insoluble matters were filtered off through Celite while washing with ethanol. The solvent was then distilled off under reduced pressure and the residue thus obtained was purified by basic silica gel chromatography (hexane: ethyl acetate, 30 : 70 - 0 : 100, V/V) to give the desired title compound (207 mg, yield 74%).
¹H-NMR (CDCl₃) δ: 0.20 (6H, s), 0.98 (9H, s), 1.49-1.68 (4H, m), 1.69-1.87 (2H, m), 3.48-3.56 (1H, m), 3.74-3.83 (3H, m), 3.86-3.93 (1H, m), 3.97-4.04 (1H, m), 4.06-4.11 (2H, m), 4.68-4.72 (1H, m), 4.79 (1H, br s), 6.22 (1H, dd, J = 8.3, 2.6 Hz), 6.30 (1H, d, J = 2.6 Hz), 6.57-6.61 (2H, m), 6.78-6.82 (2H, m), 6.88 (1H, d, J = 8.3 Hz).

### (9c) 5-{ [Tert-butyl(dimethyl)silyl]oxy}-1-{4-[2-(tetrahydro-2H-pyran-2-yloxy)ethoxy]phenyl}-1H-benzimidazole

To 4-{[tert-butyl(dimethyl)silyl]oxy}-N¹-{4-[2-(tetrahydro-2H-pyran-2-yloxy)ethoxy]phenyl}benzene-1,2-diamine (207 mg, 0.453 mmol) produced in Example 9 (9b) and orthoformic acid triethyl ester (400 µL), ytterbium trifluoromethanesulfonate (6.28 mg, 0.01mmol) was added, followed by stirring at 90°C for 10 minutes. To this, ethyl acetate and water were added for extraction, and the resulting organic layer was washed with saturated brine and dried over anhydrous sodium sulfate. After filtration, the solvent was distilled off under reduced pressure, and the residue thus obtained was purified by basic silica gel chromatography (hexane : ethyl acetate, 30 : 70 - 0 : 100, V/V) to give the desired title compound (197 mg, yield 93%).
¹H-NMR (CDCl₃) δ: 0.22 (6H, s), 1.01 (9H, s), 1.50-1.70 (4H, m), 1.71-1.89 (2H, m), 3.51-3.59 (1H, m), 3.82-3.96 (2H, m), 4.07-4.14 (1H, m), 4.20-4.26 (2H, m), 4.71-4.75 (1H, m), 6.84-6.88 (1H, m), 7.07-7.12 (2H, m), 7.27-7.31 (2H, m), 7.37-7.41 (2H, m), 7.98 (1H, s).

### (9d) 1-{4-[2-(Tetrahydro-2H-pyran-2-yloxy)ethoxy]phenyl}-1H-benzimidazol-5-ol

Into tetrahydrofuran (40 mL), 5-{[tert-butyl(dimethyl)silyl]oxy}-1-{4-[2-(tetrahydro-2H-pyran-2-yloxy)ethoxy]phenyl}-1H-benzimidazole (4.53 g, 4.55 mmol) produced in Example 9 (9c) was dissolved, and the resulting mixture was ice cooled. To this, a 1M tetrabutylammonium fluoride / tetrahydrofuran solution (6.83 mL, 6.83 mmol) was added dropwise, followed by stirring at room temperature for 30 minutes. The solvent was then distilled off under reduced pressure, and the residue thus obtained was purified by basic silica gel chromatography (hexane : ethyl acetate, 90 : 10 - 0 : 100, V/V, ethyl acetate : methanol, 100 : 0 - 95 : 5, V/V) to give the desired title compound (1.16 g, yield 72%).
¹H-NMR (CDCl₃) δ: 1.57-1.68 (4H, m), 1.73-1.90 (2H, m), 3.53-3.59 (1H, m), 3.84-3.96 (2H, m), 4.08-4.14 (1H, m), 4.21-4.26 (2H, m), 4.72-4.75 (1H, m), 5.40 (1H, s), 6.88-6.92 (1H, m), 7.08-7.12 (2H, m), 7.29-7.32 (2H, m), 7.36-7.41 (2H, m), 8.00 (1H, s).

### (9e) 1-{4-[2-(Tetrahydro-2H-pyran-2-yloxy)ethoxy]phenyl}-1H-benzimidazol-5-yl trifluoromethanesulfonate

Into dichloromethane (60 mL), 1-{4-[2-(tetrahydro-2H-pyran-2-yloxy)ethoxy]phenyl}-1H-benzimidazol-5-ol (1.00 g, 2.82 mmol) produced in Example 9 (9d) was dissolved, to which pyridine (1.14 mL, 14.1 mmol) was added at room temperature, followed by stirring at -20°C. To this, trifluoroacetic anhydride (569 µL, 3.39 mmol) was added, followed by stirring at -10°C for one hour. The resulting reaction liquid was added dropwise to a saturated aqueous solution of sodium bicarbonate for neutralization, followed by extraction. The resulting organic layer was sequentially washed with water and saturated brine, and dried over anhydrous sodium sulfate. The solvent was then distilled off under reduced pressure and the residue thus obtained was purified by silica gel chromatography (hexane : ethyl acetate, 100 : 0 - 50 : 50, V/V) to give the desired title compound (988 mg, yield 72%).
¹H-NMR (CDCl₃) δ: 1.54-1.58 (2H, m), 1.61-1.68 (2H, m), 1.74-1.80 (1H, m), 1.82-1.90 (1H, m), 3.54-3.58 (1H, m), 3.85-3.95 (2H, m), 4.10-4.12 (1H, m), 4.21-4.28 (2H, m), 4.73 (1H, t, J = 3.7 Hz), 7.10-7.14 (2H, m), 7.24 (1H, dd, J = 9.2, 2.3 Hz), 7.37-7.40 (2H, m), 7.46 (1H, d, J = 9.2 Hz), 7.78 (1H, d, J = 2.3 Hz), 8.14 (1H, s).

### (9f) 5-Pyridin-4-yl-1-{4-[2-(tetrahydro-2H-pyran-2-yloxy)ethoxy]phenyl}-1H-benzimidazole

Into ethylene glycol dimethyl ether / water (4 / 1, 10 mL), 1-{4-[2-(tetrahydro-2H-pyran-2-yloxy)ethoxy]phenyl}-1H-benzimidazol-5-yl trifluoromethanesulfonate (369.6 mg, 758. µmol) produced in Example 9 (9e) was dissolved, to which potassium carbonate (157 mg, 1.14 mmol), 4-pyridineboronic acid (111.9 mg, 910.2 µmol), and tetrakis(triphenylphosphine)palladium (87.6 mg, 75.8 µmol) were added, followed by stirring at 80°C for one hour under a nitrogen atmosphere. The resulting mixture was left to cool, and the solvent was distilled off under reduced pressure. Water and ethyl acetate were added for extraction, and the resulting organic layer was washed with saturated brine. The organic layer was dried over anhydrous sodium sulfate and then filtered. The solvent was distilled off under reduced pressure and the residue thus obtained was purified by basic silica gel chromatography (hexane : ethyl acetate, 90 : 10 - 50 : 50, V/V) to give the desired title compound (318.7 mg, yield 100%).
¹H-NMR (CDCl₃) δ: 1.51-1.70 (4H, m), 1.73-1.91 (2H, m), 3.53-3.60 (1H, m), 3.85-3.97 (2H, m), 4.09-4.16 (1H, m), 4.24-4.28 (2H, m), 4.74 (1H, t, J = 3.7 Hz), 7.12-7.16 (2H, m), 7.40-7.50 (4H, m), 7.52-7.71 (2H, m), 8.12 (1H, s), 8.16 (1H, d, J = 1.8 Hz), 8.66-8.69 (2H, m).

### (9g) 2-[4-(5-Pyridin-4-yl-1H-benzimidazol-1-yl)phenoxy]ethanol

Using 5-pyridin-4-yl-1-{4-[2-(tetrahydro-2H-pyran-2-yloxy)ethoxy]phenyl}-1H-benzimidazole produced in Example 9 (9f), the desired title compound was obtained by the same method as in Example 4 (4e).
¹H-NMR (DMSO-D₆) δ: 3.77 (2H, t, J = 4.9 Hz), 4.11 (2H, t, J = 4.9 Hz), 7.20-7.25 (2H, m), 7.64-7.69 (2H, m), 7.77 (1H, d, J = 8.7 Hz), 8.06 (1H, dd, J = 8.7, 1.7 Hz), 8.53 (2H, d, J = 6.8 Hz), 8.59 (1H, d, J = 1.7 Hz), 8.93-8.98 (3H, m).

### <Example 10>

### 2-({1-[7-(1-Acetyl-1,2,3,6-tetrahydropyridin-4-yl)imidazo[1,2-a]pyridin-3-yl]piperidin-4-yl}oxy)ethanol hydrochloride

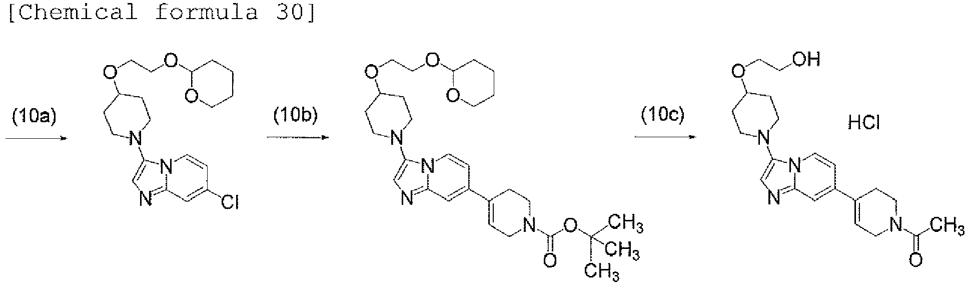

### (10a) 7-Chloro-3-{4-[2-(tetrahydro-2H-pyran-2-yloxy)ethoxy]piperidin-1-yl}imidazo[1,2-a]pyridine

Into ethanol (160 mL), 4-[2-(tetrahydro-2H-pyran-2-yloxy)ethoxy]piperidine (12.6 g, 55.0 mmol) produced by the same method as in Reference Example 5 was dissolved, to which a 8.8 M aqueous solution of glyoxal (3.13 mL, 27.5 mmol) and benzotriazole (6.55 g, 55.0 mmol) were added, followed by stirring at room temperature overnight under a nitrogen atmosphere. The reaction liquid was added to water and then extracted with ethyl acetate. The resulting organic layer was sequentially washed with water and saturated brine, to which anhydrous sodium sulfate was added, and the resulting mixture was stirred for a while. After filtration through Celite, the solvent was distilled off under reduced pressure to give a crude product of 1,1'-(1,2-bis{4-[2-(tetrahydro-2H-pyran-2-yloxy)ethoxy]piperidin-1-yl}ethane-1,2-diyl)bis(1H-benzotriazole) (17.3 g). This crude product (12.6g) was dissolved in 1,2-dichloroethane (80 mL), to which 2-amino-4-chloropyridine (2.25 g, 17.5 mmol) was added, followed by heating under reflux for 70 minutes under a nitrogen atmosphere. The resulting mixture was left to cool, and the solvent was distilled off under reduced pressure. The residue thus obtained was purified by basic silica gel column chromatography (hexane : ethyl acetate = 80 : 20 - 0 : 100, V/V) and by silica gel column chromatography (hexane : ethyl acetate = 75 : 25 - 0 : 100, V/V) to give the desired title compound (5.06 g, yield 24%).
¹H-NMR (CDCl₃) δ: 1.51-1.66 (4H, m), 1.71-1.90 (4H, m), 2.02-2.10 (2H, m), 2.84-2.90 (2H, m), 3.19-3.25 (2H, m), 3.51-3.56 (1H, m), 3.57-3.68 (2H, m), 3.71 (2H, t, J = 5.2 Hz), 3.88-3.95 (2H, m), 4.70-4.67 (1H, m), 6.78 (1H, dd, J = 7.6, 1.6 Hz), 7.24 (1H, s), 7.53 (1H, d, J = 1.6 Hz), 7.87 (1H, d, J = 7.6 Hz).

### (10b) Tert-butyl 4-(3-{4-[2-(Tetrahydro-2H-pyran-2-yloxy)ethoxy]piperidin-1-yl}imidazo[1,2-a]pyridin-7-yl]-3,6-dihydropyridine-1(2H)-carboxylate

Into dimethoxyethane / water (3 / 1, 80 mL), 7-chloro-3-{4-[2-(tetrahydro-2H-pyran-2-yloxy)ethoxy]piperidin-1-yl)imidazo[1,2-a]pyridine (1.00 g, 2.63 mmol) produced in Example 10 (10a) was dissolved, to which 3,6-dihydro-2H-pyridin-1-N-Boc-4-boronic acid pinacol ester (1.46 g, 4.74 mmol), tris(dibenzylideneacetone)dipalladium (362 mg, 0.395 mmol), 2-(dicyclohexylphosphino)biphenyl (277 mg, 0.790 mmol), and potassium carbonate (728 mg, 5.26 mmol) were added, followed by stirring at 100°C overnight under a nitrogen atmosphere. The solvent was then distilled off under reduced pressure and the residue thus obtained was purified by basic silica gel column chromatography (hexane : ethyl acetate = 85 : 15 - 0 : 100, V/V) to give the desired title compound (1.38 g, yield 100%).
¹H-NMR (DMSO-D₆) δ: 1.38-1.50 (15H, m), 1.57-1.75 (4H, m), 1.93-2.01 (2H, m), 2.76-2.83 (2H, m), 3.09-3.16 (2H, m), 3.39-3.45 (1H, m), 3.48-3.63 (6H, m), 3.69-3.80 (2H, m), 3.99-4.06 (2H, m), 4.59-4.61 (1H, m), 6.34 (1H, br s), 7.08 (1H, dd, J = 7.4, 1.7 Hz), 7.18 (1H, s), 7.38 (1H, s), 8.00 (1H, d, J = 7.4 Hz).

### (10c) 2-({1-[7-(1-Acetyl-1,2,3,6-tetrahydropyridin-4-yl)imidazo[1,2-a]pyridin-3-yl]piperidin-4-yl}oxy)ethanol hydrochloride

Using the same method as in Example 4 (4e), tert-butyl 4-(3-{4-[2-(tetrahydro-2H-pyran-2-yloxy)ethoxy]piperidin-1-yl}imidazo[1,2-a]pyridin-7-yl]-3,6-dihydropyridine-1(2H)-carboxylate (480 mg, 1.40 mmol) produced in Example 10 (10b) was converted into a crude product of 2-({1-[7-(1,2,3,6-tetrahydropyridin-4-yl)imidazo[1,2-a]pyridin-3-yl]piperidin-4-yl}oxy)ethanol. This crude product was dissolved in pyridine (20 mL), to which acetic anhydride (663 mL, 7.01 mmol) was added, followed by stirring at room temperature for two hours under a nitrogen atmosphere. The solvent was then distilled off under reduced pressure and the residue thus obtained was coarsely purified by basic silica gel column chromatography (ethyl acetate : methanol = 100 : 0 - 90 : 10, V/V). The residue obtained by concentration was dissolved in methanol (5 mL), to which sodium methoxide (7.60 mg, 141 µmol) was added, followed by stirring at room temperature for two hours under a nitrogen atmosphere. To the resulting reaction liquid, ammonium chloride was added for neutralization, and insoluble matters were filtered off. The solvent was then distilled off under reduced pressure. The residue thus obtained was purified by basic silica gel column chromatography (ethyl acetate : methanol = 100 : 0 - 93 : 7, V/V). To the product thus purified, a 1N hydrochloric acid / ethanol solution was added, followed by stirring at room temperature for one hour. The solvent was then distilled off under reduced pressure to give the desired title compound (213 mg, yield 34%).
¹H-NMR (DMSO-D₆) δ: 1.68-1.76 (2H, m), 1.97-2.03 (2H, m), 2.04-2.10 (3H, m), 2.50-2.54 (1H, m), 2.59-2.64 (1H, m), 2.92-2.86 (2H, m), 3.17-3.23 (2H, m), 3.46-3.53 (4H, m), 3.53-3.58 (1H, m), 3.65-3.71 (2H, m), 4.17-4.27 (2H, m), 6.69-6.74 (1H, m), 7.61-7.70 (2H, m), 7.86 (1H, s), 8.51 (1H, d, J = 7.4 Hz).

### <Example 11>

### 2-[4-(6-Pyridin-4-ylpyrazolo[1,5-a]pyridin-3-yl)phenoxy]ethanol

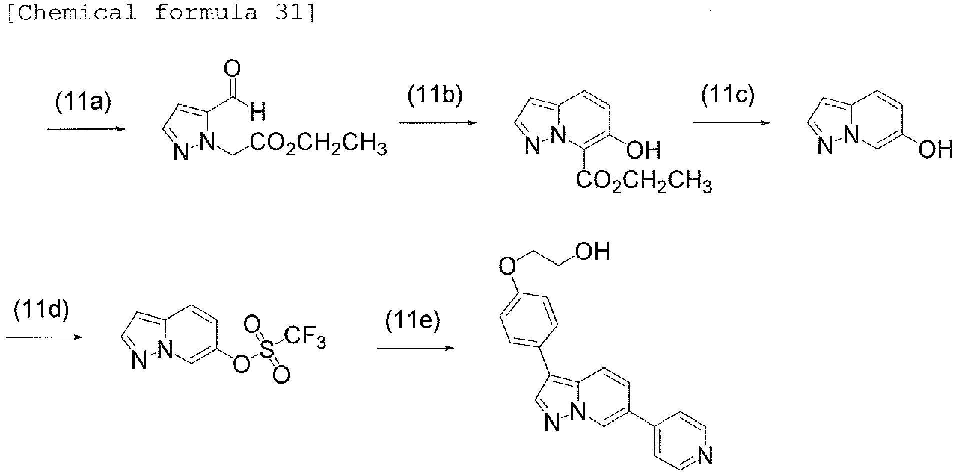

### (11a) Ethyl (5-formyl-1H-pyrazol-1-yl)acetate

Into ethanol (100 mL), 4-(dimethylamino)-1,1-dimethoxybut-3-en-2-one (4.33 g, 25.0 mmol) was dissolved, to which triethylamine (4.36 mL, 31.3 mmol) and ethyl hydrazinoacetate (4.25 g, 27.5 mmol) were added, followed by heating under reflux for two hours. The resulting mixture was left to cool, to which 0.5 N hydrochloric acid (100 mL) was added, followed by stirring at room temperature for one hour. To the resulting reaction liquid, a saturated aqueous solution of sodium bicarbonate was added, followed by extraction with ethyl acetate. The resulting organic layer was dried over anhydrous sodium sulfate. After filtration, the solvent was distilled off under reduced pressure, and the residue thus obtained was purified by silica gel column chromatography (hexane : ethyl acetate = 4 : 1 - 3 : 2, V/V) to give the desired title compound (2.53 g, yield 56%).
¹H-NMR (CDCl₃) δ: 1.28 (3H, t, J = 7.3 Hz), 4.23 (2H, q, J = 7.3 Hz), 5.30 (2H, s), 6.98 (1H, d, J = 1.8 Hz), 7.64 (1H, d, J = 1.8 Hz), 9.85 (1H, s).

### (11b) Ethyl 6-hydroxypyrazolo[1,5-a]pyridine-7-carboxylate

Into tetrahydrofuran (300 mL), ethyl [bis(2,2,2-trifluoroethoxy)phosphinyl]acetate (22.9 g, 68.9 mmol) was dissolved, and the resulting mixture was ice cooled. To this, sodium hydride (content 55%) (3.01 g, 68.9 mmol) was added, to which a solution of ethyl (5-formyl-1H-pyrazol-1-yl)acetate (57.4 mmol) produced in Example 11 (11a) in tetrahydrofuran (85 mL) was further added, followed by stirring at 0°C for 20 minutes. To this, sodium hydride (content 55%) (3.01 g, 68.9 mmol) was further added, followed by stirring at 0°C for 20 minutes. To the resulting reaction liquid, a saturated aqueous solution of ammonium chloride was added, followed by extraction with ethyl acetate. The combined organic layer was then dried over anhydrous sodium sulfate. After filtration, the solvent was distilled off under reduced pressure, and the residue thus obtained was purified by silica gel column chromatography (hexane : ethyl acetate = 3 : 1 - 2 : 1, V/V) to give the desired title compound (10.4 g, yield 88%).
¹H-NMR (CDCl₃) δ: 1.54 (3H, t, J = 7.2 Hz), 4.66 (2H, q, J = 7.2 Hz), 6.60 (1H, d, J = 2.4 Hz), 6.95 (1H, d, J = 9.5 Hz), 7.65 (1H, d, J = 9.5 Hz), 7.97 (1H, d, J = 2.4 Hz), 11. 60 (1H, s).

### (11c) Pyrazolo[1,5-a]pyridin-6-ol

Into methanol (150 mL), ethyl 6-hydroxypyrazolo[1,5-a]pyridine-7-carboxylate (11.4 g, 55.4 mmol) produced in Example 11 (11b) was dissolved, to which a 2.5 N aqueous solution of sodium hydroxide (260 mL) was added, followed by stirring at 50°C for two hours. The resulting reaction liquid was left to cool, to which concentrated hydrochloric acid was added to make the reaction liquid acidic, followed by stirring at room temperature for 30 minutes. To this, saturated sodium bicarbonate was added for neutralization, followed by extraction with ethyl acetate. The resulting organic layer was dried over anhydrous sodium sulfate. After filtration, the solvent was distilled off under reduced pressure, and the residue thus obtained was purified by silica gel column chromatography (hexane : ethyl acetate = 4 : 1 - 2 : 1, V/V) to give the desired title compound (4.14 g, yield 56%).
¹H-NMR (CDCl₃) δ: 6.41 (1H, d, J = 2.3 Hz), 6.88 (1H, dd, J = 9.6, 2.1 Hz), 7.33 (1H, d, J = 9.6 Hz), 7.78 (1H, d, J = 2.3 Hz), 8.02-8.03 (1H, m), 9.37 (1H, br s).

### (11d) Pyrazolo[1,5-a]pyridin-6-yl trifluoromethanesulfonate

Into dichloromethane (150 mL), pyrazolo[1,5-a]pyridin-6-ol (4.14 g, 30.9 mmol) produced in Example 11 (11c) was dissolved and the resulting mixture was ice cooled. To this, pyridine (3.00 mL, 37.0 mmol) and trifluoromethanesulfonic anhydride (5.71 mL, 34.0 mmol) were added, followed by stirring for one minute. The resulting reaction liquid was added to a saturated aqueous solution of sodium bicarbonate for extraction. Subsequently, the resulting organic layer was washed with a saturated aqueous solution of sodium bicarbonate and then dried over anhydrous sodium sulfate. After filtration, the solvent was distilled off under reduced pressure, and the residue thus obtained was purified by basic silica gel column chromatography (hexane : ethyl acetate = 4 : 1, V/V) to give the desired title compound (8.08 g, yield 98%).
¹H-NMR (CDCl₃) δ: 6.65 (1H, d, J = 2.3 Hz), 7.11 (1H, dd, J = 9.8, 1.8 Hz), 7.61 (1H, d, J = 9.8 Hz), 8.06 (1H, d, J = 2.3 Hz), 8.61 (1H, d, J = 1.8 Hz).

### (11e) 2-[4-(6-Pyridin-4-ylpyrazolo[1,5-a]pyridin-3-yl)phenoxy]ethanol

Using pyrazolo[1,5-a]pyridin-6-yl trifluoromethanesulfonate produced in Example 11 (11d), the desired title compound was obtained by the same method as in Example 9 (9f), Example 2 (2b), Example 2 (2c), and Example 4 (4e).
¹H-NMR (DMSO-D₆) δ: 3.75 (2H, t, J = 5.0 Hz), 4.04 (2H, t, J = 5.0 Hz), 7.04-7.08 (2H, m), 7.63-7.66 (2H, m), 7.76 (1H, dd, J = 9.4, 1.6 Hz), 7.88-7.89 (2H, m), 8.04 (1H, dd, J = 9.4, 0.9 Hz), 8.41 (1H, s), 8.66-8.67 (2H, m), 9.33 (1H, dd, J = 1.6, 0.9 Hz).

### <Example 12>

### 4-{2-[4-(1H-Benzimidazol-1-yl)phenoxy]ethoxy}benzoic acid hydrochloride

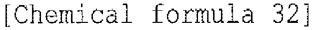

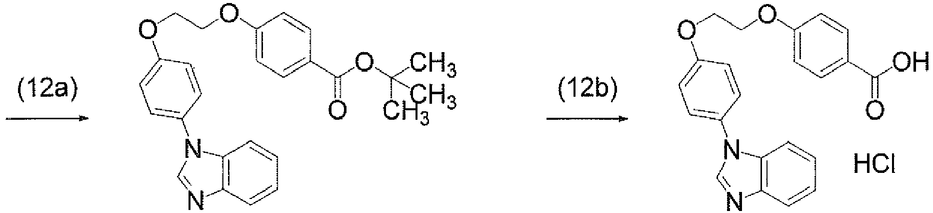

### (12a) Tert-butyl 4-{2-[4-(1H-benzimidazol-1-yl)phenoxy]ethoxy}benzoate

Into tetrahydrofuran (7 mL), 4-(1H-benzimidazol-1-yl)phenol (144.4 mg, 686.9 µmol) produced by the same method as in Example 1 (1a) and Example 9 (9d) was dissolved, to which tributylphosphine (257 µL, 1.03 mmol), 1,1'-(azodicarbonyl)dipiperidine (260 mg, 1.03 mmol), and tert-butyl 4-(2-hydroxyethoxy)benzoate (245 mg, 1.03 mmol) produced in Reference Example 7 were added under a nitrogen atmosphere, followed by stirring at room temperature for two hours. Insoluble matters were filtered off and the solvent was distilled off under reduced pressure. To the residue thus obtained, water and ethyl acetate were added for extraction, and the resulting organic layer was washed with saturated brine and dried over anhydrous sodium sulfate. After filtration, the solvent was distilled off under reduced pressure, and the residue was purified by silica gel chromatography (hexane : ethyl acetate, 100 : 0 - 50 : 50, V/V) to give the desired title compound (203.5 mg, yield 69%).
¹H-NMR (CDCl₃) δ: 1.59 (9H, s), 4.43 (4H, s), 6.96-6.99 (2H, m), 7.12-7.15 (2H, m), 7.31-7.36 (2H, m), 7.42-7.47 (3H, m), 7.86-7.90 (1H, m), 7.95-7.99 (2H, m), 8.06 (1H, s) .

### (12b) 4-{2-[4-(1H-Benzimidazol-1-yl)phenoxy]ethoxy}benzoic acid hydrochloride

Into 1,4-dioxane (2 mL), tert-butyl 4-{2-[4-(1H-benzimidazol-1-yl)phenoxy]ethoxy}benzoate (203.5 mg, 472.7 µmol) produced in (18a) was dissolved, to which a 4N hydrochloric acid / dioxane solution (8 mL) was added, followed by stirring at room temperature for 16 hours. The solvent was then distilled off under reduced pressure and the resulting product was subjected to azeotropic distillation with toluene. A solid precipitated, which was dried under reduced pressure to give the desired title compound (201.7 mg, yield 100%).
¹H-NMR (DMSO-D₆) δ: 4.47 (4H, br s), 7.09-7.12 (2H, m), 7.30-7.33 (2H, m), 7.57-7.63 (2H, m), 7.68-7.70 (1H, m), 7.72-7.75 (2H, m), 7.90-7.96 (3H, m), 9.65 (1H, s).

### <Example 13>

### 4-{1-[4-(2-Hydroxyethoxy)phenyl]-1H-benzimidazol-5-yl}benzoic acid

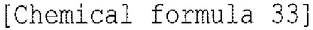

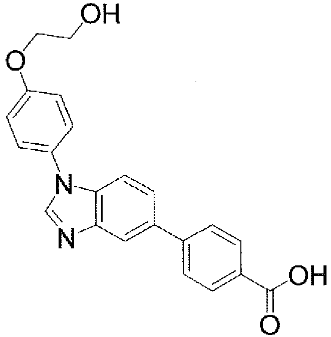

Into ethanol (3 mL), ethyl 4-(1-{4-[2-(tetrahydro-2H-pyran-2-yloxy)ethoxy]phenyl}-1H-benzimidazol-5-yl)benzoate (94.5 mg, 194 µmol) produced by the same method as in Example 9 (9f) using 1-{4-[2-(tetrahydro-2H-pyran-2-yloxy)ethoxy]phenyl}-1H-benzimidazol-5-yl trifluoromethanesulfonate was dissolved, to which a 1N aqueous solution of sodium hydroxide (2 mL) was added, followed by stirring at room temperature for 16 hours. The solvent was then distilled off under reduced pressure. The residue was dissolved in ethanol (3 mL), to which a 2N hydrochloric acid / ethanol solution (1 mL) was added, followed by stirring at room temperature for two hours. The solvent was then distilled off under reduced pressure, and the residue was purified by reverse phase silica gel column chromatography (cosmosil and water : acetonitrile, 100 : 0 - 50 : 50, V/V) to give the desired title compound (12.6 mg, yield 17%).
¹H-NMR (DMSO-D₆) δ: 3.75-3.79 (2H, m), 4.10 (2H, t, J = 4.8 Hz), 4.93 (1H, br s), 7.18-7.22 (2H, m), 7.60-7.63 (3H, m), 7.70 (1H, dd, J = 8.5, 1.6 Hz), 7.88 (2H, d, J = 8.5 Hz), 8.03 (2H, d, J = 8.7 Hz), 8.13 (1H, d, J = 1.6 Hz), 8.55 (1H, s).

### <Example 14>

### N-(4-{1-[4-(2-Hydroxyethoxy)phenyl]-1H-benzimidazol-5-yl}phenyl)acetamide

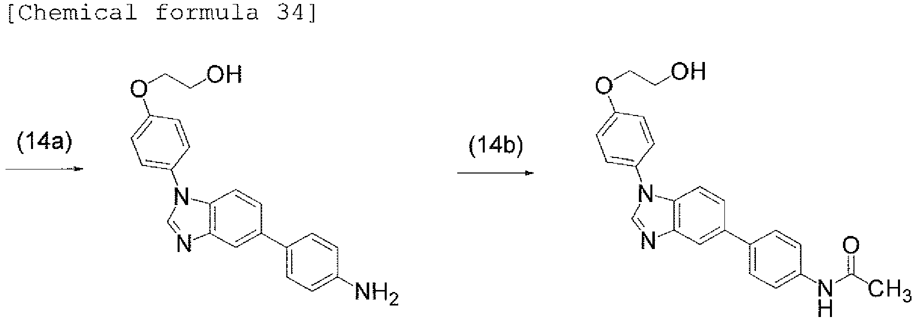

### (14a) 2-{4-[5-(4-Aminophenyl)-1H-benzimidazol-1-yl]phenoxy}ethanol

Into dichloromethane (10 mL), tert-butyl [4-(1-{4-[2-(tetrahydro-2H-pyran-2-yloxy)ethoxy]phenyl)-1H-benzimidazol-5-yl)phenyl]carbamate (634 mg, 1.17 mmol) produced by the same method as in Example 9 (9f) using 1-{4-[2-(tetrahydro-2H-pyran-2-yloxy)ethoxy]phenyl}-1H-benzimidazol-5-yl trifluoromethanesulfonate was dissolved, to which trifluoroacetic acid (3 mL) was added, followed by stirring at room temperature for four hours. The solvent was then distilled off under reduced pressure, to which water and ethyl acetate were added for extraction. The resulting organic layer was sequentially washed with water and saturated brine, and then dried over anhydrous sodium sulfate. After filtration, the solvent was distilled off under reduced pressure, and the residue was purified by silica gel chromatography (hexane : ethyl acetate, 100 : 0 - 20 : 80, V/V) to give the desired title compound (217.2 mg, yield 54%).
¹H-NMR (CDCl₃) δ: 4.38 (2H, t, J = 4.6 Hz), 4.77 (2H, t, J = 4.6 Hz), 6.78-6.81 (2H, m), 7.12-7.15 (2H, m), 7.46-7.51 (5H, m), 7.63 (1H, d, J = 8.6 Hz), 8.06 (1H, s), 8.54 (1H, br s).

### (14b) N-(4-{1-[4-(2-Hydroxyethoxy)phenyl]-1H-benzimidazol-5-yl}phenyl)acetamide

Into dichloromethane (2 mL), 2-{4-[5-(4-aminophenyl)-1H-benzimidazol-1-yl]phenoxy}ethanol (95.1 mg, 275 µmol) produced in Example 14 (14a) was dissolved, to which pyridine (2 mL) and acetic anhydride (31.2 µL, 330 µmol) were added, followed by stirring at room temperature for 16 hours. The solvent was then distilled off under reduced pressure, to which water (10 mL) was added, followed by extraction with ethyl acetate. The resulting organic layer was sequentially washed with water and saturated brine, and then dried over anhydrous sodium sulfate. After filtration, the solvent was distilled off under reduced pressure, and the residue thus obtained was purified by basic silica gel chromatography (hexane : ethyl acetate, 100 : 0 - 0 : 100, V/V, ethyl acetate : methanol, 100 : 0 - 90 : 10, V/V) to give the desired title compound (21.7 mg, yield 20%). ¹H-NMR (CDCl₃) δ: 2.22 (3H, s), 4.02-4.06 (2H, m), 4.19 (2H, t, J = 3.9 Hz), 7.12 (2H, d, J = 7.8 Hz), 7.19 (1H, s), 7.45 (2H, d, J = 7.8 Hz), 7.49-7.56 (2H, m), 7.58-7.65 (4H, m), 8.05 (1H, s), 8.08 (1H, s).

### <Example 15>

### 4-{1-[4-(2-Hydroxyethoxy)phenyl]-1H-benzimidazol-5-yl}benzamide dihydrochloride

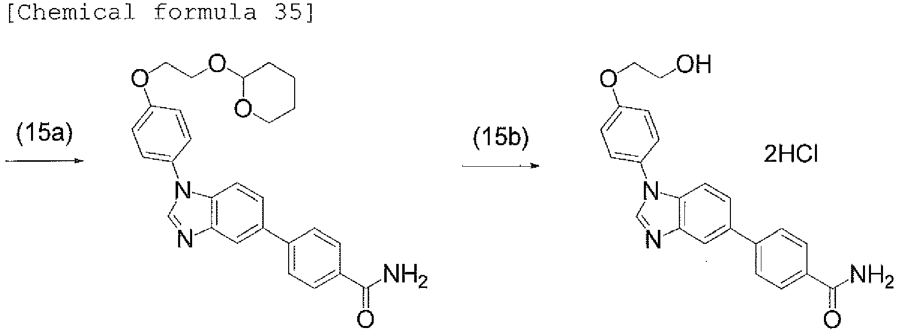

### (15a) 4-(1-{4-[2-(Tetrahydro-2H-pyran-2-yloxy)ethoxy]phenyl}-1H-benzimidazol-5-yl)benzamide

Into toluene (5 mL), ethyl 4-(1-{4-[2-(tetrahydro-2H-pyran-2-yloxy)ethoxy]phenyl}-1H-benzimidazol-5-yl)benzoate (150 mg, 308 µmol) produced by the same method as in Example 9 (9f) using 1-{4-[2-(tetrahydro-2H-pyran-2-yloxy)ethoxy]phenyl}-1H-benzimidazol-5-yl trifluoromethanesulfonate was dissolved, to which ammonium chloride (46.2 mg, 863 µmol) and a 1.8 M trimethylammonium / toluene solution (428 µL, 770 µmol) were added under a nitrogen atmosphere, followed by stirring at 70°C for two hours. To the reaction liquid, sodium sulfate decahydrate (50 mg) was added, followed by stirring. Subsequently, insoluble matters were filtered off while washing with ethyl acetate. The solvent was then distilled off under reduced pressure, and the residue thus obtained was purified by basic silica gel chromatography (hexane : ethyl acetate, 90 : 10 - 0 : 100, V/V, ethyl acetate : methanol, 100 : 0 - 90 : 10, V/V) to give the desired title compound (29.8 mg, yield 21%).
¹H-NMR (CDCl₃) 5: 1.50-1.58 (2H, m), 1.59-1.70 (2H, m), 1.74-1.88 (2H, m), 3.54-3.59 (1H, m), 3.85-3.96 (2H, m), 4.09-4.15 (1H, m), 4.24-4.27 (2H, m), 4.74 (1H, t, J = 3.7 Hz), 7.12-7.15 (2H, m), 7.43-7.45 (2H, m), 7.53 (1H, d, J = 8.7 Hz), 7.59 (1H, dd, J = 8.7, 1.6 Hz), 7.79-7.77 (2H, m), 7.90-7.93 (2H, m), 8.11 (2H, br s).

### (15b) 4-{1-[4-(2-Hydroxyethoxy)phenyl]-1H-benzimidazol-5-yl}benzamide dihydrochloride

Into ethanol (3 mL), 4-(1-{4-[2-(tetrahydro-2H-pyran-2-yloxy)ethoxy]phenyl}-1H-benzimidazol-5-yl)benzamide (29.8 mg, 65.1 µmol) produced in Example 15 (15a) was dissolved, to which 1N hydrochloric acid (163 µL, 163 µmol) was added, followed by stirring at room temperature for three hours. The solvent was then distilled off under reduced pressure. A solid precipitated, which was washed with a mixed solution of hexane / ethyl acetate (9 / 1) and then dried under reduced pressure to give the desired title compound (16.1 mg, yield 66%).
¹H-NMR (DMSO-D₆) 5: 3.78 (2H, t, J = 4.8 Hz), 4.12 (2H, t, J = 4.8 Hz), 7.23-7.27 (2H, m), 7.68-7.74 (3H, m), 7.85-7.88 (3H, m), 8.00-8.03 (2H, m), 8.18 (1H, d, J = 1.4 Hz), 9.37 (1H, s).

### <Example 16>

### 2-(4-{5-[6-(Morpholin-4-ylcarbonyl)pyridin-3-yl]-1H-benzimidazol-1-yl}phenoxy)ethanol

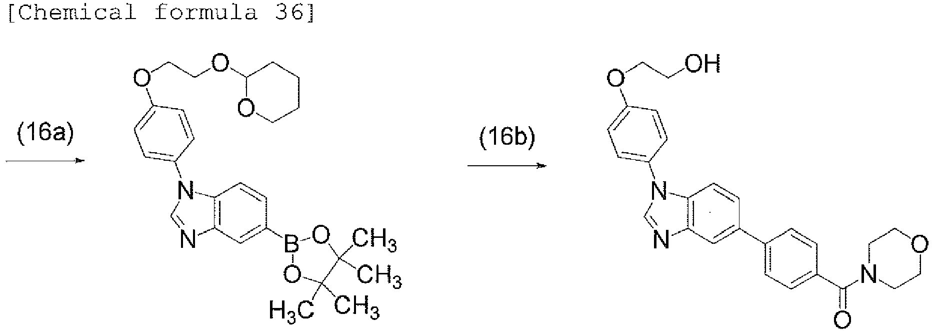

### (16a) 1-{4-[2-(Tetrahydro-2H-pyran-2-yloxy)ethoxy]phenyl}-5-(4,4,5,5,-tetramethyl-1,3,2-dioxaborolan-2-yl)-1H-benzimidazole

Into dimethylsulfoxide (6 mL), 1-{4-[2-(tetrahydro-2H-pyran-2-yloxy)ethoxy]phenyl}-1H-benzimidazol-5-yl trifluoromethanesulfonate (600 mg, 1.23 mmol) produced in Example 9 (9e), bis(pinacolato)diboron (376 mg, 1.48 mmol), [1,1'-bis(diphenylphosphino)ferrocene]dichloropalladium(II) complex with dichloromethane (302 mg, 0.369 mmol), and potassium acetate (363 mg, 3.69 mmol) were dissolved, followed by stirring at 80°C for 20 minutes under an argon atmosphere. To the resulting reaction liquid, ethyl acetate and water were added, and the organic layer thus separated was dried over anhydrous sodium sulfate. After filtration, the solvent was distilled off under reduced pressure and the residue thus obtained was purified by silica gel column chromatography (hexane : ethyl acetate = 2 : 1 - 50 : 50, V/V) to give the desired title compound (575 mg, yield 100%).
¹H-NMR (CDCl₃) δ: 1.38 (12H, s), 1.52-1.89 (6H, m), 3.53-3.59 (1H, m), 3.84-3.95 (2H, m), 4.08-4.14 (1H, m), 4.21-4.27 (2H, m), 4.74 (1H, t, J = 3.5 Hz), 7.09-7.13 (2H, m), 7.38-7.41 (2H, m), 7.43 (1H, d, J = 8.3 Hz), 7.76 (1H, d, J = 8.3 Hz), 8.07 (1H, s), 8.34 (1H, s).

### (16b) 2-(4-{5-[6-(Morpholin-4-ylcarbonyl)pyridin-3-yl]-1H-benzimidazol-1-yl}phenoxy)ethanol

Water (72 µL) and ethanol (3 mL) were added to 1-{4-[2-(tetrahydro-2H-pyran-2-yloxy)ethoxy]phenyl}-5-(4,4,5,5,-tetramethyl-1,3,2-dioxaborolan-2-yl)-1H-benzimidazole (180 mg, 0.398 mmol) produced in Example 16 (16a), dichlorobis(triphenylphosphine)palladium(II) (83 mg, 0.12 mmol), potassium carbonate (161 mg, 1.16 mmol), and 4-[(5-bromopyridin-2-yl)carbonyl]morpholine (158 mg, 0.581 mmol), followed by stirring at 80°C for one hour under an argon atmosphere. The solvent was then distilled off under reduced pressure, and the residue was purified by silica gel column chromatography (dichloromethane : methanol = 100 : 0 - 95 : 5, V/V). The purified product was dissolved in methanol (5 mL), to which a 4N hydrochloric acid / dioxane solution (5 mL) was added, followed by stirring at room temperature for two hours. The solvent was then distilled off under reduced pressure, and to the residue thus obtained, dichloromethane and a saturated aqueous solution of sodium bicarbonate were added for extraction. The resulting organic layer was dried over anhydrous sodium sulfate and the solvent was then distilled off under reduced pressure. The residue thus obtained was purified by basic silica gel column chromatography (dichloromethane : methanol = 100 : 0 - 97 : 3, V/V). The solvent was then distilled off under reduced pressure, and to the residue thus obtained, diethyl ether was added. A solid precipitated, which was collected by filtration to give the desired title compound (109 mg, yield 62%).
¹H-NMR (CDCl₃) δ: 2.12 (1H, t, J = 5.7 Hz), 3.73-3.76 (2H, m), 3.78-3.81 (2H, m), 3.83-3.87 (4H, m), 4.03-4.06 (2H, m), 4.19 (2H, t, J = 4.6 Hz), 7.12-7.15 (2H, m), 7.44-7.47 (2H, m), 7.54-7.58 (2H, m), 7.81 (1H, d, J = 8.0 Hz), 8.07 (1H, dd, J = 8.0, 2.3 Hz), 8.10-8.10 (1H, m), 8.13 (1H, s), 8.88 (1H, d, J = 2.3 Hz).

### <Example 17>

### 7-[4-(Morpholin-4-ylcarbonyl)phenyl]-3-{4-[2-(tetrahydro-2H-pyran-4-yloxy)ethoxy]piperidin-1-yl}imidazo[1,2-a]pyridine

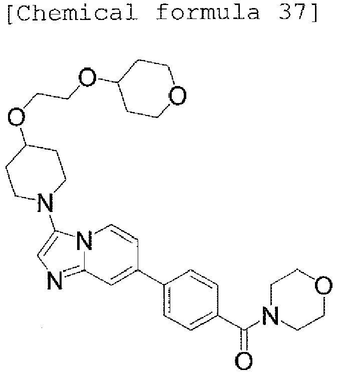

Into toluene (4 mL), ethyl 4-(3-{4-[2-(tetrahydro-2H-pyran-4-yloxy)ethoxy]piperidin-1-yl}imidazo[1,2-a]pyridin-7-yl)benzoate (109 mg, 0.23 mmol) produced by the same method as in Example 10 (10b) and Example 10 (10a) and morpholine (40 µL, 0.46 mmol) were dissolved, and nitrogen substitution was performed. A 15% trimethylaluminum / hexane solution (325 µL) was added dropwise, followed by stirring at room temperature overnight. Magnesium sulfate decahydrate (300 mg) was added and the resulting mixture was stirred for a while and then dried over anhydrous sodium sulfate. Insoluble matters were filtered off and the solvent was distilled off under reduced pressure. The residue thus obtained was purified by basic silica gel chromatography (hexane : ethyl acetate, 30 : 70 - 0 : 100, V/V) to give the desired title compound (86 mg, yield 71%).
¹H-NMR (CDCl₃) 5: 1.61-1.68 (2H, m), 1.79-1.97 (4H, m), 2.04-2.13 (2H, m), 2.86-2.95 (2H, m), 3.22-3.31 (2H, m), 3.40-3.89 (16H, m), 3.92-4.00 (2H, m), 7.07 (1H, dd, J = 7.1, 1.7 Hz), 7.31 (1H, s), 7.52 (2H, d, J = 8.3 Hz), 7.69 (2H, d, J = 8.3 Hz), 7.75 (1H, s), 8.00 (1H, d, J = 7.1 Hz).

### <Example 18>

### 3-{4-[2-(Tetrahydro-2H-pyran-2-yloxy)ethoxy]piperidin-1-yl}pyrazolo[1,5-a]pyridine hydrochloride

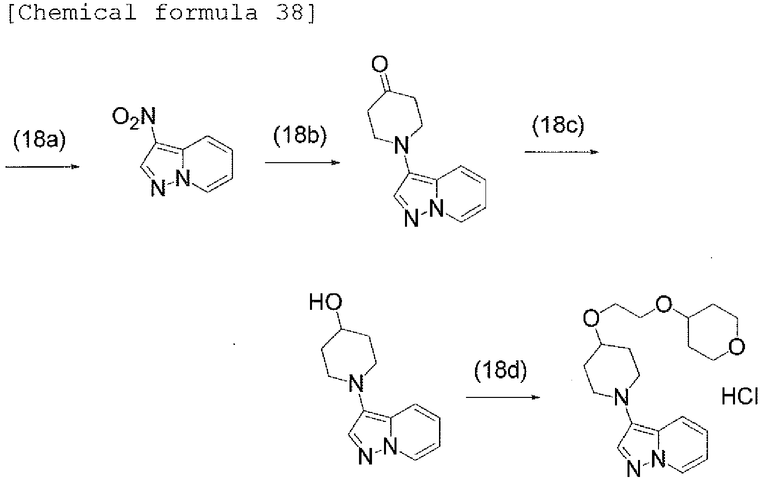

### (18a) 3-Nitropyrazolo[1,5-a]pyridine

Into concentrated sulfuric acid (7 mL), pyrazolo[1,5-a]pyridine (1.00 g, 8.47 mmol) was dissolved and the resulting mixture was cooled to 0°C. To this, fuming nitric acid (1 mL) was added dropwise. After stirring at 0°C for 10 minutes, the resulting reaction liquid was added to ice water, followed by extraction with dichloromethane. The resulting organic layer was dried over anhydrous sodium sulfate. After filtration, the solvent was distilled off under reduced pressure, and the residue thus obtained was purified by basic silica gel column chromatography (hexane : dichloromethane = 50 : 50, V/V). The solvent was then distilled off under reduced pressure, and to the residue thus obtained, diethyl ether was added. A solid precipitated, which was collected by filtration to give the desired title compound (653 mg, yield 47%).
¹H-NMR (CDCl₃) 5: 7.16 (1H, dt, J = 1.5, 7.0 Hz), 7.67-7.71 (1H, m), 8.37-8.39 (1H, m), 8.58-8.60 (1H, m), 8.64 (1H, s).

### (18b) 1-Prazolo[1,5-a]pyridin-3-ylpiperidin-4-one

Into ethanol / water (5 / 1, 120 mL), 3-nitropyrazolo[1,5-a]pyridine (1.00 g, 6.13 mmol) produced in Example 18 (18a) was dissolved, to which zinc (12.0 g, 184 mmol) and calcium chloride (680 mg, 6.13 mmol) were added. After heating under reflux for 30 minutes, insoluble matters were filtered off. The solvent was then distilled off under reduced pressure. The residue thus obtained was dissolved in N,N-dimethylformamide (30 mL), to which 1,5-dichloropentan-3-one (crude product, 7.88 mmol) produced by the method described in J. Chem. Soc. C, 1970, 2401, potassium carbonate(1.69 g, 12.3 mmol), and sodium iodide (459 mg, 3.07 mmol) were added, followed by stirring at room temperature for one hour, and for another hour at 70°C. Water and ethyl acetate were added for extraction, and the resulting aqueous layer was further extracted with dichloromethane. The combined organic layer was washed with saturated brine and dried over anhydrous sodium sulfate. After filtration, the solvent was distilled off under reduced pressure, and the residue thus obtained was purified by silica gel column chromatography (hexane : ethyl acetate = 3 : 1 - 1 : 2, V/V) to give the desired title compound (1.07 g, yield approximately 75%).
¹H-NMR (CDCl₃) δ: 2.66 (4H, t, J = 6.3 Hz), 3.39 (4H, t, J = 6.3 Hz), 6.73 (1H, dt, J = 6.9, 1.2 Hz), 7.05 (1H, ddd, J = 9.2, 6.9, 1.2 Hz), 7.54 (1H, td, J = 9.2, 1.2 Hz), 7.78 (1H, s), 8.36 (1H, br d, J = 6.9 Hz).

### (18c) 1-Pyrazolo[1,5-a]pyridin-3-ylpiperidin-4-ol

Into methanol (30 mL), 1-pyrazolo[1,5-a]pyridin-3-ylpiperidin-4-one (1.07 g, approximately 4.58 mmol) produced in Example 18 (18b) was dissolved, to which sodium borohydride (208 mg, 5.50 mmol) was added in several divided portions, followed by stirring at room temperature for one hour. To the resulting reaction liquid, water and ethyl acetate were added for extraction, and the resulting aqueous layer was further extracted with dichloromethane. The combined organic layer was dried over anhydrous sodium sulfate and the solvent was distilled off under reduced pressure. After filtration, the residue thus obtained was purified by basic silica gel column chromatography (hexane : ethyl acetate = 50 : 50, V/V) to give the desired title compound (904 mg, yield 91%).
¹H-NMR (CDCl₃) δ: 1. 64 (1H, d, J = 4. 6 Hz), 1.77-1.84 (2H, m), 2.03-2.09 (2H, m), 2.84-2.89 (2H, m), 3.29-3.33 (2H, m), 3.84-3.89 (1H, m), 6.68 (1H, dt, J = 6.9, 1.2 Hz), 6.97-7.00 (1H, m), 7.50 (1H, d, J = 9.2 Hz), 7.72 (1H, s), 8.33 (1H, d, J = 6.9 Hz).

### (18d) 3-{4-[2-(Tetrahydro-2H-pyran-2-yloxy)ethoxy]piperidin-1-yl}pyrazolo[1,5-a]pyridine hydrochloride

Into N,N-dimethylformamide (20 mL), 1-pyrazolo[1,5-a]pyridin-3-ylpiperidin-4-ol (904 mg, 4.16 mmol) produced in Example 18 (18c) and 2-(tetrahydro-2H-pyran-4-yloxy)ethyl 4-methyl benzenesulfonate(1.87 g, 6.24 mmol) were dissolved, to which sodium hydride (content 55%) (290 mg, 6.66 mmol) was added, followed by stirring at room temperature for one hour, and for another two hours at 60°C. The resulting reaction liquid was left to cool, to which water and ethyl acetate were added for extraction. The resulting organic layer was washed with saturated brine and then dried over anhydrous sodium sulfate. After filtration, the solvent was distilled off under reduced pressure and the residue thus obtained was purified by basic silica gel column chromatography (hexane : ethyl acetate = 90 : 10 - 50 : 50, V/V) and by silica gel column chromatography (hexane : ethyl acetate = 3 : 1 - 0 : 100, V/V). The purified product was dissolved in dichloromethane, to which a 4N hydrochloric acid / dioxane solution (1.0 mL) was added, followed by stirring at room temperature for one hour. The solvent was distilled off under reduced pressure. The residue was dissolved in water, which was freeze dried to give the desired title compound (270 mg, yield 19%).
¹H-NMR (CD₃OD) δ: 1.67-2.08 (2H, m), 1.90-1.96 (2H, m), 2.20-2.27 (2H, m), 2.37-2.44 (2H, m), 3.42-3.46 (2H, m), 3.57-3.63 (1H, m), 3.69-3.79 (6H, m), 3.87-3.98 (5H, m), z7 (1H, dt, J = 6.9, 1.2 Hz), 7.44-7.47 (1H, m), 8.11 (1H, d, J = 9.2 Hz), 8.38 (1H, s), 8.62 (1H, d, J = 6.9 Hz).

### <Example 19>

### 3-{4-[2-(Tetrahydro-2H-pyran-4-yloxy)ethoxy]phenyl}imidazo[1,2-a]pyridine-6-carboxamide

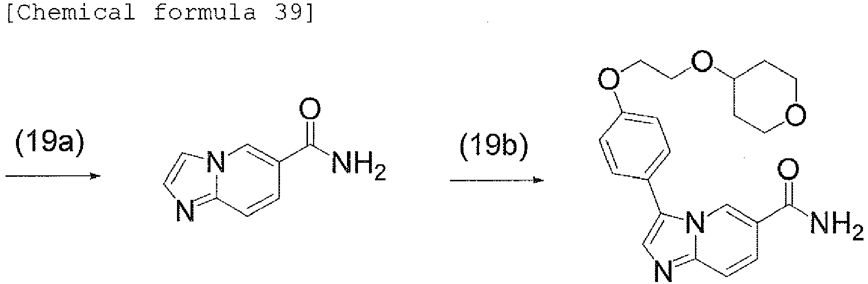

### (19a) Imidazo[1,2-a]pyridine-6-carboxamide

Into methanol / water (3 / 1, 40 mL), methyl imidazo[1,2-a]pyridine-6-carboxylate (1.00 g, 5.68 mmol) was dissolved, to which lithium hydroxide monohydrate (286 mg, 6.82 mmol) was added, followed by stirring at room temperature for 45 minutes, and then at 60°C overnight. The resulting reaction liquid was left to cool, to which ammonium chloride (911 mg, 17.0 mmol) and 4-(4,6-dimethoxy-1,3,5-triazin-2-yl)-4-methylmorpholinium chloride (3.14 g, 11.4 mmol) were added, followed by stirring at room temperature for two hours. To the reaction liquid, water and dichloromethane were added for extraction, and the resulting aqueous layer was further extracted with ethyl acetate. The combined organic layer was dried over anhydrous sodium sulfate, and the solvent was distilled off under reduced pressure. After filtration, the residue thus obtained was purified by basic silica gel column chromatography (hexane : ethyl acetate = 50 : 50, V/V, dichloromethane : methanol = 90 : 10, V/V) to give the desired title compound (0.37 g, yield 40%).
¹H-NMR (DMSO-D₆) δ: 7.51 (1H, br s), 7.59 (1H, d, J = 9.2 Hz), 7.64-7.67 (2H, m), 8.05-8.07 (2H, m), 9.12-9.13 (1H, m).

### (19b) 3-{4-[2-(Tetrahydro-2H-pyran-4-yloxy)ethoxy]phenyl}imidazo[1,2-a]pyridine-6-carboxamide

Using imidazo[1,2-a]pyridine-6-carboxamide produced in Example 19 (19a), the desired title compound was obtained by the same method as in Example 2 (2b) and Example 8 (8a).
¹H-NMR (DMSO-D₆) δ: 1.39-1.46 (2H, m), 1.86-1.91 (2H, m), 3.30-3.39 (2H, m), 3.56-3.61 (1H, m), 3.80-3.84 (4H, m), 4.18-4.20 (2H, m), 7.15-7.18 (2H, m), 7.53 (1H, br s), 7.59-7.62 (2H, m), 7.66-7.72 (2H, m), 7.75 (1H, s), 8.19 (1H, br s), 8.91-8.92 (1H, m).

### <Example 20>

### 6-Methoxy-3-{4-[2-(tetrahydro-2H-pyran-4-yloxy)ethoxy]phenyl}imidazo[1,2-a]pyridine

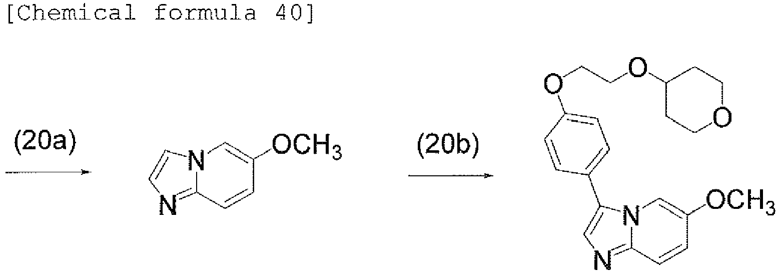

### (20a) 6-Methoxyimidazo[1,2-a]pyridine

Into a mixed solution of toluene (10 mL) and methanol (5 mL), 6-iodoimidazo[1,2-a]pyridine(1.0 g, 4.1 mmol) was dissolved, to which copper(I) iodide (160 mg, 0.84 mmol), 1,10-phenanthroline (300 mg, 1.66 mmol), and cesium carbonate (3 g, 9 mmol) were added, followed by stirring at 120°C overnight. The resulting reaction liquid was left to cool, to which water and ethyl acetate were added for extraction. The resulting organic layer was sequentially washed with water and saturated brine and then dried over anhydrous sodium sulfate. The solvent was then distilled off under reduced pressure. After filtration, the residue thus obtained was purified by silica gel column chromatography (hexane : ethyl acetate : methanol, 50 : 50 : 0 - 0 : 100 : 0 - 0 : 95 : 5 V/V) to give the desired title compound (360 mg, yield 59%).
¹H-NMR (CDCl₃) δ: 3.82 (3H, s), 6.96 (1H, dd, J = 9.6, 2.3 Hz), 7.50 (1H, d, J = 9.6 Hz), 7.52 (1H, s), 7.57 (1H, s), 7.66 (1H, d, J = 2.3 Hz).

### (20b) 6-Methoxy-3-{4-[2-(tetrahydro-2H-pyran-4-yloxy)ethoxy]phenyl}imidazo[1,2-a]pyridine

Using 6-methoxyimidazo[1,2-a]pyridine produced in Example 20 (20a), the title compound was obtained by the same method as in Example 2 (2b) and Example 8 (8a).
¹H-NMR (CDCl₃) 5: 1.61-1.71 (2H, m), 1.91-1.99 (2H, m), 3.43-3.51 (2H, m), 3.59-3.66 (1H, m), 3.77 (3H, s), 3.86-3.90 (2H, m), 3.94-4.01 (2H, m), 4.19-4.22 (2H, m), 6.96-7.01 (1H, m), 7.08 (2H, d, J = 8.7 Hz), 7.47 (2H, d, J = 8.7 Hz), 7.53-7.58 (2H, m), 7.74-7.76 (1H, m).

### <Example 21>

### 6-Ethynyl-3-{4-[2-(tetrahydro-2H-pyran-4-yloxy)ethoxy]phenyl}imidazo[1,2-a]pyridine

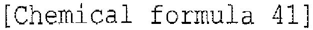

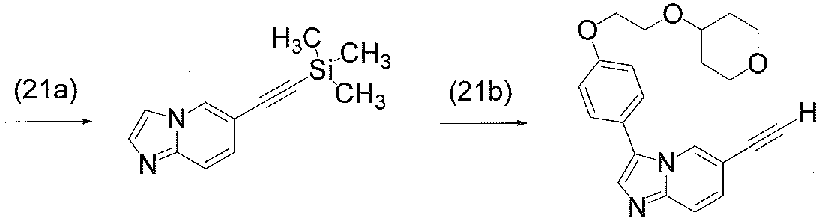

### (21a) 6-[(Trimethylsilyl)ethynyl]imidazo[1,2-a]pyridine

To triethylamine (20 mL), 6-iodoimidazo[1,2-a]pyridine (2.00 g, 8.20 mmol), trimethylsilylacetylene (4.02 g, 41.0 mmol), dichlorobis(triphenylphosphine)palladium(II) (1.17 g, 1.64 mmol), and copper(I) iodide (624 mg, 3.28 mmol) were added, followed by stirring at 60°C for 20 minutes under an argon atmosphere. Water and ethyl acetate were added, and insoluble matters were filtered off through Celite while washing with dichloromethane. After extraction, the organic layer thus separated was washed with water and then dried over anhydrous sodium sulfate. After filtration, the solvent was distilled off under reduced pressure, and the residue thus obtained was purified by silica gel column chromatography (dichloromethane : ethyl acetate = 1 : 0 - 1 : 1, V/V) and by basic silica gel column chromatography (hexane : ethyl acetate = 2 : 1, V/V) to give the desired title compound(1.73 g, yield 98%).
¹H-NMR (CDCl₃) δ: 0.26 (9H, s), 7.18 (1H, dd, J = 9.3, 1.1 Hz), 7.53-7.55 (2H, m), 7.65 (1H, d, J = 1.1 Hz), 8.31-8.31 (1H, m).

### (21b) 6-Ethynyl-3-{4-[2-{tetrahydro-2H-pyran-4-yloxy)ethoxy]phenyl}imidazo[1,2-a]pyridine

Using 6-[(trimethylsilyl)ethynyl]imidazo[1,2-a]pyridine produced in Example 21 (21a), the desired title compound was obtained by the same method as in Example 2 (2b) and Example 8 (8a).
¹H-NMR (CDCl₃) δ: 1.62-1.69 (2H, m), 1.94-1.97 (2H, m), 3.09 (1H, s), 3.44-3.49 (2H, m), 3.60-3.65 (1H, m), 3.87-3.89 (2H, m), 3.97 (2H, td, J = 8.0, 3.6 Hz), 4.19-4.21 (2H, m), 7.07-7.10 (2H, m), 7.21 (1H, dd, J = 9.7, 1.7 Hz), 7.43-7.46 (2H, m), 7.60 (1H, d, J = 9.7 Hz), 7.65 (1H, s), 8.41 (1H, br s).

### <Example 22>

### 6-Morpholin-4-yl-3-{4-[2-(tetrahydro-2H-pyran-4-yloxy)ethoxy]phenyl}imidazo[1,2-a]pyridine hydrochloride

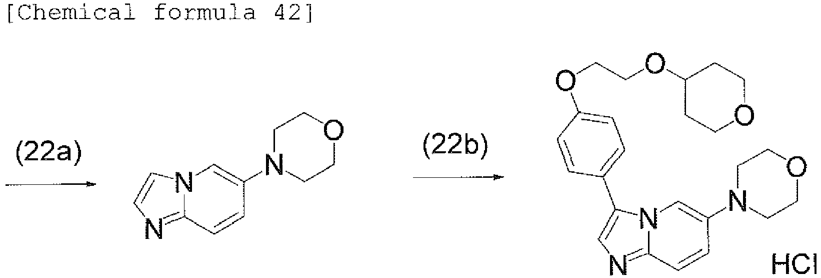

### (22a) 6-Morpholin-4-ylimidazo[1,2-a]pyridine

Toluene (6 mL) was added to 6-iodoimidazo[1,2-a]pyridine (302 mg, 1.23 mmol), morpholine (161 µL, 1.84 mmol), tris(dibenzylideneacetone)dipalladium (56 mg, 61 µmol), 2-dicyclohexylphosphino-2'-(N,N-dimethylamino)biphenyl (48.7 mg, 0.124 mmol), and sodium tert-butoxide (236 mg, 2.56 mmol), followed by stirring at 110°C for 21 hours under a nitrogen atmosphere. The resulting mixture was ice cooled, into which chloroform was poured, and insoluble matters were filtered off through Celite. The solvent was distilled off under reduced pressure and the residue thus obtained was purified by basic silica gel chromatography (hexane : ethyl acetate, 30 : 70 - 0 : 100, V/V) to give the desired title compound (99 mg, yield 39%).
¹H-NMR (CDCl₃) δ: 3.03-3.08 (4H, m), 3.86-3.91 (4H, m), 7.02-7.07 (1H, m), 7.49-7.58 (4H, m).

### (22b) 6-Morpholin-4-yl-3-{4-[2-(tetrahydro-2H-pyran-4-yloxy)ethoxy]phenyl}imidazo[1,2-a]pyridine hydrochloride

Using 6-morpholin-4-ylimidazo[1,2-a]pyridine produced in Example 22 (22a), the desired title compound was obtained by the same method as in Example 2 (2b), Example 8 (8a), and Example 4 (4e).
¹H-NMR (DMSO-D₆) δ: 1.37-1.48 (2H, m), 1.84-1.92 (2H, m), 3.14 (4H, t, J = 4.8 Hz), 3.31-3.38 (2H, m), 3.55-3.63 (1H, m), 3.76 (4H, t, J = 4.8 Hz), 3.78-3.85 (4H, m), 4.18-4.23 (2H, m), 7.20 (2H, d, J = 8.7 Hz), 7.67 (2H, d, J = 8.7 Hz), 7.73 (1H, d, J = 2.1 Hz), 7.92 (1H, d, J = 9.6 Hz), 7.99 (1H, dd, J = 9.6, 2.1 Hz), 8.22 (1H, s).

### <Example 23>

### 6-(1H-Pyrazol-1-yl)-3-{4-[2-(tetrahydro-2H-pyran-4-yloxy)ethoxylphenyl]imidazo[1,2-a]pyridine

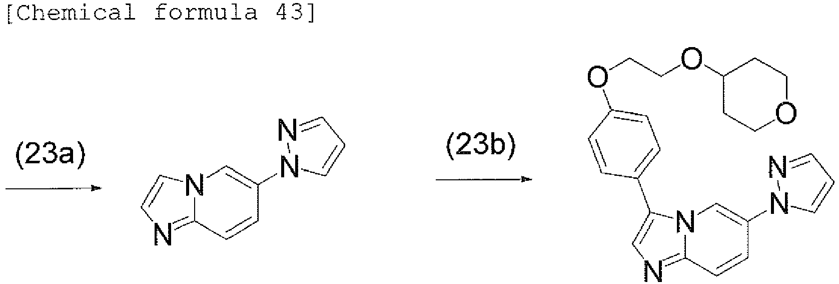

### (23a) 6-(1H-Pyrazol-1-yl)imidazo[1,2-a]pyridine

To 6-iodoimidazo[1,2-a]pyridine (300 mg, 1.23 mmol), pyrazole (108 mg , 1.59 mmol), copper(I) iodide (23 mg, 0.12 mmol), (1S,2S)-cyclohexane-1,2-diamine (28 mg, 0.25 mmol), and potassium phosphate (522 mg, 2.46 mmol), 1,2-dimethoxyethane (10 mL) was added, followed by stirring at 110°C for six days under a nitrogen atmosphere. The resulting mixture was left to cool, into which chloroform (15 mL) was poured, and insoluble matters were filtered off through Celite. The solvent was distilled off under reduced pressure and the residue thus obtained was purified by basic silica gel chromatography (hexane : ethyl acetate, 30 : 70 - 0 : 100, V/V) to give the desired title compound (91 mg, yield 40%).
¹H-NMR (CDCl₃) δ: 6.52 (1H, t, J = 2.1 Hz), 7.51 (1H, dd, J = 9.6, 2.1 Hz), 7.66-7.76 (4H, m), 7.88 (1H, d, J = 2.1 Hz), 8.63-8.68 (1H, m).

### (23b) 6-(1H-Pyrazol-1-yl)-3-{4-[2-(tetrahydro-2H-pyran-4-yloxy)ethoxy]phenyl}imidazo[1,2-a]pyridine

Using 6-(1H-pyrazol-1-yl)imidazo[1,2-a]pyridine produced in Example 23 (23a), the desired title compound was obtained by the same method as in Example 2 (2b) and Example 8 (8a).
¹H-NMR (CDCl₃) δ: 1.61-1.71 (2H, m), 1.92-2.00 (2H, m), 3.43-3.51 (2H, m), 3.59-3.67 (1H, m), 3.88 (2H, t, J = 5.0 Hz), 3.94-4.01 (2H, m), 4.21 (2H, t, J = 5.0 Hz), 6.48-6.51 (1H, m), 7.09 (2H, d, J = 8.7 Hz), 7.48-7.53 (3H, m), 7.68-7.77 (3H, m), 7.85 (1H, d, J = 2.7 Hz), 8.70 (1H, s).

### <Example 24>

### 1-{4-[2-(Tetrahydro-2H-pyran-4-yloxy)ethoxy]phenyl)-1H-benzimidazole-6-carbonitrile hydrochloride

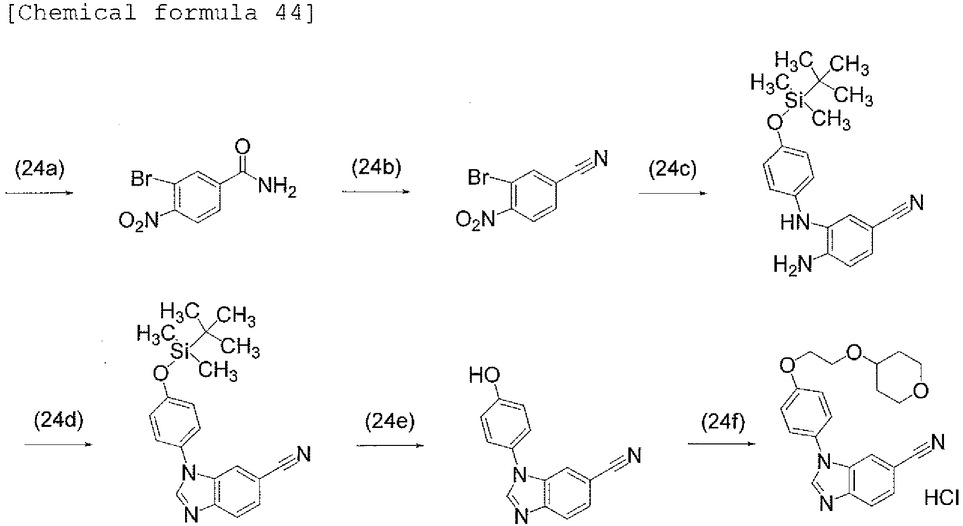

### (24a) 3-Bromo-4-nitrobenzamide

Into acetonitrile (50 mL), 3-bromo-4-nitrobenzoic acid (4.70 g, 19.1 mmol) was dissolved, to which 4-(4,6-dimethoxy-1,3,5-triazin-2-yl)-4-methylmorpholinium chloride (7.9 g, 29 mmol) and a 7 N ammonia / methanol solution (14 mL, 98 mmol) were added, followed by stirring at room temperature for two hours. Water and dichloromethane were added for extraction, and the resulting organic layer was sequentially washed with water and saturated brine, and then dried over anhydrous sodium sulfate. After filtration, the solvent was distilled off under reduced pressure and the residue thus obtained was recrystallized from ethyl acetate / hexane to give the desired title compound (4.7 g, yield 100%).
¹H-NMR (DMSO-D₆) δ: 7.79 (1H, br s), 8.05 (1H, dd, J = 8.4, 1.8 Hz), 8.12 (1H, d, J = 8.4 Hz), 8.29 (1H, br s), 8.32 (1H, d, J = 1.8 Hz).

### (24b) 3-Bromo-4-nitrobenzonitrile

Into tetrahydrofuran (100 mL), 3-bromo-4-nitrobenzamide (5.19 g, 21.2 mmol) produced in Example 24 (24a) was dissolved, to which triethylamine (8.8 mL, 64 mmol) was added. While ice cooling, trifluoroacetic anhydride (4.4 mL, 32 mmol) was added dropwise over five minutes, followed by stirring at 0°C for one hour. To the resulting reaction liquid, water and ethyl acetate were added for extraction. The resulting organic layer was sequentially washed with water and saturated brine and then dried over anhydrous sodium sulfate. After filtration, the solvent was distilled off under reduced pressure and the residue thus obtained was purified by silica gel column chromatography (hexane : dichloromethane, 50 : 50 - 0 : 100, V/V) to give the desired title compound (4.2 g, yield 87%).
¹H-NMR (CDCl₃) δ: 7.78 (1H, dd, J = 8.3, 1.7 Hz), 7.91 (1H, d, J = 8.3 Hz), 8.06 (1H, d, J = 1.7 Hz).

### (24c) 4-Amino-3-[(4-{[tert-butyl(dimethyl)silyl]oxy}phenyl)amino]benzonitrile

Using 3-bromo-4-nitrobenzonitrile (4.20 g, 18.5 mmol) produced in Example 24 (24b) and 4-{[tert-butyl(dimethyl)silyl]oxy}aniline (4.6 g, 20.6 mmol), a crude product of 3-[(4-{[term-butyl(dimethyl)silyl]oxy}phenyl)amino]-4-nitrobenzonitrile (2.6 g) was produced by the same method as in Example 22. This crude product was dissolved in acetic acid (150 mL), to which zinc (11.8 g, 180 mmol) was added, followed by stirring at room temperature for two hours. Insoluble matters were filtered off while washing with acetic acid. The solvent was distilled off under reduced pressure, to which ethyl acetate was added, and the resulting mixture was neutralized with a saturated aqueous solution of sodium bicarbonate. After extraction, the resulting organic layer was sequentially washed with water and saturated brine and then dried over anhydrous sodium sulfate. After filtration, the solvent was distilled off under reduced pressure and the residue thus obtained was purified by silica gel column chromatography (hexane : ethyl acetate, 0 : 100 - 40 : 60, V/V) to give the desired title compound (2.9 g, yield 47%).
¹H-NMR (CDCl₃) δ: 0.19 (6H, s), 0.99 (9H, s), 4.17 (1H, br s), 4.92 (1H, br s), 6.69-6.79 (4H, m), 7.19-7.23 (1H, m), 7.25-7.28 (2H, m).

### (24d) 1-(4-{[Tert-butyl(dimethyl)silyl]oxy}phenyl)-1H-benzimidazole-6-carbonitrile

Using 4-amino-3-[(4-{[tert-butyl(dimethyl)silyl]oxy}phenyl)amino]benzonitrile (2.90 g, 8.54 mmol) produced in Example 24 (24d), the desired title compound (2.44 g, yield 82%) was obtained by the same method as in Example 9 (9c).
¹H-NMR (CDCl₃) δ: 0.28 (6H, s), 1.03 (9H, s), 7.05 (2H, d, J = 8.7 Hz), 7.33 (2H, d, J = 8.7 Hz), 7.57-7.61 (1H, m), 7.79-7.80 (1H, m), 7.92-7.95 (1H, m), 8.21 (1H, s).

### (24e) 1-(4-Hydroxyphenyl)-1H-benzimidazole-6-carbonitrile

Into tetrahydrofuran (25 mL), 1-(4-{[tert-butyl(dimethyl)silyl]oxy}phenyl)-1H-benzimidazole-6-carbonitrile (2.44 g, 6.98 mmol) produced in Example 24 (24d) was dissolved, to which a 1 M tetrabutylammonium fluoride / tetrahydrofuran solution (8.4 mL, 8.4 mmol) was added, followed by stirring at room temperature for one hour. Water was added, and a solid precipitated, which was collected by filtration and dried under reduced pressure to give the desired title compound (1.58 g, yield 96%).
¹H-NMR (DMSO-D₆) δ: 6.99 (2H, d, J = 8.7 Hz), 7.50 (2H, d, J = 8.7 Hz), 7.66-7.70 (1H, m), 7.91-7.95 (1H, m), 8.01-8.03 (1H, m), 8.72 (1H, s), 9.95 (1H, s).

### (24f) 1-{4-[2-(Tetrahydro-2H-pyran-4-yloxy)ethoxy]phenyl}-1H-benzimidazole-6-carbonitrile hydrochloride

Into toluene (5 mL), 1-(4-hydroxyphenyl)-1H-benzimidazole-6-carbonitrile (400 mg, 1.45 mmol) produced in Example 24 (24e) and 2-(tetrahydro-2H-pyran-4-yloxy)ethanol (370 mg, 2.53 mmol) produced in Reference Example 3 were dissolved, to which (tributylphosphoranylidene)acetonitrile (1.00 g, 4.15 mmol) was added, followed by stirring at 80°C for two hours under a nitrogen atmosphere. The solvent was distilled off under reduced pressure, and the residue was purified by silica gel column chromatography (hexane : ethyl acetate, 50 : 50 - 100 : 0, V/V) and by basic silica gel column chromatography (hexane : ethyl acetate, 40 : 60 - 90 : 10, V/V). The residue thus obtained was treated by the same method as in Example 4 (4e) to give the desired title compound (490 mg, yield 72%).
¹H-NMR (DMSO-D₆) δ: 1.37-1.49 (2H, m), 1.84-1.93 (2H, m), 3.31-3.39 (2H, m), 3.54-3.64 (1H, m), 3.78-3.85 (4H, m), 4.18-4.22 (2H, m), 5.20 (1H, br s), 7.20 (2H, d, J = 8.7 Hz), 7.64 (2H, d, J = 8.7 Hz), 7.70-7.75 (1H, m), 7.94-7.98 (1H, m), 8.06-8.08 (1H, m), 8.85-8.88 (1H, m).

### <Example 25>

### 6-(Difluoromethoxy)-1-(4-[2-(tetrahydro-2H-pyran-4-yloxy)ethoxy]phenyl)-1H-benzimidazole hydrochloride

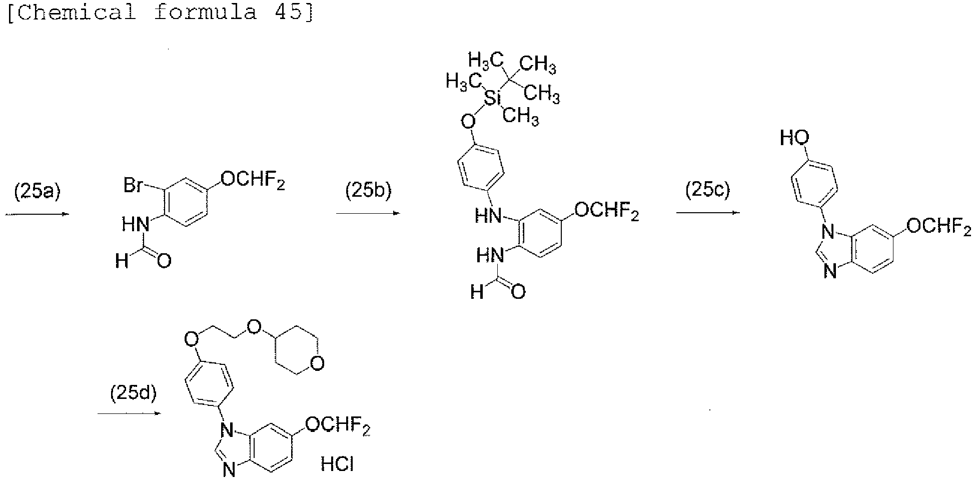

### (25a) N-[2-Bromo-4-(difluoromethoxy)phenyl]formamide

Into acetonitrile (80 mL), 2-bromo-4-(difluoromethoxy)aniline (4.40 g, 18.5 mmol) and formic acid (1.1 mL, 27.5 mmol) were dissolved, to which 4-(4,6-dimethoxy-1,3,5-triazin-2-yl)-4-methylmorpholinium chloride (7.7 g, 28 mmol) and 4-methylmorpholine (3 mL, 27 mmol) were added, followed by stirring at room temperature for six hours. Ethyl acetate and water were added for extraction, and the resulting organic layer was sequentially washed with water and saturated brine and then dried over anhydrous sodium sulfate. After filtration, the solvent was distilled off under reduced pressure and the residue thus obtained was purified by silica gel column chromatography (hexane : ethyl acetate, 100 : 0 - 60 : 40, V/V) to give the desired title compound (4.1 g, yield 83%).
¹H-NMR (CDCl₃) 5: 6.47 (1H, t, J = 73.0 Hz), 7.13 (1H, dd, J = 9.1, 2.6 Hz), 7.39 (1H, d, J = 2.6 Hz), 7.58 (1H, br s), 8.41 (1H, d, J = 9.1 Hz), 8.49 (1H, s).

### (25b) N-{2-[(4-{[Tert-butyl(dimethyl)silyl]oxy}phenyl)amino]-4-(difluoromethoxy)phenyl}formamide

Using N-[2-bromo-4-(difluoromethoxy)phenyl]formamide (4.1 g, 15.4 mmol) produced in Example 25 (25a) and 4-{[tert-butyl(dimethyl)silyl]oxy}aniline (3.8 g, 17 mmol), the desired title compound (2.6 g, yield 41%) was obtained by the same method as in Example 22 (22a). ¹H-NMR (CDCl₃) δ: 0.15 (6H, s), 0.97 (9H, s), 3.35-3.60 (2H, br m), 6.16-6.84 (9H, m).

### (25c) 4-[6-(Difluoromethoxy)-1-benzimidazol-1H-yl]phenol

Using N-{2-[(4-{[tert-butyl(dimethyl)silyl]oxy}phenyl)amino]-4-(difluoromethoxy)phenyl}formamide (2.60 g, 6.36 mmol) produced in Example 25 (25b), the desired title compound (1.6 g, yield 83%) was obtained by the same method as in Example 4 (4c).
¹H-NMR (CDCl₃) δ: 2.13 (1H, br s), 6.51 (1H, t, J = 74.0 Hz), 7.02 (2H, d, J = 8.7 Hz), 7.12-7.16 (1H, m), 7.19-7.22 (1H, m), 7.30 (2H, d, J = 8.7 Hz), 7.81 (1H, d, J = 8.7 Hz), 8.06 (1H, s).

### (25d) 6-(Difluoromethoxy)-1-{4-[2-(tetrahydro-2H-pyran-4-yloxy)ethoxy]phenyl}-1H-benzimidazole hydrochloride

Using 4-[6-(difluoromethoxy)-1-benzimidazol-1H-yl]phenol produced in Example 25 (25c) and 2-(tetrahydro-2H-pyran-4-yloxy)ethanol produced in Reference Example 3, the desired title compound was obtained by the same method as in Example 24 (24f).
¹H-NMR (DMSO-D₆) δ: 1.37-1.48 (2H, m), 1.84-1.93 (2H, m), 3.30-3.39 (2H, m), 3.55-3.63 (1H, m), 3.78-3.85 (4H, m), 4.18-4.24 (2H, m), 7.23 (2H, d, J = 9.2 Hz), 7.27-7.31 (1H, m), 7.29 (1H, t, J = 74.0 Hz), 7.36-7.38 (1H, m), 7.64 (2H, d, J = 9.2 Hz), 7.89 (1H, d, J = 9.2 Hz), 9.09 (1H, s).

Examples 26 to 150 shown in Tables 1 to 26 below were produced by the same methods as in Examples 1 to 25.
In the Tables, "Ex. No." refers to the number of the Example, "Structure" refers to the structural formula of the compound of the Example, "Data" refers to the physicochemical data of the compound of the Example, "Salt" refers to the kind of salt when the compound of the Example is present in the form of a salt, and "Mthd." refers to the production method. Also, "cis" and "trans" in the depiction of "Structure" refer to the relative position of substituents, in the case that a cyclic group has two substituents.

**[Table 1]**

| Ex. No. | Structure | Data | Salt | Mthd. |
|---|---|---|---|---|
| 26 | 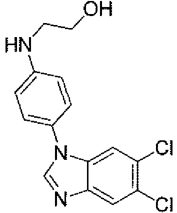 | ¹H-NMR (DMSO-D₆) δ: 3.16 (2H, q, *J* = 5.7 Hz), 3.59 (2H, q, *J =* 5.7 Hz), 4.75 (1H, t, *J* = 5.7 Hz), 6.02 (1H, t, *J* = 5.7 Hz), 6.77 (2H, d, *J* = 8.7 Hz), 7.33 (2H, d, *J* = 8.7 Hz), 7.65 (1H, s), 8.05 (1H, s), 8.51 (1H, s). | - | C |
| 7 | 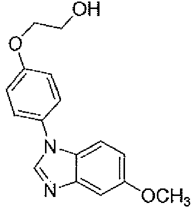 | ¹H-NMR (DMSO-D₆) δ: 3.73-3.80 (2H, m), 3.81 (3H, s), 4.07 (2H, t, *J* = 5.0 Hz), 4.94 (1H, t, *J =* 5.0 Hz), 6.94 (1H, dd, *J* = 9.2, 2.3 Hz), 7.16 (2H, d, *J* = 8.7 Hz), 7.29 (1H, d, *J* = 2.3 Hz), 7.41 (1H, d, *J* = 9.2 Hz), 7.55 (2H, d, *J* = 8.7 Hz), 8.40 (1H, s). | - | C |
| 28 | 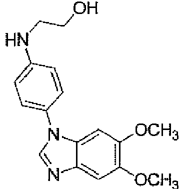 | ¹H-NMR (DMSO-D₆) δ: 3.16 (2H, q, *J=* 5.7 Hz), 3.59 (2H, q, *J* = 5.7 Hz), 3.76 (3H, s), 3.81 (3H, s), 4.74 (1H, t, *J* = 5.7 Hz), 5.92 (1H, t, *J* = 5.7 Hz), 6.77 (2H, d, *J* = 8.8 Hz), 6.91 (1H, s), 7.27-7.31 (3H, m), 8.13 (1H, s). | - | C |
| 29 | 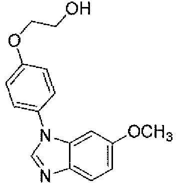 | ¹H-NMR (CDCl₃) δ: 3.83 (3H, s), 4.04 (2H, t, J = 4.4 Hz), 4.18 (2H, t, J = 4.4 Hz), 6.89 (1H, s), 6.99 (1H, d, J = 8.7 Hz), 7.12 (2H, d, J = 8.7 Hz), 7.42 (2H, d, J = 8.7 Hz), 7.76 (1H, d, J = 8.7 Hz), 8.02 (1H, s). | - | C |
| 30 | 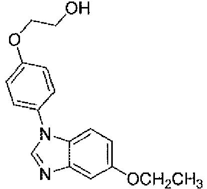 | ¹H-NMR (CDCl₃) δ: 1.26 (3H, t, *J* = 7.1 Hz), 4.04-4.07 (2H, m), 4.13 (2H, q, *J =* 7.1 Hz), 4.20 (2H, t, *J =* 4.4 Hz), 7.13-7.17 (3H, m), 7.39 (1H, d, *J* = 9.2 Hz), 7.44-7.46 (3H, m), 8.63 (1H, s). | - | C |

**[Table 2]**

| | | | | |
|---|---|---|---|---|
| 31 | 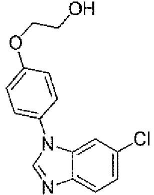 | ¹H-NMR (CDCl₃) δ: 4.02-4.06 (2H, m), 4.18 (2H, t, *J* = 4.5 Hz), 7.11 (2H, d, *J* = 8.5 Hz), 7.30 (1H, d, *J =* 8.5 Hz), 7.39-7.44 (3H, m), 7.77 (1H, d, *J* = 8.8 Hz), 8.05 (1H, s). | - | C |
| 2 | 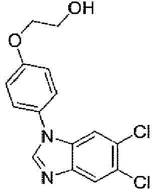 | ¹H-NMR (CDCl₃) 5: 4.03-4.06 (2H, m), 4.18 (2H, t, *J* = 4.4 Hz), 7.12 (2H, d, *J* = 8.7 Hz), 7.38 (2H, d, *J* = 8.7 Hz), 7.54 (1H, s), 7.96 (1H, s), 8.05 (1H, s). | - | C |
| 33 | 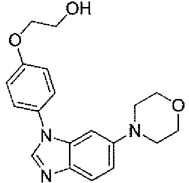 | ¹H-NMR (CDCl₃) δ: 3.13-3.16 (4H, m), 3.86-3.89 (4H, m), 4.02-4.05 (2H, m), 4.18 (2H, t, *J* = 4.5 Hz), 6.86 (1H, s), 7.03 (1H, d, *J* = 8.8 Hz), 7.11 (2H, d, *J* = 8.8 Hz), 7.41 (2H, d, *J* = 8.8 Hz), 7.75 (1H, d, *J* = 8.8 Hz), 7.93 (1H, s). | - | C |
| 34 | 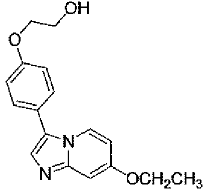 | ¹H-NMR (CDCl₃) δ: 1.47 (3H, t, *J* = 7.0 Hz), 4.01 (2H, t, *J* = 4.5 Hz), 4.09 (2H, q, *J* = 7.0 Hz), 4.15 (2H, t, *J* = 4.5 Hz), 6.51 (1H, dd, *J* = 7.3, 2.2 Hz), 6.88 (1H, d, *J* = 2.2 Hz), 7.05 (2H, d, *J* = 8.5 Hz), 7.44 (2H, d, *J* = 8.5 Hz), 7.46 (1H, s), 8.06 (1H, d, *J* = 7.3 Hz). | - | H |
| 35 | 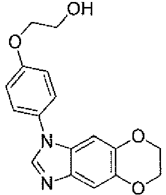 | ¹H-NMR (CDCl₃) δ: 4.03 (2H, t, *J* = 4.4 Hz), 4.16 (2H, t, *J* = 4.4 Hz), 4.29 (4H, s), 6.93 (1H, s), 7.07 (2H, d, *J* = 8.7 Hz), 7.33 (1H, s), 7.38 (2H, d, *J* = 8.7 Hz), 7.91 (1H, s). | - | H |
| 36 | 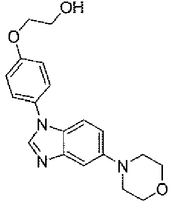 | ¹H-NMR (CDCl₃) δ: 2.02-2.06 (1H, m), 3.19 (4H, t, *J* = 4.6 Hz), 3.92 (4H, t, *J* = 4.6 Hz), 4.01-4.05 (2H, m), 4.17 (2H, t, *J* = 4.4 Hz), 7.05 (1H, d, *J* = 8.9 Hz), 7.09 (2H, d, *J* = 8.9 Hz), 7.35-7.38 (2H, m), 7.41 (2H, d, *J* = 8.9 Hz), 8.00 (1H, s). | - | C |

**[Table 3]**

| | | | | |
|---|---|---|---|---|
| 37 | 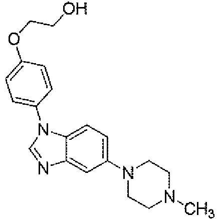 | ¹H-NMR (DMSO-D₆) δ: 2.50 (3H, s), 2.83-2.85 (2H, m), 3.14-3.22 (2H, m), 3.52-3.55 (2H, m), 3.77 (2H, t, *J* = 4.9 Hz), 3.88-3.90 (2H, m), 4.11 (2H, t, *J* = 4.9 Hz), 7.23 (2H, d, *J* = 8.8 Hz), 7.33-7.37 (2H, m), 7.58 (1H, d, *J* = 9.0 Hz), 7.68 (2H, d, *J* = 8.8 Hz), 9.49 (1H, s). | 2HCl | C |
| 38 | 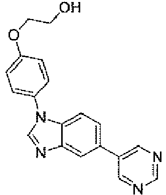 | ¹H-NMR (CDCl₃) δ: 4.02-4.07 (2H, m), 4.19 (2H, t, *J* = 4.6 Hz), 7.14 (2H, d, *J* = 8.7 Hz), 7.46 (2H, d, *J =* 8.7 Hz), 7.53 (1H, d, *J* = 8.7 Hz), 7.60 (1H, d, *J* = 8.7 Hz), 8.09 (1H, s), 8.14 (1H, s), 9.04 (2H, s), 9.22 (1H, s). | - | C |
| 39 | 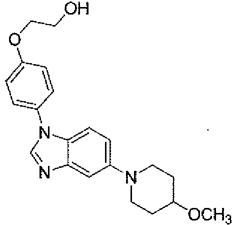 | ¹H-NMR (CDCl₃) δ: 1.75-1.84 (2H, m), 2.05-2.13 (2H, m), 2.91-2.97 (2H, m), 3.35-3.39 (1H, m), 3.40 (3H, s), 3.48-3.53 (2H, m), 4.00-4.06 (2H, m), 4.16 (2H, t, *J* = 4.8 Hz), 7.07-7.11 (3H, m), 7.34 (1H, d, *J =* 9.2 Hz), 7.39 (1H, s), 7.41 (2H, d, *J* = 8.7 Hz), 7.98 (1H, s). | - | C |
| 40 | 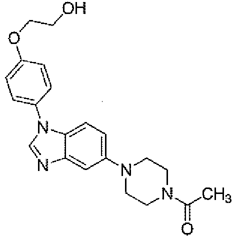 | ¹H-NMR (CDCl₃) δ: 2.16 (3H, s), 3.15-3.20 (4H, m), 3.68 (2H, t, *J* = 5.0 Hz), 3.83 (2H, t, *J* = 5.0 Hz), 4.01-4.06 (2H, m), 4.17 (2H, t, *J* = 4.8 Hz), 7.06 (1H, d, *J* = 9.2 Hz), 7.10 (2H, d, *J* = 8.7 Hz), 7.36-7.38 (2H, m), 7.41 (2H, d, *J* = 8.7 Hz), 8.01 (1H, s). | - | G |
| 41 | 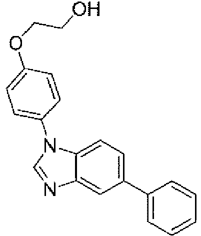 | ¹H-NMR (CDCl₃) δ: 4.02-4.06 (2H, m), 4.17-4.20 (2H, m), 7.12 (2H, d, *J* = 8.7 Hz), 7.33-7.38 (1H, m), 7.44-7.47 (3H, m), 7.50 (1H, d, *J* = 6.0 Hz), 7.53 (1H, s), 7.58 (1H, dd, *J* = 8.5, 1.6 Hz), 7.66-7.69 (2H, m), 8.08-8.09 (2H, m). | - | C |

**[Table 4]**

| | | | | |
|---|---|---|---|---|
| 42 | 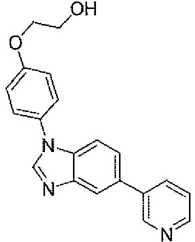 | ¹H-NMR (CDCl₃) δ: 2.10 (1H, t, *J* = 6.4 Hz), 4.03-4.07 (2H, m), 4.19 (2H, t, *J* = 4.4 Hz), 7.13 (2H, d, *J* = 8.7 Hz), 7.40 (1H, dd, *J* = 7.8, 5.0 Hz), 7.46 (2H, d, *J* = 8.7 Hz), 7.56 (2H, s), 7.96 (1H, d, *J* = 7.8 Hz), 8.08 (1H, s), 8.12 (1H, s), 8.60 (1H, d, *J* = 5.0 Hz), 8.93 (1H, s). | - | C |
| 43 | 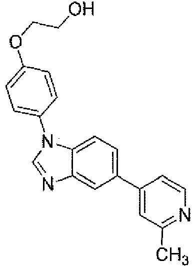 | ¹H-NMR (CDCl₃) δ: 2.12 (1H, t, *J* = 6.0 Hz), 2.65 (3H, s), 4.05 (2H, q, *J* = 4.7 Hz), 4.19 (2H, t, *J =* 4.7 Hz), 7.13 (2H, d, *J* = 8.7 Hz), 7.40 (1H, d, J = 5.5 Hz), 7.44 (1H, s), 7.46 (2H, s), 7.54 (1H, d, *J* = 8.3 Hz), 7.60 (1H, d, *J* = 8.7 Hz), 8.13 (2H, d, *J* = 8.3 Hz), 8.56 (1H, d, *J* = 5.5 Hz). | - | C |
| 44 | 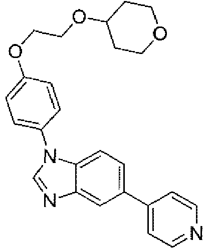 | ¹H-NMR (DMSO-D₆) δ: 1.40-1.47 (2H, m), 1.87-1.91 (2H, m), 3.33-3.38 (2H, m), 3.57-3.62 (2H, m), 3.80-3.84 (3H, m), 4.21 (2H, t, *J* = 4.6 Hz), 7.23 (2H, d, *J* = 9.2 Hz), 7.65 (2H, d, *J* = 9.2 Hz), 7.75 (1H, d, *J* = 8.6 Hz), 8.04 (1H, *d, J* = 8.6 Hz), 8.50 (2H, d, *J* = 6.3 Hz), 8.58 (1H, s), 8.81 (1H, s), 8.93 (2H, d, *J* = 6.3 Hz). | HCl | A |
| 45 | 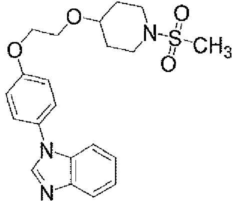 | ¹H-NMR (DMSO-D₆) δ: 1.56-1.63 (2H, m), 1.89-1.94 (2H, m), 2.97-3.02 (2H, m), 3.29-3.34 (2H, m), 3.57-3.61 (1H, m), 3.82 (2H, t, *J* = 4.6 Hz), 4.23 (2H, t, *J* = 4.6 Hz), 7.26 (2H, d, *J* = 8.6 Hz), 7.55-7.58 (2H, m), 7.66 (1H, d, *J* = 6.3 Hz), 7.70 (2H, d, *J* = 8.6 Hz), 7.92 (1H, d, *J* = 6.3 Hz), 9.51 (1H, s). | HCl | G |
| 46 | 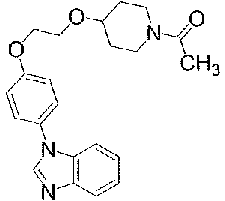 | ¹H-NMR (CDCl₃) δ: 1.62-1.72 (2H, m), 1.86-1.95 (2H, m), 2.11 (3H, s), 3.25-3.39 (2H, m), 3.65-3.72 (2H, m), 3.86-3.97 (3H, m), 4.21 (2H, t, *J =* 4.8 Hz), 7.10 (2H, d, *J* = 9.2 Hz), 7.31-7.34 (2H, m), 7.42 (2H, d, *J* = 9.2 Hz), 7.44-7.47 (1H, m), 7.86-7.89 (1H, m), 8.06 (1H, s). | - | G |

**[Table 5]**

| | | | | |
|---|---|---|---|---|
| 47 | 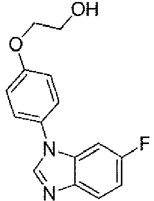 | ¹H-NMR (CDCl₃) δ: 2.91 (1H, t, *J* = 6.2 Hz), 4.02-4.06 (2H, m), 4.18 (2H, t, *J* = 4.6 Hz), 7.07 (1H, dd, *J* = 9.2, 2.3 Hz), 7.10-7.14 (3H, m), 7.40 (2H, d, *J* = 9.2 Hz), 7.79 (1H, dd, *J* = 8.9, 4.8 Hz), 8.03 (1H, s). | - | C |
| 48 | 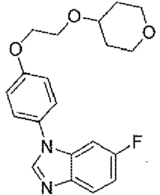 | ¹H-NMR (CDCl₃) δ: 1.62-1.71 (2H, m), 1.94-1.99 (2H, m), 3.44-3.50 (2H, m), 3.60-3.66 (1H, m), 3.89 (2H, t, *J* = 4.8 Hz), 3.95-4.00 (2H, m), 4.21 (2H, t, *J* = 4.8 Hz), 7.05-7.14 (4H, m), 7.38 (2H, d, *J* = 8.7 Hz), 7.79 (1H, dd, *J* = 8.7, 4.8 Hz), 8.03 (1H, s). | - | A |
| 49 | 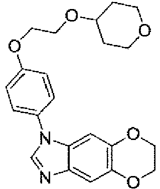 | ¹H-NMR (CDCl₃) δ: 1.61-1.70 (2H, m), 1.93-1.99 (2H, m), 3.44-3.50 (2H, m), 3.59-3.66 (1H, m), 3.88 (2H, t, *J* = 4.8 Hz), 3.95-4.00 (2H, m), 4.20 (2H, t, *J* = 4.8 Hz), 4.29 (4H, s), 6.93 (1H, s), 7.07 (2H, d, *J* = 8.7 Hz), 7.33 (1H, s), 7.37 (2H, d, *J=* 8.7 Hz), 7.91 (1H, s). | - | A |
| 50 | 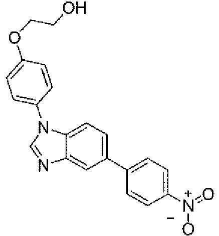 | ¹H-NMR (CDCl₃) δ: 2.02 (1H, t, *J* = 6.2 Hz), 4.03-4.07 (2H, m), 4.19 (2H, t, *J =* 4.6 Hz), 7.14 (2H, d, *J* = 8.6 Hz), 7.46 (2H, d, *J* = 8.6 Hz), 7.55-7.61 (2H, m), 7.82 (2H, d, *J* = 9.2 Hz), 8.13 (2H, s), 8.33 (2H, d, *J* = 9.2 Hz). | - | C |
| 51 | 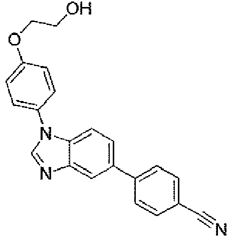 | ¹H-NMR (CDCl₃) δ: 2.00 (1H, t, *J* = 6.2 Hz), 4.03-4.07 (2H, m), 4.19 (2H, t, *J* = 5.0 Hz), 7.13 (2H, d, *J* = 8.7 Hz), 7.45 (2H, d, *J* = 8.7 Hz), 7.55 (2H, s), 7.76 (4H, s), 8.09 (1H, s), 8.12 (1H, s). | - | C |

**[Table 6]**

| | | | | |
|---|---|---|---|---|
| 52 | 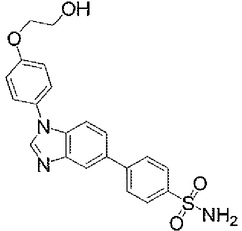 | ¹H-NMR (DMSO-D₆) δ: 3.75-3.78 (2H, m), 4.09 (2H, t, *J* = 5.2 Hz), 4.93 (1H, t, *J* = 5.7 Hz), 7.20 (2H, d, *J* = 8.6 Hz), 7.39 (2H, s), 7.60-7.64 (3H, m), 7.70 (1H, d, *J* = 8.3 Hz), 7.90 (2H, d, *J =* 8.6 Hz), 7.95 (2H, d, *J* = 8.6 Hz), 8.14 (1H, s), 8.56 (1H, s). | | C |
| 53 | 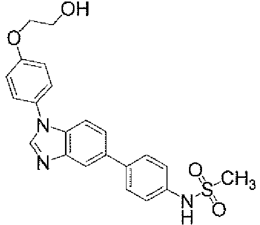 | ¹H-NMR (DMSO-D₆) δ: 3.03 (3H, s), 3.75-3.79 (2H, m), 4.09 (2H, t, *J* = 5.2 Hz), 4.92 (1H, t, *J* = 5.5 Hz), 7.99 (2H, d, *J* = 9.2 Hz), 7.31 (2H, d, *J* = 8.7 Hz), 7.56-7.62 (4H, m), 7.72 (2H, d, *J* = 8.7 Hz), 8.00 (1H, s), 8.50 (1H, s), 9.80 (1H, s). | - | C |
| 54 | 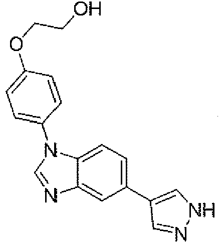 | 1H-NMR (CDCl₃) δ: 4.02-4.06 (2H, m), 4.17-4.19 (2H, m), 7.12 (2H, d, *J* = 6.4 Hz), 7.43-7.52 (5H, m), 7.91 (2H, s), 7.99 (1H, s), 8.05 (1H, s). | - | C |
| 56 | 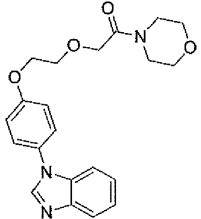 | ¹H-NMR (DMSO-D₆) δ: 3.42-3.44 (4H, m), 3.54-3.59 (4H, m), 3.83-3.85 (2H, m), 4.24-4.27 (4H, m), 7.25 (2H, d, *J* = 9.2 Hz), 7.56-7.59 (2H, m), 7.66-7.68 (1H, m), 7.71 (2H, d, *J* = 9.2 Hz), 7.92-7.94 (1H, m), 9.57 (1H, s). | HCl | A |
| 57 | 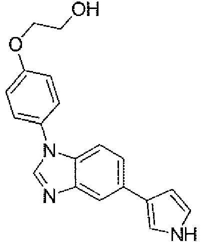 | ¹H-NMR (DMSO-D₆) δ: 3.76 (2H, t, *J* = 4.9 Hz), 4.08 (2H, t, *J* = 4.9 Hz), 6.50 (1H, q, *J =* 2.3 Hz), 6.80 (1H, q, *J* = 2.3 Hz), 7.17 (2H, d, *J* = 9.2 Hz), 7.23-7.25 (1H, m), 7.43 (1H, d, *J* = 8.6 Hz), 7.52 (1H, d, *J* = 8.6 Hz), 7.57 (2H, d, *J* = 9.2 Hz), 7.87 (1H, s), 8.39 (1H, s), 16.88 (1H, br s). | 0.4;HCl | c |

**[Table 7]**

| | | | | |
|---|---|---|---|---|
| 59 | 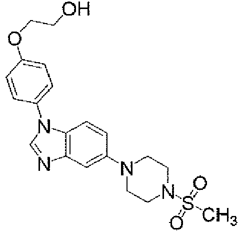 | ¹H-NMR (CDCl₃) δ: 2.85 (3H, s), 3.29-3.32 (4H, m), 3.43-3.46 (4H, m), 4.03 (2H, t, *J* = 4.4 Hz), 4.17 (2H, t, *J =* 4.4 Hz), 7.06 (1H, d, *J* = 8.7 Hz), 7.10 (2H, d, *J* = 8.3 Hz), 7.37 (1H, d, *J* = 8.7 Hz), 7.40-7.42 (3H, m), 8.04 (1H, s). | - | G |
| 60 | 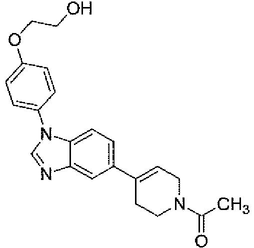 | ¹H-NMR (CDCl₃) δ: 2.16 (1.5H, s), 2.20 (1.5H, s), 2.65 (1H, br s), 2.70 (1H, br s), 3.72 (1H, t, *J* = 5.7 Hz), 3.87 (1H, t, *J* = 5.7 Hz), 4.02-4.05 (2H, m), 4.17-4.19 (3H, m), 4.27-4.28 (1H, m), 6.06 (0.5H, br s), 6.12 (0.5H, br s), 7.11 (2H, d, *J* = 8.7 Hz), 7.38-7.44 (4H, m), 7.83 (0.5H, s), 7.86 (0.5H, s), 8.05 (1H, s). | - | G |
| 61 | 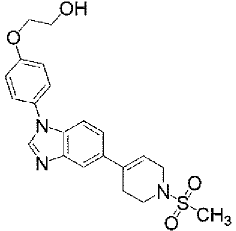 | ¹H-NMR (CDCl₃) δ: 2.77 (2H, br s), 2.88 (3H, s), 3.58 (2H, t, *J* = 5.3 Hz), 4.01-4.06 (4H, m), 4.17-4.19 (3H, m), 6.11 (1H, br s), 7.11 (2H, d, *J* = 7.3 Hz), 7.36-7.44 (4H, m), 7.85 (1H, s), 8.06 (1H, s). | - | G |
| 62 | 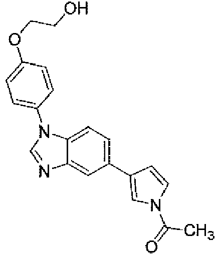 | ¹H-NMR (CDCl₃) δ: 2.05 (1H, t, *J* = 6.2 Hz), 2.63 (3H, s), 4.02-4.06 (2H, m), 4.18 (2H, t, *J* = 4.4 Hz), 6.71-6.72 (1H, m), 7.12 (2H, d, *J* = 9.2 Hz), 7.37 (1H, br s), 7.43-7.46 (3H, m), 7.53 (1H, d, *J* = 8.7 Hz), 7.62 (1H, br s), 8.02 (1H, s), 8.05 (1H, s). | - | G |
| 63 | 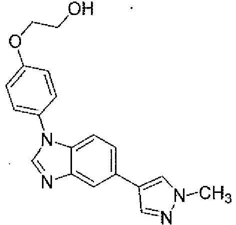 | ¹H-NMR (CDCl₃) δ: 2.11 (1H, t, *J* = 6.2 Hz), 3.97 (3H, s), 4.02-4.06 (2H, m), 4.18 (2H, t, *J* = 4.4 Hz), 7.11 (2H, d, *J* = 8.7 Hz), 7.42-7.44 (4H, m), 7.66 (1H, s), 7.81 (1H, s), 7.94 (1H, s), 8.04 (1H, s). | - | c |

**[Table 8]**

| | | | | |
|---|---|---|---|---|
| 64 | 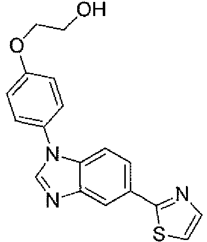 | ¹H-NMR (CDCl₃) δ: 2.06 (1H, t, *J* = 6.3 Hz), 4.03-4.06 (2H, m), 4.19 (2H, t, *J* = 4.6 Hz), 7.13 (2H, d, *J* = 8.6 Hz), 7.33 (1H, d, *J =* 3.4 Hz), 7.45 (2H, d, *J* = 8.6 Hz), 7.50 (1H, d, *J* = 8.3 Hz), 7.87 (1H, d, *J* = 3.4 Hz), 8.03 (1H, d, *J* = 8.3 Hz), 8.11 (1H, s), 8.43 (1H, s). | - | C |
| 65 | 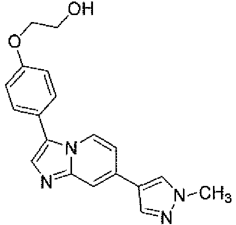 | ¹H-NMR (CDCl₃) δ: 3.98 (3H, s), 4.03 (2H, t, *J* = 4.4 Hz), 4.17 (2H, t, *J* = 4.4 Hz), 6.93 (1H, d, *J* = 7.3 Hz), 7.08 (2H, d, *J* = 8.7 Hz), 7.49 (2H, d, *J* = 8.7 Hz), 7.61 (1H, s), 7.70-7.71 (2H, m), 7.83 (1H, s), 8.22 (1H, d, *J* = 6.4 Hz). | - | C |
| 6 | 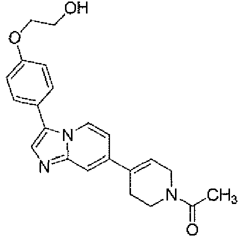 | ¹H-NMR (CDCl₃) δ: 2.05 (1H, br s), 2.17 (1.5H, s), 2.20 (1.5H, s), 2.60 (1H, br s), 2.66 (1H, br s), 3.70-3.74 (1H, m), 3.85-3.88 (1H, m), 4.03 (2H, br s), 4.16-4.20 (3H, m), 4.31 (1H, br s), 6.20 (0.5H, br s), 6.28 (0.5H, br s), 6.90-6.95 (1H, m), 7.08 (2H, d, *J* = 8.7 Hz), 7.48 (2H, d, *J =* 8.7 Hz), 7.57 (1H, br s), 7.63 (1H, brs), 8.18-8.21 (1H, m). | - | G |
| 67 | 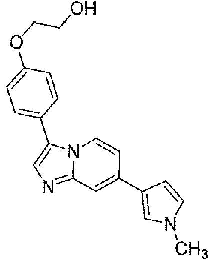 | ¹H-NMR (CDCl₃) δ: 3.72 (3H, s), 4.02 (2H, t, *J* = 4.3 Hz), 4.17 (2H, t, *J* = 4.3 Hz), 6.50-6.51 (1H, m), 6.67-6.68 (1H, m), 6.97 (1H, d, *J* = 7.4 Hz), 7.00-7.01 (1H, m), 7.07 (2H, d, *J* = 8.6 Hz), 7.48 (2H, d, *J* = 8.6 Hz), 7.56 (1H, s), 7.69 (1H, s), 8.17 (1H, d, *J =* 6.3 Hz). | - | C |

**[Table 9]**

| | | | | |
|---|---|---|---|---|
| 68 | 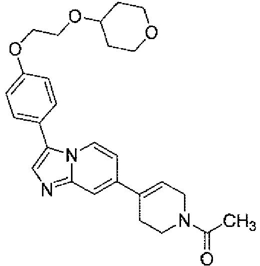 | ¹H-NMR (CDCl₃) δ: 1.61-1.70 (2H, m), 1.93-1.99 (2H, m), 2.16 (1.5H, s), 2.20 (1.5H, s), 2.60 (1H, br s), 2.65 (1H, br s), 3.44-3.50 (2H, m), 3.60-3.66 (1H, m), 3.72 (1H, t, J = 5.7 Hz), 3.85-3.90 (3H, m), 3.95-4.00 (2H, m), 4.19-4.21 (3H, m), 4.29-4.32 (1H, m), 6.19 (0.5H, br s), 6.28 (0.5H, br s), 6.90-6.95 (1H, m), 7.07 (2H, d, *J* = 8.7 Hz), 7.47 (2H, d, *J* = 8.7 Hz), 7.54 (0.5H, s), 7.59 (0.5H, s), 7.62 (1H, d, *J* = 3.7 Hz), 8.17-8.20 (1H, m). | - | G |
| 69 | 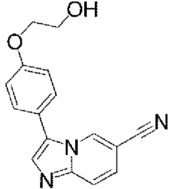 | ¹H-NMR (DMSO-D₆) δ: 3.75 (2H, dt, J = 5.3, 4.8 Hz), 4.06 (2H, t, J = 4.8 Hz), 4.92 (1H, t, J = 5.3 Hz), 7.12 (2H, d, J = 8.7 Hz), 7.50 (1H, dd, J = 9.2, 1.1 Hz), 7.63 (2H, d, J = 8.7 Hz), 7.78 (1H, d, J = 9.2 Hz), 7.85 (1H, s), 9.13-9.15 (1H, d, J = 1.1 Hz). | - | C |
| 70 | 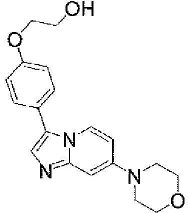 | ¹H-NMR (DMSO-D₆) δ: 3.17-3.22 (4H, m), 3.71-3.78 (6H, m), 4.03 (2H, t, J = 5.0 Hz), 4.90 (1H, t, J = 5.0 Hz), 6.74 (1H, d, J = 2.3 Hz), 6.86 (1H, dd, J = 7.8, 2.3 Hz), 7.07 (2H, d, J = 8.7 Hz), 7.42 (1H, s), 7.49 (2H, d, J = 8.7 Hz), 8.24 (1H, d, J = 7.8 Hz). | - | C |
| 71 | 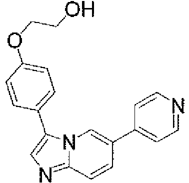 | ¹H-NMR (DMSO-D₆) δ: 3.75 (2H, dt, J = 5.5, 5.0 Hz), 4.07 (2H, t, J = 5.0 Hz), 4.91 (1H, t, J = 5.5 Hz), 7.13 (2H, d, J = 8.7 Hz), 7.71-7.64 (3H, m), 7.80-7.73 (4H, m), 8.63 (2H, d, J = 6.0 Hz), 8.72 (1H, s). | - | C |
| 72 | 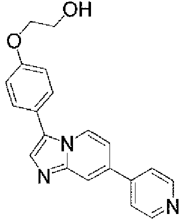 | ¹H-NMR (DMSO-D₆) δ: 3.76 (2H, t, J =4.8 Hz), 4.10 (2H, t, J = 4.8 Hz), 7.19-7.24 (2H, m), 7.66-7.72 (2H, m), 7.89 (1H, dd, J = 7.3, 1.8 Hz), 8.26 (2H, d, J = 6.4 Hz), 8.39 (1H, s), 8.52-8.54 (1H, m), 8.84 (1H, d, J = 7.3 Hz), 8.92-8.95 (2H, m). | 2HCl | C |

**[Table 10]**

| | | | | |
|---|---|---|---|---|
| 73 | 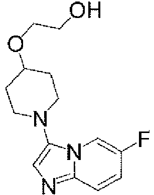 | ¹H-NMR (DMSO-D₆) δ: 1.68-1.79 (2H, m), 1.94-2.04 (2H, m), 2.84-2.95 (2H, m), 3.14-3.23 (2H, m), 3.59-3.53 (1H, m), 3.53-3.45 (4H, m), 7.95-8.01 (3H, m), 8.80-8.84 (1H, m). | HCl | C |
| 74 | 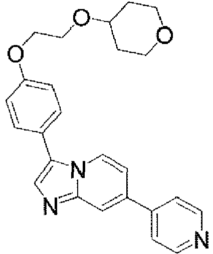 | ¹H-NMR (DMSO-D₆) δ: 1.37-1.46 (2H, m), 1.84-1.91 (2H, m), 3.24-3.42 (2H, m), 3.54-3.61 (1H, m), 3.78-3.83 (4H, m), 4.15-4.19 (2H, m), 7.14 (2H, d, J = 8.6 Hz), 7.42 (1H, dd, J = 7.1, 1.7 Hz), 7.61 (2H, d, J = 8.6 Hz), 7.80 (1H, s), 7.86-7.89 (2H, m), 8.20-8.22 (1H, m), 8.56 (1H, d, J = 7.1 Hz), 8.64-8.68 (2H, m). | - | B |
| 75 | 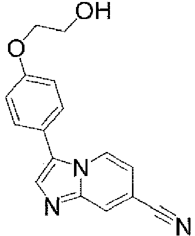 | ¹H-NMR (DMSO-D₆) δ: 3.74 (2H, q, J = 5.0 Hz), 4.05-4.08 (2H, m), 4.92 (1H, t, J = 5.0 Hz), 7.11-7.16 (2H, m), 7.20 (1H, dd, J = 7.3, 1.8 Hz), 7.59-7.64 (2H, m), 7.98 (1H, s), 8.40-8.42 (1H, m), 8.59 (1H, dd, J = 7.3, 0.9 Hz). | - | C |
| 76 | 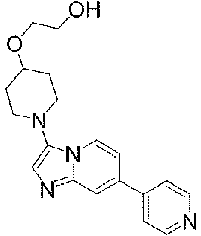 | ¹H-NMR (DMSO-D₆) δ: 1.66-1.75 (2H, m), 1.97-2.04 (2H, m), 2.82-2.87 (2H, m), 3.15-3.22 (2H, m), 3.47-3.56 (5H, m), 4.60 (1H, t, J = 4.6 Hz), 7.36-7.39 (2H, m), 7.82-7.85 (2H, m), 8.05-8.06 (1H, m), 8.22 (1H, d, J = 8.0 Hz), 8.63-8.66 (2H, m). | | C |

**[Table 11]**

| | | | | |
|---|---|---|---|---|
| 77 | 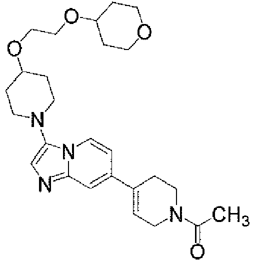 | ¹H-NMR (DMSO-D₆) δ: 1.33-1.42 (2H, m), 1.67-1.76 (2H, m), 1.80-1.87 (2H, m), 1.97-2.03 (2H, m), 2.04-2.10 (3H, m), 2.50-2.54 (1H. m), 2.59-2.64 (1H, m), 2.82-2.93 (2H, m), 3.15-3.23 (2H, m), 3.28-3.34 (4H, m), 3.54-3.60 (4H, m), 3.64-3.71 (2H, m), 3.76-3.82 (2H, m), 4.17-4.27 (2H, m), 6.74-6.69 (1H, m), 7.71-7.61 (2H, m), 7.86 (1H, s), 8.51 (1H, d, J = 6.9 Hz). | HCl | G |
| 78 | 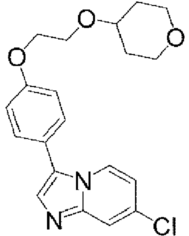 | ¹H-NMR (DMSO-D₆) δ: 1.36-1.47 (2H, m), 1.82-1.91 (2H, m), 3.32-3.37 (2H, m), 3.53-3.61 (1H, m), 3.76-3.84 (4H, m), 4.14-4.18 (2H, m), 6.98 (1H, dd, J = 7.3, 2.1 Hz), 7.10-7.15 (2H, m), 7.53-7.57 (2H, m), 7.70 (1H, s), 7.79-7.80 (1H, m), 8.45 (1H, dd, J = 7.3, 0.9 Hz). | - | D |
| 79 | 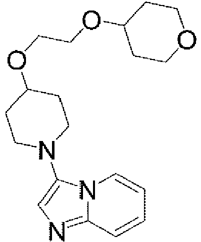 | ¹H-NMR (DMSO-D₆) δ: 1.34-1.45 (2H, m), 1.68-1.78 (2H, m), 1.81-1.89 (2H, m), 1.97-2.06 (2H, m), 2.87-2.95 (2H, m), 3.17-3.25 (2H, m), 3.30-3.37 (2H, m), 3.44 (1H, br s), 3.48-3.55 (1H, m), 3.55-3.61 (5H, m), 3.77-3.84 (2H, m), 7.46-7.51 (1H, m), 7.88-7.96 (3H, m), 8.81 (1H, d, J = 6.9 Hz). | HCl | E |
| 80 | 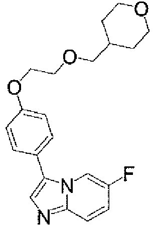 | ¹H-NMR (DMSO-D₆) δ: 1.14-1.27 (2H, m), 1.54-1.61 (2H, m), 1.75-1.86 (1H, m), 3.24-3.36 (4H, m), 3.74-3.78 (2H, m), 3.80-3.87 (2H, m), 4.18-4.23 (2H, m), 7.19 (2H, d, J = 8.7 Hz), 7.65 (2H, d, J = 8.7 Hz), 7.95-8.02 (1H, m), 8.04-8.10 (1H, m), 8.32 (1H, s), 8.86-8.90 (1H, m). | HCl | D |
| 81 | 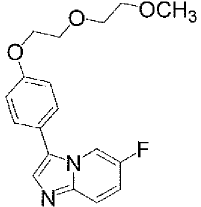 | ¹H-NMR (DMSO-D₆) δ: 3.26 (3H, s), 3.46-3.50 (2H, m), 3.59-3.63 (2H, m), 3.77-3.81 (2H, m), 4.18-4.22 (2H, m), 7.19 (2H, d, J = 8.8 Hz), 7.65 (2H, d, J = 8.8 Hz), 7.92-8.00 (1H, m), 8.03-8.08 (1H, m), 8.29 (1 H, s), 8.86-8.89 (1H, m). | HCl | D |

**[Table 12]**

| | | | | |
|---|---|---|---|---|
| 82 | 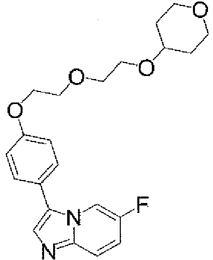 | ¹H-NMR (DMSO-D₆) δ: 1.32-1.43 (2H, m), 1.80-1.88 (2H, m), 3.27-3.35 (2H, m), 3.45-3.53 (1H, m), 3.56-3.64 (4H, m), 3.76-3.83 (4H, m), 4.19-4.23 (2H, m), 7.19 (2H, d, J = 8.7 Hz), 7.65 (2H, d, J = 8.7 Hz), 7.91-8.00 (1H, m), 8.01-8.09 (1H, m), 8.26-8.30 (1H, m), 8.84-8.88 (1H, m). | HCl | D |
| 83 | 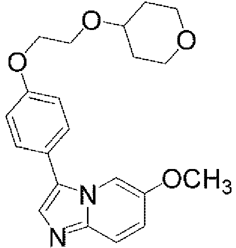 | ¹H-NMR (CDCl3) δ: 1.61-1.71 (2H, m), 1.91-1.99 (2H, m), 3.43-3.51 (2H, m), 3.59-3.66 (1H, m), 3.77 (3H, s), 3.86-3.90 (2H, m), 3.94-4.01 (2H, m), 4.19-4.22 (2H, m), 6.96-7.01 (1H, m), 7.08 (2H, d, J = 8.7 Hz), 7.47 (2H, d, J = 8.7 Hz), 7.53-7.58 (2H, m), 7.74-7.76 (1H, m). | - | D |
| 84 | 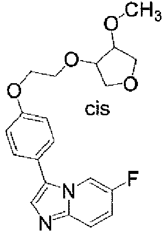 | ¹H-NMR (DMSO-D₆) δ: 3.34 (3H, s), 3.60-3.66 (2H, m), 3.79-3.96 (5H, m), 4.13-4.18 (1H, m), 4.20-4.24 (2H, m), 7.20 (2H, d, J = 8.7 Hz), 7.65 (2H, d, J = 8.7 Hz), 7.95-8.02 (1H, m), 8.04-8.1 (1H, m), 8.30-8.33 (1H, m), 8.87-8.90 (1H, m). | HCl | D |
| 85 | 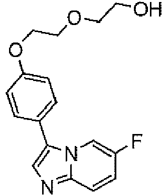 | ¹H-NMR (CDCl₃) δ: 2.13-2.18 (1H, br m), 3.69-3.73 (2H, m), 3.77-3.83 (2H, m), 3.91-3.95 (2H, m), 4.21-4.25 (2H, m), 7.06-7.14 (3H, m), 7.46 (2H, d, J = 8.7 Hz), 7.60-7.68 (2H, m), 8.15-8.19 (1H, m). | - | C |
| 86 | 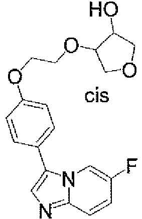 | ¹H-NMR (CDCl₃) δ: 2.99-3.02 (1H, m), 3.75-3.85 (2H, m), 3.92-4.06 (4H, m), 4.11-4.16 (1H, m), 4.18-4.29 (2H, m), 4.32-4.38 (1H, m), 7.06-7.15 (3H, m), 7.47 (2H, d, J = 8.7 Hz), 7.61-7.69 (2H, m), 8.15-8.19 (1H, m). | - | D |

**[Table 13]**

| | | | | |
|---|---|---|---|---|
| 87 | 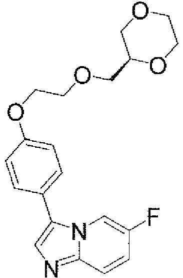 | ¹H-NMR (DMSO-D₆) δ: 3.24-3.32 (1H, m), 3.40-3.81 (10H, m), 4.17-4.23 (2H, m), 7.19 (2H, d, J = 8.7 Hz), 7.65 (2H, d, J = 8.7 Hz), 7.94-8.01 (1H, m), 8.03-8.10 (1H, m), 8.31 (1H, s), 8.86-8.89 (1H, m). | HCl | D |
| 88 | 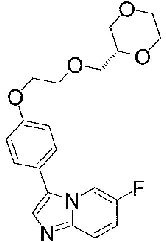 | ¹H-NMR (DMSO-D₆) δ: 3.24-3.31 (1H, m), 3.40-3.81 (10H, m), 4.17-4.23 (2H, m), 7.20 (2H, d, J = 8.7 Hz), 7.65 (2H, d, J = 8.7 Hz), 7.97-8.04 (1H, m), 8.05-8.11 (1H, m), 8.34 (1H, s), 8.87-8.91 (1H, m). | HCl | D |
| 89 | 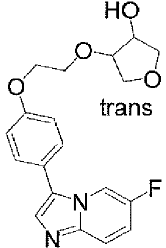 | ¹H-NMR (CDCl₃) δ: 1.96-2.00 (1H, m), 3.74-3.85 (2H, m), 3.91-3.95 (2H, m), 3.97-4.03 (1H, m), 4.04-4.07 (1H, m), 4.10-4.15 (1H, m), 4.18-4.22 (2H, m), 4.37-4.41 (1H, m), 7.05-7.15 (3H, m), 7.45 (2H, d, J = 8.7 Hz), 7.60-7.69 (2H, m), 8.15-8.18 (1H, m). | - | D |
| 90 | 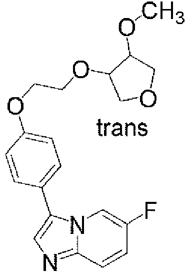 | ¹H-NMR (DMSO-D₆) δ: 3.30 (3H, s), 3.63-3.69 (2H, m), 3.77-3.91 (5H, m), 4.06-4.10 (1H, m), 4.19-4.24 (2H, m), 7.20 (2H, d, J = 8.7 Hz), 7.66 (2H, d, J = 8.7 Hz), 7.96-8.03 (1H, m), 8.05-8.11 (1H, m), 8.33 (1H, s), 8.86-8.91 (1H, m). | HCl | D |
| 91 | 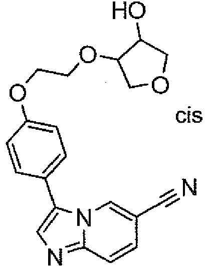 | ¹H-NMR (CDCl₃) δ: 2.96-2.99 (1H, m), 3.75-3.85 (2H, m), 3.92-4.07 (4H, m), 4.11-4.16 (1H, m), 4.20-4.31 (2H, m), 4.32-4.38 (1H, m), 7.12 (2H, d, J = 8.8 Hz), 7.26-7.30 (1H, m), 7.45 (2H, d, J = 8.8 Hz), 7.72-7.77 (2H, m), 8.64-8.66 (1H, m). | - | D |

**[Table 14]**

| | | | | |
|---|---|---|---|---|
| 92 | 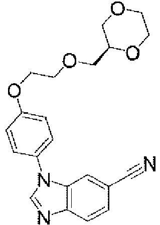 | ¹H-NMR (DMSO-D₆) δ: 3.25-3.32 (1H, m), 3.41-3.82 (10H, m), 4.18-4.22 (2H, m), 6.05 (1H, br s), 7.20 (2H, d, J = 9.2 Hz), 7.65 (2H, d, J = 9.2 Hz), 7.71-7.75 (1H, m), 7.97 (1H, d, J = 8.2 Hz), 8.07-8.09 (1H, m), 8.89 (1H, s). | HCl | A |
| 93 | 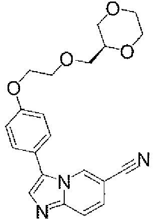 | ¹H-NMR (DMSO-D₆) δ: 3.24-3.32 (1H, m), 3.40-3.82 (10H, m), 4.17-4.22 (2H, m), 7.17 (2H, d, J = 9.2 Hz), 7.66 (2H, d, J = 9.2 Hz), 7.81-7.89 (1H, m), 7.95-8.00 (1H, m), 8.12-8.16 (1H, m), 9.32 (1H, s). | HCl | D |
| 94 | 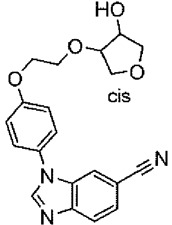 | ¹H-NMR (DMSO-D₆) δ: 3.50-3.60 (2H, m), 3.77-3.88 (3H, m), 3.95-4.03 (2H, m), 4.17-4.26 (3H, m), 4.73-4.75 (1H, m), 7.20 (2H, d, J = 9.0 Hz), 7.64 (2H, d, J = 9.0 Hz), 7.67-7.71 (1H, m), 7.95 (1H, d, J = 8.2 Hz), 8.04-8.06 (1H, m), 8.77 (1H, s). | - | A |
| 95 | 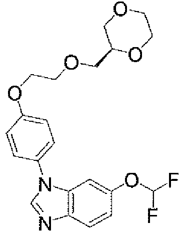 | ¹H-NMR (DMSO-D₆) δ: 3.24-3.31 (1H, m), 3.41-3.81 (10H, m), 4.18-4.22 (2H, m), 7.22 (2H, d, J = 8.7 Hz), 7.26-7.31 (1H, m), 7.28 (1H, t, J = 74.0 Hz), 7.35-7.38 (1H, m), 7.64 (2H, d, J = 8.7 Hz), 7.89 (1H, d, J = 8.7 Hz), 9.07 (1H, s). | HCl | A |
| 96 | 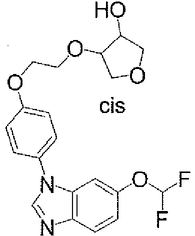 | ¹H-NMR (DMSO-D₆) δ: 3.49-3.60 (2H, m), 3.76-3.88 (3H, m), 3.95-4.04 (2H, m), 4.97-4.27 (3H, m), 7.24 (2H, d, J = 8.7 Hz), 7.27-7.34 (1H, m), 7.29 (1H, t, J = 74.2 Hz), 7.36-7.40 (1H, m), 7.65 (2H, d, J = 8.7 Hz), 7.87-7.93 (1H, m), 9.00-9.25 (1H, m). | HCl | A |

**[Table 15]**

| | | | | |
|---|---|---|---|---|
| 97 | 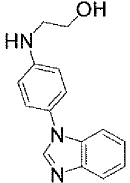 | ¹H-NMR (CDCl₃) δ: 2.19 (1H, br s), 3.38 (2H, t, J = 5.1 Hz), 3.92 (2H, t, J = 5.1 Hz), 4.31 (1H, br s), 6.77 (2H, d, J = 8.8 Hz), 7.27-7.34 (4H, m), 7.41-7.47 (1H, m), 7.82-7.88 (1H, m), 7.97 (1H, s). | - | C |
| 98 | 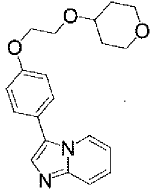 | ¹H-NMR (CDCl₃) δ: 1.61-1.71 (2H, m), 1.92-2.00 (2H, m), 3.43-3.51 (2H, m), 3.59-3.67 (1H, m), 3.88 (2H, t, J = 5.0 Hz), 3.94-4.01 (2H, m), 4.20 (2H, t, J = 5.0 Hz), 6.77-6.82 (1H, m), 7.05-7.10 (2H, m), 7.15-7.21 (1H, m), 7.44-7.49 (2H, m), 7.63 (1H, s), 7.66 (1H, d, J = 10.0 Hz), 8.26 (1H, d, J = 8.1 Hz). | - | D |
| 99 | 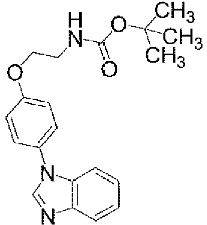 | ¹H-NMR (CDCl₃) δ: 1.47 (9H, s), 3.55-3.61 (2H, m), 4.10 (2H, t, J = 5.1 Hz), 5.04 (1H, br s), 6.76-6.82 (1H, m), 7.02-7.07 (2H, m), 7.15-7.21 (1H, m), 7.45-7.50 (2H, m), 7.63 (1H, s), 7.66 (1H, d, J = 8.8 Hz), 8.25 (1H, d, J = 7.1 Hz). | - | A |
| 100 | 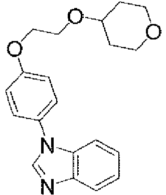 | ¹H-NMR (DMSO-D₆) δ: 1.36-1.47 (2H, m), 1.83-1.91 (2H, m), 3.30-3.38 (2H, m), 3.53-3.62 (2H, m), 3.77-3.84 (3H, m), 4.18-4.23 (2H, m), 7.24 (2H, d, J = 9.0 Hz), 7.52-7.61 (2H, m), 7.63-7.71 (3H, m), 7.89-7.93 (1H, m), 9.59 (1H, br s). | HCl | A |
| 101 | 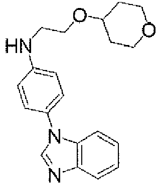 | ¹H-NMR (DMSO-D₆) δ: 1.36-1.46 (2H, m), 1.81-1.89 (2H, m), 3.25-3.37 (4H, m), 3.48-3.56 (1H, m), 3.59-3.64 (2H, m), 3.76-3.83 (2H, m), 6.84-6.89 (2H, m), 7.45-7.49 (2H, m), 7.58-7.70 (3H, m), 7.92-7.95 (1H, m), 9.79 (1H, s). | - | C |

**[Table 16]**

| | | | | |
|---|---|---|---|---|
| 102 | 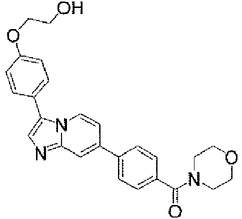 | ¹H-NMR (CDCl₃) δ: 2.17 (1H, br s), 3.40-3.95 (8H, m), 4.01-4.08 (2H, m), 4.18 (2H, t, J = 4.7 Hz), 7.07-7.14 (3H, m), 7.49-7.58 (4H, m), 7.68-7.75 (3H, m), 7.90 (1H, s), 8.31-8.34 (1H, m). | - | C |
| 03 | 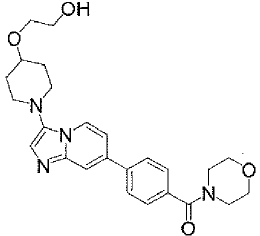 | ¹H-NMR (CDCl₃) δ: 1.59-1.73 (1H, m), 1.80-1.91 (2H, m), 2.05-2.15 (2H, m), 2.87-2.97 (2H, m), 3.23-3.31 (2H, m), 3.38-3.90 (13H, m), 7.06-7.10 (1H, m), 7.32 (1H, s), 7.52 (2H, d, J = 8.5 Hz), 7.69 (2H, d, J = 8.5 Hz), 7.76 (1H, br s), 7.99-8.02 (1H, m). | - | C |
| 104 | 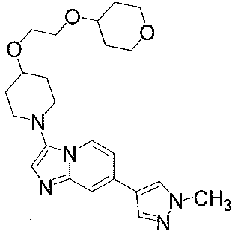 | ¹H-NMR (CDCl₃) δ: 1.57-1.67 (2H, m), 1.77-1.97 (4H, m), 2.02-2.11 (2H, m), 2.81-2.92 (2H, m), 3.18-3.28 (2H, m), 3.41-3.49 (2H, m), 3.52-3.62 (2H, m), 3.63-3.70 (4H, m), 3.91-4.00 (5H, m), 6.90-6.94 (1H, m), 7.22 (1H, s), 7.55-7.58 (1H, m), 7.67 (1H, s), 7.80 (1H, d, J = 0.7 Hz), 7.88-7.92 (1H, m). | - | B |
| 105 | 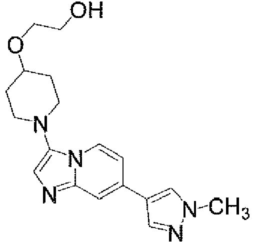 | ¹H-NMR (CDCl₃) δ: 1.78-2.13 (4H, m), 2.84-2.93 (2H, m), 3.20-3.28 (2H, m), 3.55-3.62 (1H, m), 3.64 (2H, t, J = 4.6 Hz), 3.78 (2H, t, J = 4.6 Hz), 3.97 (3H, s), 6.90-6.95 (1H, m), 7.22 (1H, s), 7.56-7.58 (1H, m), 7.68 (1H, s), 7.81 (1H, s), 7.90 (1H, d, J = 7.3 Hz). | - | C |
| 106 | 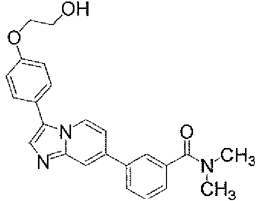 | ¹H-NMR (CDCl₃) δ: 2.18 (1H, br s), 3.05 (3H, br s), 3.16 (3H, br s), 4.00-4.06 (2H, m), 4.15-4.21 (2H, m), 7.06-7.13 (3H, m), 7.41-7.57 (4H, m), 7.65-7.76 (3H, m), 7.87 (1H, s), 8.31 (1H, d, J = 7.3 Hz). | - | C |

**[Table 17]**

| | | | | |
|---|---|---|---|---|
| 107 | 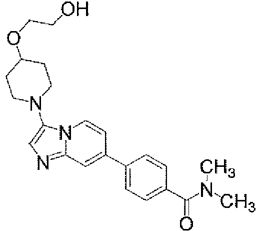 | ¹H-NMR (CDCl₃) δ: 1.80-1.91 (2H, m), 2.06-2.14 (2H, m), 2.88-2.96 (2H, m), 3.05 (3H, br s), 3.14 (3H, br s), 3.23-3.30 (2H, m), 3.56-3.63 (1H, m), 3.65 (2H, t, J = 4.6 Hz), 3.79 (2H, t, J = 4.6 Hz), 7.07-7.11 (1H, m), 7.31 (1H, s), 7.53 (2H, d, J = 8.5 Hz), 7.68 (2H, d, J = 8.5 Hz), 7.75-7.77 (1H, m), 7.98-8.02 (1H, m). | - | C |
| 108 | 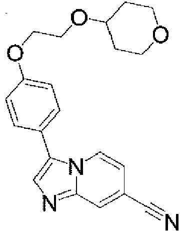 | ¹H-NMR (CDCl₃) δ: 1.60-1.71 (2H, m), 9.91-2.00 (2H, m), 3.42-3.51 (2H, m), 3.58-3.67 (1H, m), 3.88 (2H, t, J = 4.8 Hz), 3.93-4.01 (2H, m), 4.21 (2H, t, J = 4.8 Hz), 6.94 (1H, d, J = 6.9 Hz), 7.10 (2H, d, J = 8.2 Hz), 7.46 (2H, d, J = 8.2 Hz), 7.85 (1H, s), 8.05-8.08 (1H, m), 8.31 (1H, d, J = 6.9 Hz). | - | D |
| 109 | 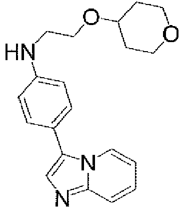 | ¹H-NMR (DMSO-D₆) δ: 1.36-1.47 (2H, m), 1.82-1.90 (2H, m), 3.27-3.37 (4H, m), 3.49-3.56 (1H, m), 3.62 (2H, t, J = 6.0 Hz), 3.78-3.86 (2H, m), 6.82-6.91 (2H, m), 7.41-7.50 (3H, m), 7.93-8.06 (2H, m), 8.25 (1H, s), 8.71 (1H, d, J = 6.9 Hz). | 2HCl | C |
| 110 | 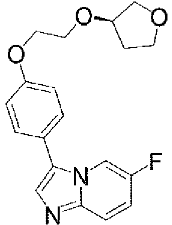 | ¹H-NMR (CDCl₃) δ: 2.02-2.09 (2H, m), 3.79-3.98 (6H, m), 4.19 (2H, t, J = 4.8 Hz), 4.24-4.29 (1H, m), 7.05-7.14 (3H, m), 7.45 (2H, d, J = 8.2 Hz), 7.63 (1H, dd, J = 9.6, 5.0 Hz), 7.67 (1H, s), 8.16-8.19 (1H, m). | - | D |
| 111 | 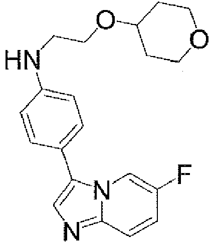 | ¹H-NMR (CDCl₃) δ: 1.57-1.68 (2H, m), 1.89-1.97 (2H, m), 3.34-3.40 (2H, m), 3.42-3.50 (2H, m), 3.51-3.59 (1H, m), 3.73 (2H, t, J = 5.0 Hz), 3.93-4.00 (2H, m), 4.31 (1H, br s), 6.77 (2H, d, J = 8.2 Hz), 7.04-7.11 (1H, m), 7.33 (2H, d, J = 8.2 Hz), 7.61 (1H, dd, J = 9.8, 5.3 Hz), 7.63 (1H, s), 8.15-8.20 (1H, m). | - | C |

**[Table 18]**

| | | | | |
|---|---|---|---|---|
| 112 | 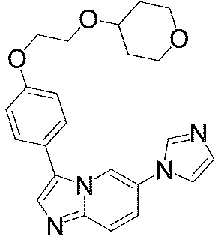 | ¹H-NMR (CDCl₃) δ: 1.60-1.70 (2H, m), 1.92-1.99 (2H, m), 3.42-3.50 (2H, m), 3.59-3.66 (1H, m), 3.88 (2H, t, J = 5.0 Hz), 3.93-4.00 (2H, m), 4.21 (2H, t, J = 5.0 Hz), 7.10 (2H, d, J = 8.7 Hz), 7.29-7.28 (3H, m), 7.46 (2H, d, J = 8.7 Hz), 7.74 (1H, s), 7.77 (1H, s), 7.79 (1H, s), 8.34 (1H, d, J = 1.4 Hz). | - | D |
| 113 | 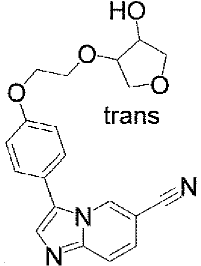 | ¹H-NMR (CDCl₃) δ: 2.02 (1H, d, J = 5.5 Hz), 3.75-3.79 (1H, m), 3.80-3.85 (1H, m), 3.92-3.96 (2H, m), 3.98-4.02 (1H, m), 4.04-4.08 (1H, m), 4.10-4.15 (1H, m), 4.21 (2H, t, J = 4.8 Hz), 4.37-4.41 (1H, m), 7.10 (2H, d, J = 8.7 Hz), 7.28 (1H, d, J = 9.4 Hz), 7.44 (2H, d, J = 8.7 Hz), 7.74 (1H, d, J = 9.4 Hz), 7.75 (1H, s), 8.65 (1H, s). | - | D |
| 114 | 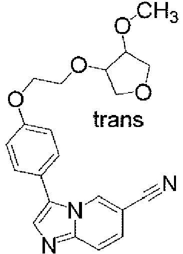 | ¹H-NMR (DMSO-D₆) δ: 3.30 (3H, s), 3.62-3.70 (2H, m), 3.77-3.91 (5H, m), 4.07-4.10 (1H, m), 4.22 (2H, t, J = 4.6 Hz), 7.19 (2H, d, J = 8.7 Hz), 7.67 (2H, d, J = 8.7 Hz), 7.93-8.08 (2H, br m), 8.21-8.29 (1H, br m), 9.38 (1H, br s). | - | D |
| 115 | 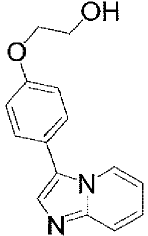 | ¹H-NMR (CDCl₃) δ: 2.40 (1H, br s), 4.03 (2H, t, J = 4.6 Hz), 4.17 (2H, t, J = 4.6 Hz), 6.84 (1H, t, J = 5.5 Hz), 7.06-7.10 (2H, m), 7.21-7.25 (1H, m), 7.46-7.49 (2H, m), 7.70-7.73 (1H, m), 7.65 (1H, s), 8.27 (1H, d, J = 6.9 Hz). | - | H |
| 116 | 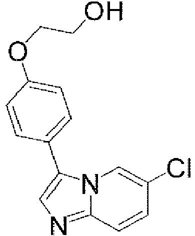 | ¹H-NMR (DMSO-D₆) δ: 3.77 (2H, t, J = 4.8 Hz), 4.10 (2H, t, J = 4.8 Hz), 7.17-7.22 (2H, m), 7.64-7.67 (2H, m), 7.97 (1H, dd, J = 9.6, 1.8 Hz), 8.06 (1H, d, J = 9.6 Hz), 8.33 (1H, s), 8.80 (1H, d, J = 1.8 Hz). | HCl | H |

**[Table 19]**

| | | | | |
|---|---|---|---|---|
| 117 | 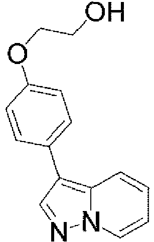 | ¹H-NMR (DMSO-D₆) δ: 3.74 (2H, t, J = 5.2 Hz), 4.03 (2H, t, J = 5.2 Hz), 6.92 (1H, dt, J = 6.9, 1.2 Hz), 7.02-7.05 (2H, m), 7.28 (1H, ddd, J = 9.2, 6.9, 1.2 Hz), 7.57-7.61 (2H, m), 7.90 (1H, td, J = 9.2, 1.2 Hz), 8.28 (1H, s), 8.70 (1H, td, J = 6.9, 1.2 Hz). | HCl | H |
| *118* | 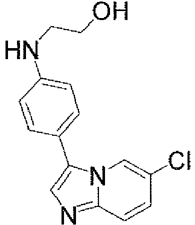 | ¹H-NMR (DMSO-D₆) δ: 3.22 (2H, t, *J* = 6.0 Hz), 3.62 (2H, t, *J* = 6.0 Hz), 6.98 (2H, d, *J* = 8.7 Hz), 7.50 (2H, d, *J* = 8.7 Hz), 8.03 (1H, dd, *J* = 9.6, 1.8 Hz), 8.09 (1H, dd, *J* = 9.6, 0.9 Hz), 8.33 (1H, s), 8.80 (1H, dd, *J* = 1.8, 0.9 Hz). | 2HCl | C |
| 119 | 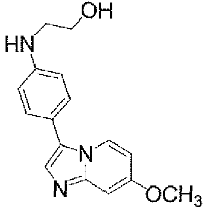 | ¹H-NMR (CDCl₃) δ: 1.87 (1H, br s), 3.36-3.40 (2H, m), 3.86 (3H, s), 3.90 (2H, t, *J* = 5.3 Hz), 4.26 (1H, br s), 6.49 (1H, dd, *J* = 7.3, 2.3 Hz), 6.75 (2H, d, *J* = 8.3 Hz), 6.89 (1H, d, *J* = 2.3 Hz), 7.30 (2H, d, *J* = 8.3 Hz), 7.37 (1H, s), 8.05 (1H, d, *J =* 7.3 Hz). | - | C |
| *20* | 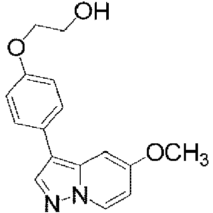 | ¹H-NMR (DMSO-D₆) δ: 3.74 (2H, t, *J* = 5.0 Hz), 3.88 (3H, s), 4.02 (2H, t, *J* = 5.0 Hz), 6.60 (1H, dd, *J* = 7.8, 2.8 Hz), 7.01-7.04 (2H, m), 7.08 (1H, d, *J* = 2.8 Hz), 7.55-7.59 (2H, m), 8.16 (1H, s), 8.56 (1H, d, *J*= 7.8 Hz). | HCl | D |
| 121 | 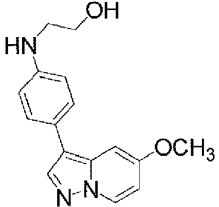 | ¹H-NMR (CDCl₃) δ: 3.46-3.50 (2H, m), 3.86 (3H, s), 4.00 (2H, t, *J =* 4.6 Hz), 6.48 (1H, dd, *J* = 7.3, 2.0 Hz), 6.90 (1H, br s), 7.22-7.28 (2H, m), 7.49 (2H, d, *J* = 8.3 Hz), 7.97 (1H, s), 8.28 (1H, d, *J* = 7.3 Hz). | - | C |

**[Table 20]**

| | | | | |
|---|---|---|---|---|
| 122 | 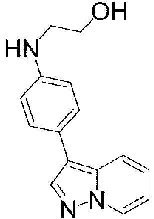 | ¹H-NMR (DMSO-D₆) δ: 3.13 (2H, t, *J* = 6.0 Hz), 3.58 (2H, t, *J* = 6.0 Hz), 4.70 (1H, br s), 5.59 (1H, br s), 6.70 (2H, d, *J* = 8.3 Hz), 6.87 (1H, t, *J* = 7.1 Hz), 7.22 (1H, dd, *J* = 8.8, 7.1 Hz), 7.39 (2H, d, *J* = 8.3 Hz), 7.84 (1H, d, *J* = 8.8 Hz), 8.17 (1H, s), 8.65 (1H, d, *J* = 7.1 Hz). | - | C |
| 123 | 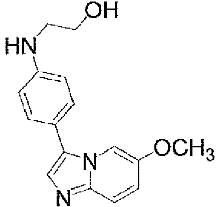 | ¹H-NMR (CDCl₃) δ: 2.35 (1H, br s), 3.37-3.41 (2H, m), 3.76 (3H, s), 3.91 (2H, t, *J* = 5.0 Hz), 4.29 (1H, br s), 6.76-6.80 (2H, m), 6.96 (1H, dd, *J* = 9.9, 2.5 Hz), 7.33-7.37 (2H, m), 7.49 (1H, s), 7.52 (1H, d, *J* = 9.9 Hz), 7.75 (1H, d, *J* = 2.5 Hz). | - | C |
| 124 | 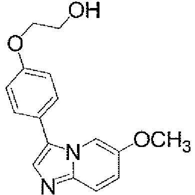 | ¹H-NMR (CDCl₃) δ: 2.32 (1H, br s), 3.77 (3H, s), 4.03 (2H, t, *J* = 4.4 Hz), 4.17 (2H, t, *J* = 4.4 Hz), 6.99 (1H, dd, *J* = 9.6, 2.3 Hz), 7.06-7.10 (2H, m), 7.46-7.50 (2H, m), 7.55 (1H, d, *J* = 9.6 Hz), 7.57 (1H, s), 7.75 (1H, d, *J* = 2.3 Hz). | - | C |
| 125 | 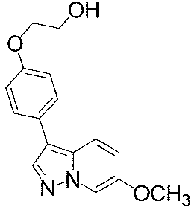 | ¹H-NMR (CDCl₃) δ: 2.07 (1H, t, *J* = 6.3 Hz), 3.86 (3H, s), 3.98-4.03 (2H, m), 4.14 (2H, t, *J* = 4.5 Hz), 6.97 (1H, dd, *J* = 9.8, 2.2 Hz), 6.99-7.03 (2H, m), 7.47-7.51 (2H, m), 7.64 (1H, d, *J* = 9.8 Hz), 7.98 (1H, s), 8.07 (1H, d, *J* = 2.2 Hz). | - | C |
| *126* | 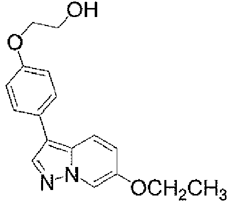 | ¹H-NMR (CDCl₃) δ: 1.47 (3H, t, *J* = 6.9 Hz), 2.07 (1H, br s), 4.00-4.07 (4H, m), 4.14 (2H, t, *J* = 4.6 Hz), 6.97 (1H, dd, *J* = 9.6, 2.3 Hz), 6.99-7.03 (2H, m), 7.47-7.50 (2H, m), 7.64 (1H, d, *J* = 9.6 Hz), 7.97 (1H, s), 8.06 (1H, d, *J* = 2.3 Hz). | - | C |

**[Table 21]**

| | | | | |
|---|---|---|---|---|
| 127 | 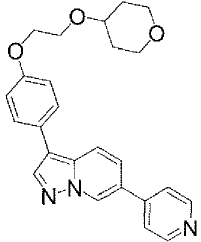 | ¹H-NMR (CDCl₃) δ: 1.63-1.70 (2H, m), 1.94-1.98 (2H, m), 3.44-3.49 (2H, m), 3.60-3.66 (1H, m), 3.88 (2H, t, *J* = 5.2 Hz), 3.98 (2H, td, *J* = 8.2, 3.8 Hz), 4.19 (2H, t, *J* = 5.2 Hz), 7.03-7.06 (2H, m), 7.44 (1H, dd, *J* = 9.2, 1.7 Hz), 7.52-7.55 (4H, m), 7.87 (1H, d, *J* = 9.2 Hz), 8.16 (1H, s), 8.71-8.72 (2H, m), 8.80 (1H, s). | - | D |
| 128 | 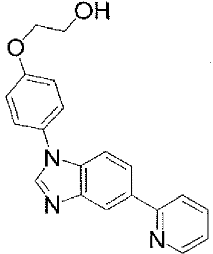 | ¹H-NMR (DMSO-D₆) δ: 3.75-3.79 (2H, m), 4.09 (2H, t, *J* = 5.3 Hz), 4.93 (1H, t, *J* = 5.4 Hz), 7.18-7.22 (2H, m), 7.31-7.35 (1H, m), 7.60-7.63 (3H, m), 7.88 (1H, dt, *J* = 7.8, 1.7 Hz), 8.06 (1H, dd, *J* = 7.8, 0.9 Hz), 8.13 (1H, dd, *J* = 8.7, 1.4 Hz), 8.47 (1H, d, *J* = 0.9 Hz), 8.54 (1H, s), 8.66-8.68 (1H, m). | - | C |
| 129 | 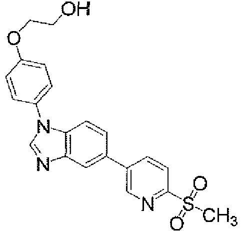 | ¹H-NMR (CDCl₃) δ: 2.05 (1H, t, *J* = 6.2 Hz), 3.29 (3H, s), 4.03-4.07 (2H, m), 4.20 (2H, t, *J* = 4.4 Hz), 7.12-7.16 (2H, m), 7.44-7.47 (2H, m), 7.55-7.61 (2H, m), 8.12-8.21 (4H, m), 9.02-9.02 (1H, m). | - | C |
| 130 | 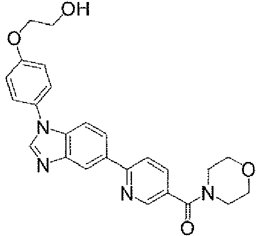 | ¹H-NMR (DMSO-D₆) δ: 3.42-3.69 (8H, m), 3.76-3.79 (2H, m), 4.10 (2H, t, *J* = 4.6 Hz), 4.94 (1H, t, *J* = 5.5 Hz), 7.20 (2H, d, *J* = 8.7 Hz), 7.61-7.65 (3H, m), 7.92-7.95 (1H, m), 8.15-8.18 (2H, m), 8.53 (1H, s), 8.56 (1H, s), 8.73 (1H, br s). | | C |
| 131 | 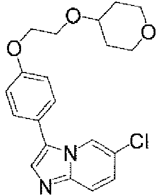 | ¹H-NMR (DMSO-D₆) δ: 1.39-1.46 (2H, m), 1.87-1.90 (2H, m), 3.33-3.37 (2H, m), 3.56-3.62 (1H, m), 3.80-3.84 (4H, m), 4.21 (2H, t, *J* = 4.6 Hz), 7.19-7.22 (2H, m), 7.64-7.66 (2H, m), 7.97 (1H, dd, *J* = 9.7, 1.5 Hz), 8.05 (1H, d, *J* = 9.7 Hz), 8.31 (1H, s), 8.78 (1H, d, *J* = 1.5 Hz). | HCl | D |

**[Table 22]**

| | | | | |
|---|---|---|---|---|
| 132 | 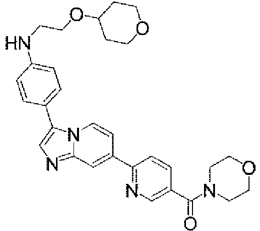 | ¹H-NMR (DMSO-D₆) δ: 1.39-1.46 (2H, m), 1.85-1.88 (2H, m), 3.29-3.70 (15H, m), 3.82 (2H, td, *J* = 12.0, 3.8 Hz), 6.88 (2H, d, *J* = 8.6 Hz), 7.48 (2H, d, *J* = 8.6 Hz), 8.11 (1H, dd, *J* = 8.0, 2.3 Hz), 8.20 (1H, dd, *J* = 7.5, 1.7 Hz), 8.33 (1H, s), 8.40 (1H, d, *J* = 8.0 Hz), 8.69 (1H, d, *J* = 1.7 Hz), 8.83 (1H, d, *J* = 7.5 Hz), 8.86 (1H, d, *J* = 2.3 Hz). | 3HCl | C |
| 133 | 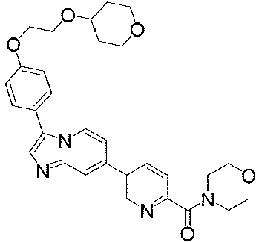 | ¹H-NMR (CDCl₃) δ: 1.63-1.70 (2H, m), 1.95-1.98 (2H, m), 3.45-3.50 (2H, m), 3.61-3.66 (1H, m), 3.73-3.79 (4H, m), 3.82-3.91 (6H, m), 3.98 (2H, td, J = 4.4, 11.7 Hz), 4.21-4.23 (2H, m), 7.08 (1H, dd, J = 7.2, 2.0 Hz), 7.09-7.12 (2H, m), 7.49-7.52 (2H, m), 7.73 (1H, s), 7.84 (1H, d, J = 8.0 Hz), 7.92 (1H, br s), 8.08 (1H, dd, J = 8.0, 2.3 Hz), 8.37 (1H, dd, J = 7.2, 1.2 Hz), 8.90 (1H, d, J = 2.3 Hz). | - | B |
| 134 | 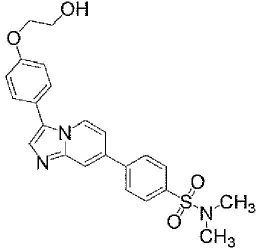 | ¹H-NMR (CDCl₃) δ: 2.20 (1H, t, J = 6.0 Hz), 2.78 (6H, s), 4.02-4.05 (2H, m), 4.18-4.19 (2H, m), 7.09-7.12 (3H, m), 7.50-7.53 (2H, m), 7.72 (1H, s), 7.81-7.84 (2H, m), 7.88-7.90 (2H, m), 7.92-7.93 (1H, m), 8.35-8.36 (1H, m). | - | C |
| 135 | 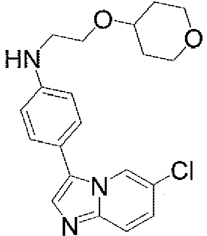 | ¹H-NMR (DMSO-D₆) δ: 1.39-1.46 (2H, m), 1.83-1.88 (2H, m), 3.29 (2H, t, *J* = 5.7 Hz), 3.31-3.36 (2H, m), 3.51-3.56 (1H, m), 3.62 (2H, t, *J* = 5.7 Hz), 3.81 (2H, td, *J* = 7.9, 4.0 Hz), 6.85 (2H, d, *J* = 8.6 Hz), 7.43 (2H, d, *J* = 8.6 Hz), 8.00 (1H, dd, *J* = 9.7, 1.5 Hz), 8.06 (1H, d, *J* = 9.7 Hz), 8.26 (1H, s), 8.74 (1H, d, *J* = 1.5 Hz). | 2HCl | c |

**[Table 23]**

| | | | | |
|---|---|---|---|---|
| 136 | 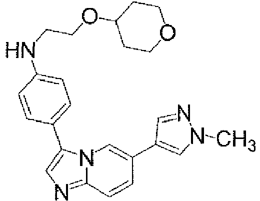 | ¹H-NMR (DMSO-D₆) δ: 1.39-1.46 (2H, m), 1.84-1.89 (2H, m), 3.31-3.37 (4H, m), 3.51-3.57 (1H, m), 3.65 (2H, t, *J* = 5.7 Hz), 3.81 (2H, td, *J =* 8.0, 3.6 Hz), 3.88 (3H, s), 6.96 (2H, d, *J* = 8.2 Hz), 7.52 (2H, d, *J* = 8.2 Hz), 8.04-8.06 (2H, m), 8.20 (1H, dd, *J* = 9.2, 1.7 Hz), 8.23 (1H, s), 8.40 (1H, s), 8.65 (1H, s). | 3HCl | C |
| 137 | 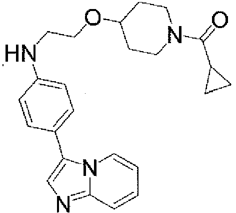 | ¹H-NMR (DMSO-D₆) δ: 0.66-0.72 (4H, m), 1.36-1.45 (2H, m), 1.79-1.88 (2H, m), 1.94-1.99 (1H, m), 3.06-3.12 (1H, m), 3.30 (2H, t, *J* = 5.7 Hz), 3.34-3.41 (1H, m), 3.58-3.61 (1H, m), 3.63 (2H, t, *J* = 5.7 Hz), 3.82-3.96 (2H, m), 6.86 (2H, d, *J* = 8.6 Hz), 7.43 (2H, d, *J* = 8.6 Hz), 7.48 (1H, dt, *J* = 6.9, 1.2 Hz), 7.95-7.98 (1H, m), 8.02-8.04 (1H, m), 8.24 (1H, s), 8.71 (1H, d, *J* = 6. 9 Hz). | 2HCl | G |
| 138 | 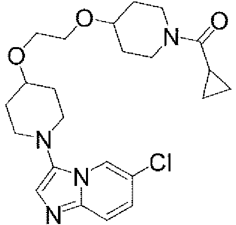 | ¹H-NMR (DMSO-D₆) δ: 0.65-0.72 (4H, m), 1.28-1.37 (1H, m), 1.40-1.48 (1H, m), 1.72-1.81 (3H, m), 1.86-1.92 (1H, m), 1.94-2.04 (3H, m), 2.88-2.93 (2H, m), 3.05-3.11 (1H, m), 3.18-3.22 (2H, m), 3.35-3.41 (1H, m), 3.53-3.70 (6H, m), 3.82-3.88 (1H, m), 3.91-3.97 (1H, m), 7.95-8.00 (2H, m), 8.03 (1H, s), 8.81-8.82 (1H, m). | 2HCl | G |
| 139 | 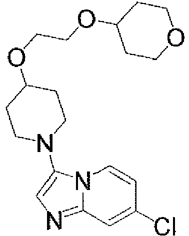 | ¹H-NMR (DMSO-D₆) δ: 1.35-1.43 (2H, m), 1.68-1.75 (2H, m), 1.83-1.87 (2H, m), 1.97-2.03 (2H, m), 2.87-2.92 (2H, m), 3.17-3.21 (2H, m), 3.31-3.36 (2H, m), 3.49-3.60 (6H, m), 3.80 (2H, td, *J* = 7.9, 4.0 Hz), 7.53 (1H, dd, *J =* 7.5, 2.3 Hz), 7.93 (1H, s), 8.11 (1H, d, *J* = 2.3 Hz), 8.61 (1H, d, *J* = 7.5 Hz). | HCl | E |

**[Table 24]**

| | | | | |
|---|---|---|---|---|
| 140 | 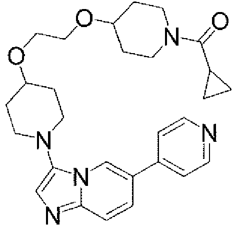 | ¹H-NMR (CDCl₃) δ: 0.75 (2H, dd, *J* = 3.4, 8.0 Hz), 0.96-0.98 (2H, m), 1.56-1.70 (2H, m), 1.73-1.78 (1H, m), 1.83-1.96 (4H, m), 2.06-2.12 (2H, m), 2.90-2.95 (2H, m), 3.24-3.34 (3H, m), 3.40-3.46 (1H, m), 3.59-3.70 (6H, m), 3.93-3.99 (2H, m), 7.34 (1H, s), 7.40 (1H, dd, *J* = 9.5, 2.0 Hz), 7.52-7.53 (2H, m), 7.64 (1H, d, *J* = 9.5 Hz), 8.20-8.21 (1H, m), 8.71-8.72 (2H, m). | - | B |
| 141 | 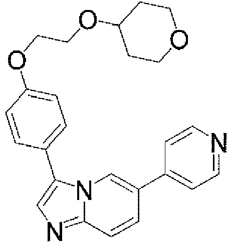 | ¹H-NMR (CDCl₃) δ: 1.64-1.69 (2H, m), 1.94-1.98 (2H, m), 3.44-3.49 (2H, m), 3.61-3.66 (1H, m), 3.89 (2H, t, *J* = 4.9 Hz), 3.97 (2H, td, *J* = 4.0, 8.0 Hz), 4.22 (2H, t, *J* = 4.9 Hz), 7.10-7.13 (2H, m), 7.45-7.47 (3H, m), 7.48-7.51 (2H, m), 7.69 (1H, s), 7.77 (1H, d, *J* = 9.2 Hz), 8.49 (1H, s), 8.67-8.69 (2H, m). | - | B |
| 142 | 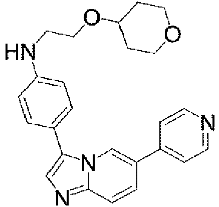 | ¹H-NMR (DMSO-D₆) δ: 1.38-1.45 (2H, m), 1.84-1.89 (2H, m), 3.30-3.36 (4H, m), 3.51-3.56 (1H, m), 3.63 (2H, t, *J* = 5.7 Hz), 3.81 (2H, td, *J* = 3.6, 8.0 Hz), 6.89 (2H, d, *J* = 8.6 Hz), 7.56 (2H, d, *J* = 8.6 Hz), 8.24 (1H, d, *J* = 9.5 Hz), 8.35 (1H, s), 8.42 (2H, d, *J* = 6.9 Hz), 8.44 (1H, dd, *J* = 9.5, 1.7 Hz), 9.01 (2H, d, *J* = 6.9 Hz), 9.10-9.11 (1H, m). | 3HCl | C |
| 143 | 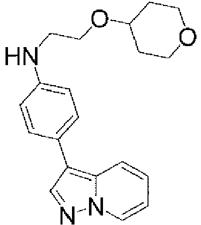 | ¹H-NMR (CDCl₃) δ: 1.58-1.66 (2H, m), 1.91-1.94 (2H, m), 3.36 (2H, t, *J* = 5.4 Hz), 3.43-3.48 (2H, m), 3.52-3.57 (1H, m), 3.72 (2H, t, *J* = 5.4 Hz), 3.96 (2H, td, *J* = 8.0, 3.6 Hz), 4.15 (1H, br s), 6.73-6.77 (3H, m), 7.09-7.13 (1H, m), 7.41-7.43 (2H, m), 7.76 (1H, d, *J* = 9.2 Hz), 8.06 (1H, s), 8.46 (1H, d, *J =* 6.9 Hz). | - | C |

**[Table 25]**

| | | | | |
|---|---|---|---|---|
| 144 | 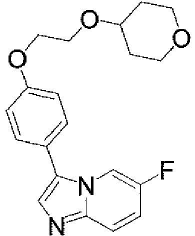 | ¹H-NMR (DMSO-D₆) δ: 1.39-1.46 (2H, m), 1.87-1.90 (2H, m), 3.33-3.37 (2H, m), 3.56-3.61 (1H, m), 3.80-3.84 (4H, m), 4.19-4.21 (2H, m), 7.19-7.22 (2H, m), 7.64-7.67 (2H, m), 8.03 (1H, ddd, *J* = 9.7, 8.0, 2.3 Hz), 8.11 (1H, dd, *J* = 9.7, 4.9 Hz), 8.37 (1H, s), 8.90 (1H, dd, *J* = 3.4, 2.3 Hz). | HCl | D |
| 145 | 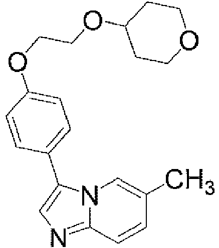 | ¹H-NMR (DMSO-D₆) δ: 1.39-1.46 (2H, m), 1.87-1.90 (2H, m), 2.42 (3H, s), 3.33-3.37 (2H, m), 3.56-3.61 (1H, m), 3.80-3.84 (4H, m), 4.20-4.22 (2H, m), 7.20-7.23 (2H, m), 7.63-7.66 (2H, m), 7.84 (1 H, dd, *J* = 9.2, 1.2 Hz), 7.95 (1 H, d, *J* = 9.2 Hz), 8.27 (1 H, s), 8.51-8.52 (1 H, m). | HCl | D |
| 146 | 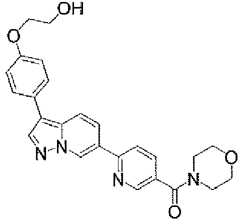 | ¹H-NMR (CDCl₃) δ: 2.10 (1H, t, *J* = 6.3 Hz), 3.52-3.88 (8H, m), 4.00-4.03 (2H, m), 4.16 (2H, t, *J* = 4.6 Hz), 7.03-7.06 (2H, m), 7.53-7.56 (2H, m), 7.80 (1H, d, *J* = 8.0 Hz), 7.83-7.87 (2H, m), 7.89 (1H, dd, *J* = 8.0, 2.3 Hz), 8.17 (1H, s), 8.76 (1H, d, *J* = 2.3 Hz), 9.21 (1H, br s). | - | C |
| 147 | 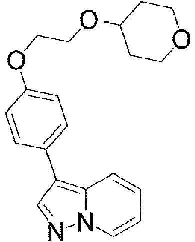 | ¹H-NMR (DMSO-D₆) δ: 1.39-1.46 (2H, m), 1.85-1.90 (2H, m), 3.32-3.37 (2H, m), 3.55-3.60 (1H, m), 3.77-3.84 (4H, m), 4.12-4.14 (2H, m), 6.92 (1H, dt, *J* = 6.9, 1.1 Hz), 7.03-7.06 (2H, m), 7.28 (1H, ddd, *J* = 8.9, 6.9, 1.1 Hz), 7.58-7.61 (2H, m), 7.89-7.91 (1H, m), 8.28 (1H, s), 8.70 (1H, td, *J* = 6.9, 1.1 Hz). | - | D |
| 148 | 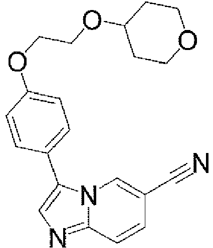 | ¹H-NMR (CDCl₃) δ: 1.63-1.70 (2H, m), 1.95-1.98 (2H, m), 3.45-3.50 (2H, m), 3.61-3.66 (1H, m), 3.88-3.90 (2H, m), 3.98 (2H, td, *J* = 8.0, 3.6 Hz), 4.21-4.23 (2H, m), 7.10-7.13 (2H, m), 7.28 (1H, dd, *J* = 9.2, 1.7 Hz), 7.42-7.45 (2H, m), 7.73 (1H, dd, *J* = 9.2, 1.1 Hz), 7.76 (1H, s), 8.65 (1H, dd, *J* = 1.7, 1.1 Hz). | - | c |

**[Table 26]**

| | | | | |
|---|---|---|---|---|
| 149 | 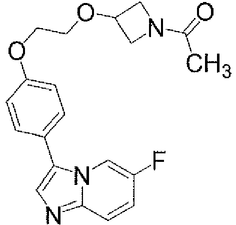 | ¹H-NMR (CDCl₃) δ: 1.89 (3H, s), 3.83-3.85 (2H, m), 3.96 (1H, dd, *J =* 10.9, 4.0 Hz), 4.09 (1H, ddd, *J* = 9.2, 4.0, 1.1 Hz), 4.19-4.23 (3H, m), 4.30-4.34 (1H, m), 4.42-4.46 (1H, m), 7.05-7.13 (3H, m), 7.45-7.48 (2H, m), 7.64 (1H, dd, *J* = 9.7, 5.2 Hz), 7.68 (1H, s), 8.17 (1H, dd, *J* = 4.3, 2.0 Hz). | - | G |
| 150 | 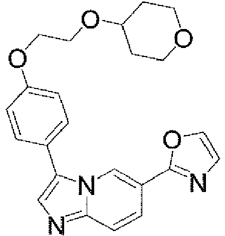 | ¹H-NMR (CDCl₃) δ: 1.63-1.70 (2H, m), 1.95-1.99 (2H, m), 3.45-3.50 (2H, m), 3.61-3.67 (1H, m), 3.88-3.90 (2H, m), 3.98 (2H, td, *J =* 12.0, 4.6 Hz), 4.21-4.23 (2H, m), 7.10-7.13 (2H, m), 7.24 (1H, s), 7.49-7.51 (2H, m), 7.68 (1H, s), 7.72 (1H, s), 7.73 (1H, d, *J* = 9.7 Hz), 7.83 (1H, dd, *J* = 9.7, 1.7 Hz), 8.94-8.95 (1H, m). | - | B |

## Claims

1. A compound having the general formula (I) or a pharmacologically acceptable salt thereof: 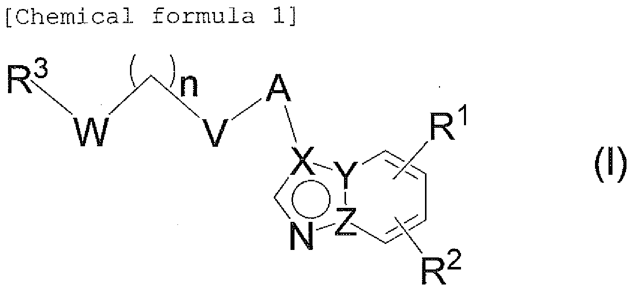 wherein each substituent is defined as follows:
R¹ and R² each independently represent
a hydrogen atom or a group selected from a substituent group α, or together form a substituent having bonds at two positions,
R³ represents:
a hydrogen atom,
a C1-C6 alkyl group optionally substituted by a substituent group α,
a tetrahydropyranyl group optionally substituted by a substituent group α,
a tetrahydrofuranyl group optionally substituted by a substituent group α,
a dioxanyl group optionally substituted by a substituent group α,
a C1-C6 alkoxycarbonyl group,
a heterocyclic group optionally substituted by a group selected from a substituent group α, or
a C6-C10 aryl group optionally substituted by a substituent group α,
X, Y, and Z represent:
when X is a nitrogen atom, Y and Z are carbon atoms,
when Y is a nitrogen atom, X and Z are carbon atoms, or
when Z is a nitrogen atom, X and Y are carbon atoms,
A represents: a phenylene group optionally substituted by
a group selected from a substituent group α, or
a hetero ring having bonds at two positions, wherein the hetero ring is optionally substituted by a group selected from a substituent group α,
V represents: -O-, -NH-, or -S-,
n represents: an integer from 1 to 6, and
W represents: -O-, -NH-, or -S-, wherein
the substituent group α includes:
a hydroxyl group, a halogen group, a cyano group, a C1-C6 alkyl group, a C3-C6 cycloalkyl group, a C3-C6 cycloalkoxy group, a halo C1-C6 alkyl group, a C2-C6 alkynyl group, a C1-C6 alkoxy group, a halo C1-C6 alkoxy group, a C1-C6 alkylsulfonyl group, a formyl group, a C1-C6 alkylcarbonyl group, a carboxy group, a C1-C6 alkoxycarbonyl group, a C1-C6 alkylamino group, a C3-C6 cycloalkylcarbonyl group, a phenyl group optionally substituted by a group selected from a substituent group
β, a heterocyclic group optionally substituted by a group selected from a substituent group β, a carbonyl group to which a heterocyclic group is bound, and a C1-C6 alkylenedioxylene group, and
the substituent group β includes:
a nitro group, a cyano group, an aminosulfonyl group, a di C1-C6 alkylamino group, a di C1-C6 alkylaminocarbonyl group, a di C1-C6 alkylaminocarbonyloxy group, a di C1-C6 alkylaminosulfonyl group, a carboxy group, a C1-C6 alkyl group, a C1-C6 alkoxy group, a formyl group, a C1-C6 alkylcarbonyl group, a C1-C6 alkylcarbonylamino group, a C1-C6 alkylsulfonylamino group, a morpholinylcarbonyl group, and a carbamoyl group.

2. A compound or a pharmacologically acceptable salt thereof according to Claim 1, wherein the heterocyclic group is an azetidinyl group, a pyrrolidinyl group, a piperidinyl group, a morpholinyl group, a pyrazinyl group, a pyridinyl group, a tetrahydropyridinyl group, a 2-oxo-1,2-dihydropyridinyl group, a pyrrolyl group, a tetrahydropyranyl group, a tetrahydrofuranyl group, a dioxanyl group, a pyrimidyl group, a pyrazoyl group, an imidazoyl group, or an oxazoyl group, and the hetero ring is azetidine, pyrrolidine, piperidine, morpholine, pyrazine, pyridine, tetrahydropyridine, 2-oxo-1,2-dihydropyridine, pyrrole, tetrahydropyran, tetrahydrofuran, dioxane, pyrimidine, pyrazole, imidazole, or oxazole.

3. A compound or a pharmacologically acceptable salt thereof according to Claim 1 or 2, wherein A is a group selected from the following groups: 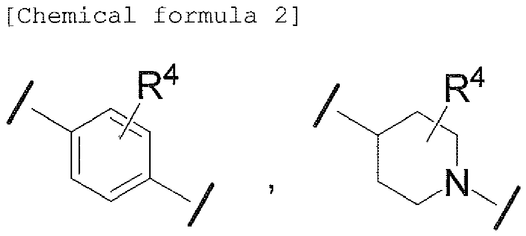 wherein R⁴ represents: a hydrogen atom or a group selected from the substituent group α.

4. A compound or a pharmacologically acceptable salt thereof according to any one of Claims 1 to 3, wherein R¹ and R² are each independently a hydrogen atom, a hydroxyl group, a cyano group, a fluorine atom, a chlorine atom, a methyl group, an ethyl group, a methoxy group, an ethoxy group, a difluoromethyl group, a trifluoromethyl group, a difluoromethoxy group, a trifluoromethoxy group, a phenyl group optionally substituted by a group selected from the substituent group β, a heterocyclic group optionally substituted by a group selected from the substituent group β, or a carbamoyl group.

5. A compound or a pharmacologically acceptable salt thereof according to Claim 1, wherein the general formula (I) is the general formula (Ia):

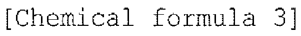

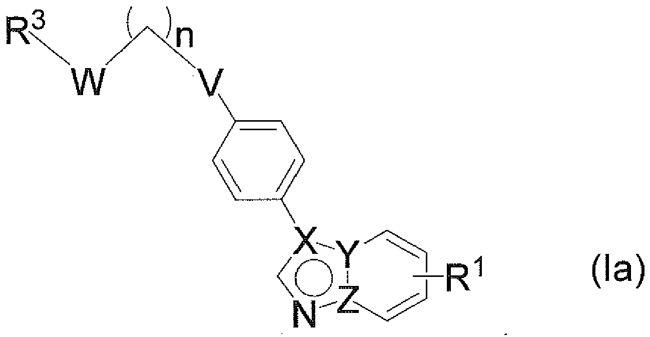

6. A compound or a pharmacologically acceptable salt thereof according to any one of Claims 1 to 5, wherein R³ is a hydrogen atom, a C1-C6 alkyl group substituted by a hydroxyl group, a tetrahydropyranyl group, a tetrahydrofuranyl group, a dioxanyl group, a C1-C6 alkoxycarbonyl group, a piperidinyl group optionally substituted by a group selected from the substituent group α, or a phenyl group optionally substituted by the substituent group α.

7. A compound or a pharmacologically acceptable salt thereof according to any one of Claims 1 to 6, wherein V is -O- or -NH-.

8. A compound or a pharmacologically acceptable salt thereof according to any one of Claims 1 to 7, wherein W is -O- or -NH-.

9. A compound or a pharmacologically acceptable salt thereof according to any one of Claims 1 to 8, wherein n is an integer from 1 to 3.

10. A compound selected from the following group of compounds or a pharmacologically acceptable salt thereof:
2-[4-(1H-benzimidazol-1-yl)phenoxy]ethanol,
2-[4-(7-chloroimidazo[1,2-a]pyridin-3-yl)phenoxy]ethanol,
2-(2-{[4-(1H-benzimidazol-1-yl)phenyl]amino}ethoxy)ethanol,
2-{[4-(6-chloro-1H-benzimidazol-1-yl)phenyl]amino}ethanol,
2-{[4-(5-methoxy-1H-benzimidazol-1-yl)phenyl]amino}ethanol,
1-[4-(2-methoxyethoxy)phenyl]-1H-benzimidazole,
2-[4-(6-fluoroimidazo[1,2-a]pyridin-3-yl)phenoxy]ethanol,
2-{4-[6-(1H-pyrrol-3-yl)imidazo[1,2-a]pyridin-3-yl]phenoxy}ethanol,
2-[4-(5-pyridin-4-yl-1H-benzimidazol-1-yl)phenoxy]ethanol,
2-({1-[7-(1-acetyl-1,2,3,6-tetrahydropyridin-4-yl)imidazo[1,2-a]pyridin-3-yl]piperidin-4-yl}oxy)ethanol,
2-[4-(6-pyridin-4-ylpyrazolo[1,5-a]pyridin-3-yl)phenoxy]ethanol,
4-{2-[4-(1H-benzimidazol-1-yl)phenoxy]ethoxy}benzoic acid,
4-{1-[4-(2-hydroxyethoxy)phenyl]-1H-benzimidazol-5-yl}benzoic acid,
N-(4-{1-[4-(2-hydroxyethoxy)phenyl]-1H-benzimidazol-5-yl}phenyl)acetamide,
4-{1-[4-(2-hydroxyethoxy)phenyl]-1H-benzimidazol-5-yl}benzamide,
2-(4-{5-[6-(morpholin-4-ylcarbonyl)pyridin-3-yl]-1H-benzimidazol-1-yl}phenoxy)ethanol,
7-[4-(morpholin-4-ylcarbonyl)phenyl]-3-{4-[2-(tetrahydro-2H-pyran-4-yloxy)ethoxy]piperidin-1-yl}imidazo[1,2-a]pyridine,
3-{4-[2-(tetrahydro-2H-pyran-2-yloxy)ethoxy]piperidin-1-yl}pyrazolo[1,5-a]pyridine,
3-{4-[2-(tetrahydro-2H-pyran-4-yloxy)ethoxy]phenyl}imidazo[1,2-a]pyridine-6-carboxamide,
6-methoxy-3-{4-[2-(tetrahydro-2H-pyran-4-yloxy)ethoxy]phenyl}imidazo[1,2-a]pyridine,
6-ethynyl-3-{4-[2-(tetrahydro-2H-pyran-4-yloxy)ethoxy]phenyl}imidazo[1,2-a]pyridine,
6-morpholin-4-yl-3-{4-[2-(tetrahydro-2H-pyran-4-yloxy)ethoxy]phenyl}imidazo[1,2-a]pyridine,
6-(1H-pyrazol-1-yl)-3-{4-[2-(tetrahydro-2H-pyran-4-yloxy)ethoxy]phenyl}imidazo[1,2-a]pyridine,
1-{4-[2-(tetrahydro-2H-pyran-4-yloxy)ethoxylphenyl}-1H-benzimidazole-6-carbonitrile, and
6-(difluoromethoxy)-1-{4-[2-(tetrahydro-2H-pyran-4-yloxy)ethoxy]phenyl}-1H-benzimidazole.

11. A pharmaceutical composition comprising a compound or a pharmacologically acceptable salt thereof according to any one of Claims 1 to 10 as an active ingredient.

12. A pharmaceutical composition according to Claim 11, wherein the pharmaceutical composition is used for promoting osteogenesis.

13. A pharmaceutical composition according to Claim 11, wherein the pharmaceutical composition is used for improving bone metabolism.

14. A pharmaceutical composition according to Claim 11, wherein the pharmaceutical composition is used for the prevention or treatment of a disease associated with bone metabolism.

15. A pharmaceutical composition according to Claim 14, wherein the disease associated with bone metabolism is osteoporosis.

16. A method for improving bone metabolism, comprising administering an effective amount of a pharmaceutical composition according to Claim 11 to a mammal.

17. A method for preventing or treating a disease associated with bone metabolism, comprising administering an effective amount of a pharmaceutical composition according to Claim 11 to a mammal.

18. A method for preventing or treating osteoporosis, comprising administering an effective amount of a pharmaceutical composition according to Claim 11 to a mammal.
